(19) Europäisches Patentamt
European Patent Office
Office européen des brevets

(11) EP 4 703 386 A1

(12) EUROPEAN PATENT APPLICATION
published in accordance with Art. 153(4) EPC

(43) Date of publication:
04.03.2026 Bulletin 2026/10

(21) Application number: 24796310.1

(22) Date of filing: 28.04.2024

(51) International Patent Classification (IPC):
C07K 16/28 (2006.01)   C12N 5/10 (2006.01)
A61K 39/00 (2006.01)   C12N 15/13 (2006.01)
A61K 39/395 (2006.01)   A61P 35/00 (2006.01)

(52) Cooperative Patent Classification (CPC):
A61K 39/00; A61K 39/395; A61K 45/00;
A61K 47/68; A61P 35/00; C07K 16/00;
C07K 16/28; C07K 19/00; C12N 5/10; C12N 15/62;
C12N 15/861; C12N 15/863; C12N 15/867;
G01N 33/68

(86) International application number:
PCT/CN2024/090299

(87) International publication number:
WO 2024/222941 (31.10.2024 Gazette 2024/44)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 28.04.2023 CN 202310480737

(71) Applicant: Shanghai Sinobay Biotechnology Co.,
Ltd.
Shanghai 201506 (CN)

(72) Inventors:
• XU, Jianqing
Shanghai 201506 (CN)
• ZHANG, Xiaoyan
Shanghai 201506 (CN)
• DING, Xiangqing
Shanghai 201506 (CN)

(74) Representative: Uexküll & Stolberg
Partnerschaft von
Patent- und Rechtsanwälten mbB
Beselerstraße 4
22607 Hamburg (DE)

Remarks:
The complete document including Reference
Table(s) and the Sequence Listing(s) can be
downloaded from the EPO website

(54) **ANTIBODY SPECIFICALLY BINDING TO CD276, PREPARATION METHOD THEREFOR AND USE THEREOF**

(57) Provided are an antibody specifically binding to CD276 and use thereof. Specifically provided are a CD276 antibody or an antigen binding fragment thereof, and the like. Also provided are a polynucleotide encoding the CD276 antibody, a nucleic acid construct comprising the polynucleotide, an expression vector comprising the nucleic acid construct, a preparation method and a transformed cell thereof. The provided CD276 antibody can bind to CD276 protein at a high affinity and a high specificity, can be prepared into a target recognition domain of CAR-T cells, and can also be prepared into a bispecific T-cell engager (BiTE) to exert an anti-tumor effect for preventing or treating cancers.

Fig. 1

EP 4 703 386 A1

**Description**

**TECHNICAL FIELD**

The present invention belongs to the technical field of biomedicine, and particularly, relates to antibodies capable of specifically binding to CD276 protein or an antigen binding fragment thereof. The present invention further relates to a preparation method of the antibodies and use thereof.

**BACKGROUND ART**

In recent years, people have placed high hopes on immunotherapy for the treatment of tumors. Chimeric antigen receptor-T cells (CAR-T) are a novel immunotherapy targeting specific antigens on the surfaces of tumor cells, which has a function of specifically and efficiently killing tumor cells expressing tumor antigens. Compared with traditional chemotherapy and hematopoietic stem cell transplantation, CAR-T is more accurate in killing tumor cells, thereby greatly reducing toxic and side effects while improving the efficacy. However, due to the lack of suitable target antigens, the short duration of CAR-T cells in the body, immune escape, immunosuppressive tumor microenvironment and other factors, it is not good for the treatment of solid tumors. Therefore, the selection of target antigens is crucial to the specificity and effectiveness of CAR, and the safety of genetically modified T cells themselves.

During the human immune response, the activation of T cells requires a T cell receptor (TCR) to first recognize an MHC complex (pMHC) in which an antigen peptide is bound to antigen-presenting cells (APC). This recognition cannot fully activate T cells, and requires the participation of a second-level signal for costimulatory molecule interaction, thereby fully activating the T cells.

B7 family proteins have been shown to be involved in regulating T cell responses. More than 10 types of B7 protein molecules have been discovered so far, and they are divided into three categories based on signals they transduce and functions during T cell activation: I) co-stimulation; II) co-inhibition; and III) co-stimulation/inhibition. B7 family proteins also show positive and negative regulatory functions in immune responses, becoming co-inhibitory or co-stimulatory ligands that regulate anti-tumor immune responses.

B7-H3, also known as CD276, is a type I transmembrane protein and a member of B7 immune co-stimulatory and co-inhibitory family. Its ligand is still unclear. CD276 is widely expressed at an mRNA level, but protein expression is relatively limited to the surfaces of non-immune cells, such as quiescent fibroblasts, endothelial cells, osteoblasts, and amniotic fluid stem cells, as well as induced antigen-presenting cells and NK cells. Studies have shown that CD276 expression is abnormally elevated in tumor cells, and the adhesion ability of cell adhesion proteins increases accordingly, leading to enhanced migration and invasion abilities of tumor cells. In addition, CD276 can lead to increased NF-κB activity, resulting in increased expression of VEGF and IL-8, further promoting tumor-related angiogenesis and tumor invasion. More evidences show that the B7-H3 mainly plays a co-inhibitory role in immune cells, which is conducive to tumor cells to escape immune surveillance.

Therefore, overexpression of CD276 is associated with poor prognosis in tumor patients and the invasion and metastasis potentials of tumors in *in-vitro* models. CD276 is a powerful potential target for immunotherapy. In previous studies, CD276-targeted therapy included monoclonal antibodies that block CD276, CD276-specific antibody-dependent cell-mediated cytotoxicity (ADCC), antibody drug conjugates (ADCs), and bispecific antibodies targeting CD276/CD3, CD276-specific small molecule inhibitors, and CAR-T cell therapy. However, it is unclear what scFv can achieve the best therapeutic effect for this target. In addition, mouse-derived antibodies traditionally used, due to their heterogeneity, can cause human anti-mouse antibody reaction (HAMA), resulting in CAR-T being quickly cleared from the circulatory system and losing efficacy.

At present, small molecules, monoclonal antibodies, bispecific antibodies, ADC, radioimmunotherapy, CAR-T, CAR-NK and other drug forms targeting B7-H3 targets continue to emerge and continue to be verified in preclinical and clinical studies. Enoblituzumab monoclonal antibody is the first monoclonal antibody targeting CD276 protein and having an Fc optimization function. In clinical studies, according to the data published at the 2018 Annual Meeting of the Society for Immunotherapy of Cancer, the objective response rate (ORR) of Enoblituzumab combined with a PD-1 monoclonal antibody in the treatment of patients with head and neck squamous cell carcinoma reached 33.3%; and for patients with non-small cell lung cancer having a low PD-L1 expression (<1%), the ORR also reached 35.7%. In particular, for patients who have previously received PD-1/L1 antibody drug treatment, not only did they respond to the treatment again (partial remission, PR), but the disease control also reached the same proportion as PD-1/L1 treatment-naive patients. Given that CD276 is widely expressed in many types of tumors, it means that drugs targeting CD276 theoretically have a broad therapeutic field, and it is believed that its clinical and commercial value will be continuously explored and gradually released. In addition, there are many other different types of CD276-targeted drugs being studied toward more solid tumors. Although most of them are in the early stages, they have also shown clinical benefit signals. There are relatively few developments targeting the CD276 target, but CD276 has also begun to show its great value. There will definitely be more breakthrough clinical data in the future to meet the clinical needs of patients with CD276 positive or overexpression.

Therefore, there is an urgent need to develop more efficient anti-CD276 fully humanized antibodies to provide new ideas and prospects for the treatment of cancers with abnormal expression of CD276.

## DISCLOSURE OF THE INVENTION

An object of the present invention is to address the deficiencies of the prior art, and screen anti-CD276 antibodies by using human phage display library technology. The antibodies specifically binding to CD276 provided by the present invention can all recognize and bind to human CD276, and have a strong activity in neutralizing CD276. The anti-CD276 antibodies of the present invention, as CAR-T cell recognition regions, can enhance the accuracy of antigen recognition and effectively avoid immune rejection, help improve the effectiveness and safety of CD276-CAR-T cell therapy, and exert anti-tumor effects.

The object of the present invention is achieved by the following technical solutions:

A first aspect of the present invention provides an antibody capable of specifically binding to CD276 protein or an antigen binding fragment thereof, the antibody or the antigen binding fragment thereof comprising:

(a) a heavy chain variable region comprising the following three complementary determining regions: (i) VH CDR1, consisting of the following sequence: SEQ ID NO: 3, 141 or 144, or a sequence having one or more amino acid substitutions, deletions or additions relative to any of them; (ii) VH CDR2, consisting of the following sequence: SEQ ID NO: 4, 142 or 145, or a sequence having one or more amino acid substitutions, deletions or additions relative to any of them; and (iii) VH CDR3, consisting of the following sequence: SEQ ID NO: 5, 143 or 146, or a sequence having one or more amino acid substitutions, deletions or additions relative to any of them; and/or

(b) a light chain variable region comprising the following three complementary determining regions: (iv) VL CDR1, consisting of the following sequence: SEQ ID NO: 8, 147 or 150, or a sequence having one or more amino acid substitutions, deletions or additions relative to any of them; (v) VL CDR2, consisting of the following sequence: SEQ ID NO: 9, 148 or 151, or a sequence having one or more amino acid substitutions, deletions or additions relative to any of them; and (vi) VL CDR3, consisting of the following sequence: SEQ ID NO: 10, 149 or 152, or a sequence having one or more amino acid substitutions, deletions or additions relative to any of them.

Preferably, the substitution in any one of (i) to (vi) is a conservative substitution.

Preferably, VH in the antibody or the antigen binding fragment thereof comprises: VH CDR1 as shown in SEQ ID NO: 3, VH CDR2 as shown in SEQ ID NO: 4, and VH CDR3 as shown in SEQ ID NO: 5; and VL in the antibody or the antigen binding fragment thereof comprises: VL CDR1 as shown in SEQ ID NO: 8, VL CDR2 as shown in SEQ ID NO: 9, and VL CDR3 as shown in SEQ ID NO: 10.

Preferably, VH in the antibody or the antigen binding fragment thereof comprises: VH CDR1 as shown in SEQ ID NO: 141, VH CDR2 as shown in SEQ ID NO: 142, and VH CDR3 as shown in SEQ ID NO: 143; and VL in the antibody or the antigen binding fragment thereof comprises: VL CDR1 as shown in SEQ ID NO: 147, VL CDR2 as shown in SEQ ID NO: 148, and VL CDR3 as shown in SEQ ID NO: 149.

Preferably, VH in the antibody or the antigen binding fragment thereof comprises: VH CDR1 as shown in SEQ ID NO: 144, VH CDR2 as shown in SEQ ID NO: 145, and VH CDR3 as shown in SEQ ID NO: 146; and VL in the antibody or the antigen binding fragment thereof comprises: VL CDR1 as shown in SEQ ID NO: 150, VL CDR2 as shown in SEQ ID NO: 151, and VL CDR3 as shown in SEQ ID NO: 152.

An antibody capable of specifically binding to CD276 protein or an antigen binding fragment thereof is provided, the antibody or the antigen binding fragment thereof comprising:

(a) a heavy chain variable region comprising the following three complementary determining regions: (i) VH CDR1, consisting of the following sequence: SEQ ID NO: 13, 153 or 156, or a sequence having one or more amino acid substitutions, deletions or additions relative to any of them; (ii) VH CDR2, consisting of the following sequence: SEQ ID NO: 14, 154 or 157, or a sequence having one or more amino acid substitutions, deletions or additions relative to any of them; and (iii) VH CDR3, consisting of the following sequence: SEQ ID NO: 15, 155 or 158, or a sequence having one or more amino acid substitutions, deletions or additions relative to any of them; and/or

(b) a light chain variable region comprising the following three complementary determining regions: (iv) VL CDR1, consisting of the following sequence: SEQ ID NO: 18, 159 or 162, or a sequence having one or more amino acid substitutions, deletions or additions relative to any of them; (v) VL CDR2, consisting of the following sequence: SEQ ID NO: 19, 160 or 163, or a sequence having one or more amino acid substitutions, deletions or additions relative to any of them; and (vi) VL CDR3, consisting of the following sequence: SEQ ID NO: 20, 161 or 164, or a sequence having one or more amino acid substitutions, deletions or additions relative to any of them.

Preferably, the substitution in any one of (i) to (vi) is a conservative substitution.

Preferably, VH in the antibody or the antigen binding fragment thereof comprises: VH CDR1 as shown in SEQ ID NO: 13, VH CDR2 as shown in SEQ ID NO: 14, and VH CDR3 as shown in SEQ ID NO: 15; and VL in the antibody or the antigen binding fragment thereof comprises: VL CDR1 as shown in SEQ ID NO: 18, VL CDR2 as shown in SEQ ID NO: 19, and VL CDR3 as shown in SEQ ID NO: 20.

Preferably, VH in the antibody or the antigen binding fragment thereof comprises: VH CDR1 as shown in SEQ ID NO: 153, VH CDR2 as shown in SEQ ID NO: 154, and VH CDR3 as shown in SEQ ID NO: 155; and VL in the antibody or the antigen binding fragment thereof comprises: VL CDR1 as shown in SEQ ID NO: 159, VL CDR2 as shown in SEQ ID NO: 160, and VL CDR3 as shown in SEQ ID NO: 161.

Preferably, VH in the antibody or the antigen binding fragment thereof comprises: VH CDR1 as shown in SEQ ID NO: 156, VH CDR2 as shown in SEQ ID NO: 157, and VH CDR3 as shown in SEQ ID NO: 158; and VL in the antibody or the antigen binding fragment thereof comprises: VL CDR1 as shown in SEQ ID NO: 162, VL CDR2 as shown in SEQ ID NO: 163, and VL CDR3 as shown in SEQ ID NO: 164.

An antibody capable of specifically binding to CD276 protein or an antigen binding fragment thereof is provided, the antibody or the antigen binding fragment thereof comprising:

(a) a heavy chain variable region comprising the following three complementary determining regions: (i) VH CDR1, consisting of the following sequence: SEQ ID NO: 23, 165 or 168, or a sequence having one or more amino acid substitutions, deletions or additions relative to any of them; (ii) VH CDR2, consisting of the following sequence: SEQ ID NO: 24, 166 or 169, or a sequence having one or more amino acid substitutions, deletions or additions relative to any of them; and (iii) VH CDR3, consisting of the following sequence: SEQ ID NO: 25, 167 or 170, or a sequence having one or more amino acid substitutions, deletions or additions relative to any of them; and/or

(b) a light chain variable region comprising the following three complementary determining regions: (iv) VL CDR1, consisting of the following sequence: SEQ ID NO: 28, 171 or 174, or a sequence having one or more amino acid substitutions, deletions or additions relative to any of them; (v) VL CDR2, consisting of the following sequence: SEQ ID NO: 29, 172 or 175, or a sequence having one or more amino acid substitutions, deletions or additions relative to any of them; and (vi) VL CDR3, consisting of the following sequence: SEQ ID NO: 30, 173 or 176, or a sequence having one or more amino acid substitutions, deletions or additions relative to any of them.

Preferably, the substitution in any one of (i) to (vi) is a conservative substitution.

Preferably, VH in the antibody or the antigen binding fragment thereof comprises: VH CDR1 as shown in SEQ ID NO: 23, VH CDR2 as shown in SEQ ID NO: 24, and VH CDR3 as shown in SEQ ID NO: 25; and VL in the antibody or the antigen binding fragment thereof comprises: VL CDR1 as shown in SEQ ID NO: 28, VL CDR2 as shown in SEQ ID NO: 29, and VL CDR3 as shown in SEQ ID NO: 30.

Preferably, VH in the antibody or the antigen binding fragment thereof comprises: VH CDR1 as shown in SEQ ID NO: 165, VH CDR2 as shown in SEQ ID NO: 166, and VH CDR3 as shown in SEQ ID NO: 167; and VL in the antibody or the antigen binding fragment thereof comprises: VL CDR1 as shown in SEQ ID NO: 171, VL CDR2 as shown in SEQ ID NO: 172, and VL CDR3 as shown in SEQ ID NO: 173.

Preferably, VH in the antibody or the antigen binding fragment thereof comprises: VH CDR1 as shown in SEQ ID NO: 168, VH CDR2 as shown in SEQ ID NO: 169, and VH CDR3 as shown in SEQ ID NO: 170; and VL in the antibody or the antigen binding fragment thereof comprises: VL CDR1 as shown in SEQ ID NO: 174, VL CDR2 as shown in SEQ ID NO: 175, and VL CDR3 as shown in SEQ ID NO: 176.

An antibody capable of specifically binding to CD276 protein or an antigen binding fragment thereof is provided, the antibody or the antigen binding fragment thereof comprising:

(a) a heavy chain variable region comprising the following three complementary determining regions: (i) VH CDR1, consisting of the following sequence: SEQ ID NO: 33, 177 or 180, or a sequence having one or more amino acid substitutions, deletions or additions relative to any of them; (ii) VH CDR2, consisting of the following sequence: SEQ ID NO: 34, 178 or 181, or a sequence having one or more amino acid substitutions, deletions or additions relative to any of them; and (iii) VH CDR3, consisting of the following sequence: SEQ ID NO: 35, 179 or 182, or a sequence having one or more amino acid substitutions, deletions or additions relative to any of them; and/or

(b) a light chain variable region comprising the following three complementary determining regions: (iv) VL CDR1, consisting of the following sequence: SEQ ID NO: 38, 183 or 186, or a sequence having one or more amino acid substitutions, deletions or additions relative to any of them; (v) VL CDR2, consisting of the following sequence: SEQ ID NO: 39, 184 or 187, or a sequence having one or more amino acid substitutions, deletions or additions relative to any of them; and (vi) VL CDR3, consisting of the following sequence: SEQ ID NO: 40, 185 or 188, or a sequence having one or more amino acid substitutions, deletions or additions relative to any of them.

Preferably, the substitution in any one of (i) to (vi) is a conservative substitution.

Preferably, VH in the antibody or the antigen binding fragment thereof comprises: VH CDR1 as shown in SEQ ID NO: 33, VH CDR2 as shown in SEQ ID NO: 34, and VH CDR3 as shown in SEQ ID NO: 35; and VL in the antibody or the antigen binding fragment thereof comprises: VL CDR1 as shown in SEQ ID NO: 38, VL CDR2 as shown in SEQ ID NO: 39, and VL CDR3 as shown in SEQ ID NO: 40.

Preferably, VH in the antibody or the antigen binding fragment thereof comprises: VH CDR1 as shown in SEQ ID NO: 177, VH CDR2 as shown in SEQ ID NO: 178, and VH CDR3 as shown in SEQ ID NO: 179; and VL in the antibody or the antigen binding fragment thereof comprises: VL CDR1 as shown in SEQ ID NO: 183, VL CDR2 as shown in SEQ ID NO: 184, and VL CDR3 as shown in SEQ ID NO: 185.

Preferably, VH in the antibody or the antigen binding fragment thereof comprises: VH CDR1 as shown in SEQ ID NO: 180, VH CDR2 as shown in SEQ ID NO: 181, and VH CDR3 as shown in SEQ ID NO: 182; and VL in the antibody or the antigen binding fragment thereof comprises: VL CDR1 as shown in SEQ ID NO: 186, VL CDR2 as shown in SEQ ID NO: 187, and VL CDR3 as shown in SEQ ID NO: 188.

An antibody capable of specifically binding to CD276 protein or an antigen binding fragment thereof is provided, the antibody or the antigen binding fragment thereof comprising:

(a) a heavy chain variable region comprising the following three complementary determining regions: (i) VH CDR1, consisting of the following sequence: SEQ ID NO: 43, 189 or 192, or a sequence having one or more amino acid substitutions, deletions or additions relative to any of them; (ii) VH CDR2, consisting of the following sequence: SEQ ID NO: 44, 190 or 193, or a sequence having one or more amino acid substitutions, deletions or additions relative to any of them; and (iii) VH CDR3, consisting of the following sequence: SEQ ID NO: 45, 191 or 194, or a sequence having one or more amino acid substitutions, deletions or additions relative to any of them; and/or
(b) a light chain variable region comprising the following three complementary determining regions: (iv) VL CDR1, consisting of the following sequence: SEQ ID NO: 48, 195 or 198, or a sequence having one or more amino acid substitutions, deletions or additions relative to any of them; (v) VL CDR2, consisting of the following sequence: SEQ ID NO: 49, 196 or 199, or a sequence having one or more amino acid substitutions, deletions or additions relative to any of them; and (vi) VL CDR3, consisting of the following sequence: SEQ ID NO: 50, 197 or 200, or a sequence having one or more amino acid substitutions, deletions or additions relative to any of them.

Preferably, the substitution in any one of (i) to (vi) is a conservative substitution.

Preferably, VH in the antibody or the antigen binding fragment thereof comprises: VH CDR1 as shown in SEQ ID NO: 43, VH CDR2 as shown in SEQ ID NO: 44, and VH CDR3 as shown in SEQ ID NO: 45; and VL in the antibody or the antigen binding fragment thereof comprises: VL CDR1 as shown in SEQ ID NO: 48, VL CDR2 as shown in SEQ ID NO: 49, and VL CDR3 as shown in SEQ ID NO: 50.

Preferably, VH in the antibody or the antigen binding fragment thereof comprises: VH CDR1 as shown in SEQ ID NO: 189, VH CDR2 as shown in SEQ ID NO: 190, and VH CDR3 as shown in SEQ ID NO: 191; and VL in the antibody or the antigen binding fragment thereof comprises: VL CDR1 as shown in SEQ ID NO: 195, VL CDR2 as shown in SEQ ID NO: 196, and VL CDR3 as shown in SEQ ID NO: 197.

Preferably, VH in the antibody or the antigen binding fragment thereof comprises: VH CDR1 as shown in SEQ ID NO: 192, VH CDR2 as shown in SEQ ID NO: 193, and VH CDR3 as shown in SEQ ID NO: 194; and VL in the antibody or the antigen binding fragment thereof comprises: VL CDR1 as shown in SEQ ID NO: 198, VL CDR2 as shown in SEQ ID NO: 199, and VL CDR3 as shown in SEQ ID NO: 200.

An antibody capable of specifically binding to CD276 protein or an antigen binding fragment thereof is provided, the antibody or the antigen binding fragment thereof comprising:

(a) a heavy chain variable region comprising the following three complementary determining regions: (i) VH CDR1, consisting of the following sequence: SEQ ID NO: 53, 201 or 204, or a sequence having one or more amino acid substitutions, deletions or additions relative to any of them; (ii) VH CDR2, consisting of the following sequence: SEQ ID NO: 54, 202 or 205, or a sequence having one or more amino acid substitutions, deletions or additions relative to any of them; and (iii) VH CDR3, consisting of the following sequence: SEQ ID NO: 55, 203 or 206, or a sequence having one or more amino acid substitutions, deletions or additions relative to any of them; and/or
(b) a light chain variable region comprising the following three complementary determining regions: (iv) VL CDR1, consisting of the following sequence: SEQ ID NO: 58, 207 or 210, or a sequence having one or more amino acid substitutions, deletions or additions relative to any of them; (v) VL CDR2, consisting of the following sequence: SEQ ID NO: 59, 208 or 211, or a sequence having one or more amino acid substitutions, deletions or additions relative to any of them; and (vi) VL CDR3, consisting of the following sequence: SEQ ID NO: 60, 209 or 212, or a sequence having one or more amino acid substitutions, deletions or additions relative to any of them.

Preferably, the substitution in any one of (i) to (vi) is a conservative substitution.

Preferably, VH in the antibody or the antigen binding fragment thereof comprises: VH CDR1 as shown in SEQ ID NO: 53, VH CDR2 as shown in SEQ ID NO: 54, and VH CDR3 as shown in SEQ ID NO: 55; and VL in the antibody or the antigen binding fragment thereof comprises: VL CDR1 as shown in SEQ ID NO: 58, VL CDR2 as shown in SEQ ID NO: 59, and VL CDR3 as shown in SEQ ID NO: 60.

Preferably, VH in the antibody or the antigen binding fragment thereof comprises: VH CDR1 as shown in SEQ ID NO: 201, VH CDR2 as shown in SEQ ID NO: 202, and VH CDR3 as shown in SEQ ID NO: 203; and VL in the antibody or the antigen binding fragment thereof comprises: VL CDR1 as shown in SEQ ID NO: 207, VL CDR2 as shown in SEQ ID NO: 208, and VL CDR3 as shown in SEQ ID NO: 209.

Preferably, VH in the antibody or the antigen binding fragment thereof comprises: VH CDR1 as shown in SEQ ID NO: 204, VH CDR2 as shown in SEQ ID NO: 205, and VH CDR3 as shown in SEQ ID NO: 206; and VL in the antibody or the antigen binding fragment thereof comprises: VL CDR1 as shown in SEQ ID NO: 210, VL CDR2 as shown in SEQ ID NO: 211, and VL CDR3 as shown in SEQ ID NO: 212.

An antibody capable of specifically binding to CD276 protein or an antigen binding fragment thereof is provided, the antibody or the antigen binding fragment thereof comprising:

(a) a heavy chain variable region comprising the following three complementary determining regions: (i) VH CDR1, consisting of the following sequence: SEQ ID NO: 63, 213 or 216, or a sequence having one or more amino acid substitutions, deletions or additions relative to any of them; (ii) VH CDR2, consisting of the following sequence: SEQ ID NO: 64, 214 or 217, or a sequence having one or more amino acid substitutions, deletions or additions relative to any of them; and (iii) VH CDR3, consisting of the following sequence: SEQ ID NO: 65, 215 or 218, or a sequence having one or more amino acid substitutions, deletions or additions relative to any of them; and/or
(b) a light chain variable region comprising the following three complementary determining regions: (iv) VL CDR1, consisting of the following sequence: SEQ ID NO: 68, 219 or 222, or a sequence having one or more amino acid substitutions, deletions or additions relative to any of them; (v) VL CDR2, consisting of the following sequence: SEQ ID NO: 69, 220 or 223, or a sequence having one or more amino acid substitutions, deletions or additions relative to any of them; and (vi) VL CDR3, consisting of the following sequence: SEQ ID NO: 70, 221 or 224, or a sequence having one or more amino acid substitutions, deletions or additions relative to any of them.

Preferably, the substitution in any one of (i) to (vi) is a conservative substitution.

Preferably, VH in the antibody or the antigen binding fragment thereof comprises: VH CDR1 as shown in SEQ ID NO: 63, VH CDR2 as shown in SEQ ID NO: 64, and VH CDR3 as shown in SEQ ID NO: 65; and VL in the antibody or the antigen binding fragment thereof comprises: VL CDR1 as shown in SEQ ID NO: 68, VL CDR2 as shown in SEQ ID NO: 69, and VL CDR3 as shown in SEQ ID NO: 70.

Preferably, VH in the antibody or the antigen binding fragment thereof comprises: VH CDR1 as shown in SEQ ID NO: 213, VH CDR2 as shown in SEQ ID NO: 214, and VH CDR3 as shown in SEQ ID NO: 215; and VL in the antibody or the antigen binding fragment thereof comprises: VL CDR1 as shown in SEQ ID NO: 219, VL CDR2 as shown in SEQ ID NO: 220, and VL CDR3 as shown in SEQ ID NO: 221.

Preferably, VH in the antibody or the antigen binding fragment thereof comprises: VH CDR1 as shown in SEQ ID NO: 216, VH CDR2 as shown in SEQ ID NO: 217, and VH CDR3 as shown in SEQ ID NO: 218; and VL in the antibody or the antigen binding fragment thereof comprises: VL CDR1 as shown in SEQ ID NO: 222, VL CDR2 as shown in SEQ ID NO: 223, and VL CDR3 as shown in SEQ ID NO: 224.

An antibody capable of specifically binding to CD276 protein or an antigen binding fragment thereof is provided, the antibody or the antigen binding fragment thereof comprising:

(a) a heavy chain variable region comprising the following three complementary determining regions: (i) VH CDR1, consisting of the following sequence: SEQ ID NO: 73, 225 or 228, or a sequence having one or more amino acid substitutions, deletions or additions relative to any of them; (ii) VH CDR2, consisting of the following sequence: SEQ ID NO: 74, 226 or 229, or a sequence having one or more amino acid substitutions, deletions or additions relative to any of them; and (iii) VH CDR3, consisting of the following sequence: SEQ ID NO: 75, 227 or 230, or a sequence having one or more amino acid substitutions, deletions or additions relative to any of them; and/or
(b) a light chain variable region comprising the following three complementary determining regions: (iv) VL CDR1, consisting of the following sequence: SEQ ID NO: 78, 231 or 234, or a sequence having one or more amino acid substitutions, deletions or additions relative to any of them; (v) VL CDR2, consisting of the following sequence: SEQ ID NO: 79, 232 or 235, or a sequence having one or more amino acid substitutions, deletions or additions relative to any of them; and (vi) VL CDR3, consisting of the following sequence: SEQ ID NO: 80, 233 or 236, or a sequence having one or more amino acid substitutions, deletions or additions relative to any of them.

Preferably, the substitution in any one of (i) to (vi) is a conservative substitution.

Preferably, VH in the antibody or the antigen binding fragment thereof comprises: VH CDR1 as shown in SEQ ID NO: 73, VH CDR2 as shown in SEQ ID NO: 74, and VH CDR3 as shown in SEQ ID NO: 75; and VL in the antibody or the antigen binding fragment thereof comprises: VL CDR1 as shown in SEQ ID NO: 78, VL CDR2 as shown in SEQ ID NO: 79, and VL CDR3 as shown in SEQ ID NO: 80.

Preferably, VH in the antibody or the antigen binding fragment thereof comprises: VH CDR1 as shown in SEQ ID NO: 225, VH CDR2 as shown in SEQ ID NO: 226, and VH CDR3 as shown in SEQ ID NO: 227; and VL in the antibody or the antigen binding fragment thereof comprises: VL CDR1 as shown in SEQ ID NO: 231, VL CDR2 as shown in SEQ ID NO: 232, and VL CDR3 as shown in SEQ ID NO: 233.

Preferably, VH in the antibody or the antigen binding fragment thereof comprises: VH CDR1 as shown in SEQ ID NO: 228, VH CDR2 as shown in SEQ ID NO: 229, and VH CDR3 as shown in SEQ ID NO: 230; and VL in the antibody or the antigen binding fragment thereof comprises: VL CDR1 as shown in SEQ ID NO: 234, VL CDR2 as shown in SEQ ID NO: 235, and VL CDR3 as shown in SEQ ID NO: 236.

An antibody capable of specifically binding to CD276 protein or an antigen binding fragment thereof is provided, the antibody or the antigen binding fragment thereof comprising:

(a) a heavy chain variable region comprising the following three complementary determining regions: (i) VH CDR1, consisting of the following sequence: SEQ ID NO: 83, 237 or 240, or a sequence having one or more amino acid substitutions, deletions or additions relative to any of them; (ii) VH CDR2, consisting of the following sequence: SEQ ID NO: 84, 238 or 241, or a sequence having one or more amino acid substitutions, deletions or additions relative to any of them; and (iii) VH CDR3, consisting of the following sequence: SEQ ID NO: 85, 239 or 242, or a sequence having one or more amino acid substitutions, deletions or additions relative to any of them; and/or
(b) a light chain variable region comprising the following three complementary determining regions: (iv) VL CDR1, consisting of the following sequence: SEQ ID NO: 88, 243 or 246, or a sequence having one or more amino acid substitutions, deletions or additions relative to any of them; (v) VL CDR2, consisting of the following sequence: SEQ ID NO: 89, 244 or 247, or a sequence having one or more amino acid substitutions, deletions or additions relative to any of them; and (vi) VL CDR3, consisting of the following sequence: SEQ ID NO: 90, 245 or 248, or a sequence having one or more amino acid substitutions, deletions or additions relative to any of them.

Preferably, the substitution in any one of (i) to (vi) is a conservative substitution.

Preferably, VH in the antibody or the antigen binding fragment thereof comprises: VH CDR1 as shown in SEQ ID NO: 83, VH CDR2 as shown in SEQ ID NO: 84, and VH CDR3 as shown in SEQ ID NO: 85; and VL in the antibody or the antigen binding fragment thereof comprises: VL CDR1 as shown in SEQ ID NO: 88, VL CDR2 as shown in SEQ ID NO: 89, and VL CDR3 as shown in SEQ ID NO: 90.

Preferably, VH in the antibody or the antigen binding fragment thereof comprises: VH CDR1 as shown in SEQ ID NO: 237, VH CDR2 as shown in SEQ ID NO: 238, and VH CDR3 as shown in SEQ ID NO: 239; and VL in the antibody or the antigen binding fragment thereof comprises: VL CDR1 as shown in SEQ ID NO: 243, VL CDR2 as shown in SEQ ID NO: 244, and VL CDR3 as shown in SEQ ID NO: 245.

Preferably, VH in the antibody or the antigen binding fragment thereof comprises: VH CDR1 as shown in SEQ ID NO: 240, VH CDR2 as shown in SEQ ID NO: 241, and VH CDR3 as shown in SEQ ID NO: 242; and VL in the antibody or the antigen binding fragment thereof comprises: VL CDR1 as shown in SEQ ID NO: 246, VL CDR2 as shown in SEQ ID NO: 247, and VL CDR3 as shown in SEQ ID NO: 248.

An antibody capable of specifically binding to CD276 protein or an antigen binding fragment thereof is provided, the antibody or the antigen binding fragment thereof comprising:

(a) a heavy chain variable region comprising the following three complementary determining regions: (i) VH CDR1, consisting of the following sequence: SEQ ID NO: 93, 249 or 252, or a sequence having one or more amino acid substitutions, deletions or additions relative to any of them; (ii) VH CDR2, consisting of the following sequence: SEQ ID NO: 94, 250 or 253, or a sequence having one or more amino acid substitutions, deletions or additions relative to any of them; and (iii) VH CDR3, consisting of the following sequence: SEQ ID NO: 95, 251 or 254, or a sequence having one or more amino acid substitutions, deletions or additions relative to any of them; and/or
(b) a light chain variable region comprising the following three complementary determining regions: (iv) VL CDR1, consisting of the following sequence: SEQ ID NO: 98, 255 or 258, or a sequence having one or more amino acid substitutions, deletions or additions relative to any of them; (v) VL CDR2, consisting of the following sequence: SEQ ID NO: 99, 256 or 259, or a sequence having one or more amino acid substitutions, deletions or additions relative to any of them; and (vi) VL CDR3, consisting of the following sequence: SEQ ID NO: 100, 257 or 260, or a sequence having one or more amino acid substitutions, deletions or additions relative to any of them.

Preferably, the substitution in any one of (i) to (vi) is a conservative substitution.

Preferably, VH in the antibody or the antigen binding fragment thereof comprises: VH CDR1 as shown in SEQ ID NO: 93, VH CDR2 as shown in SEQ ID NO: 94, and VH CDR3 as shown in SEQ ID NO: 95; and VL in the antibody or the antigen binding fragment thereof comprises: VL CDR1 as shown in SEQ ID NO: 98, VL CDR2 as shown in SEQ ID NO: 99, and VL CDR3 as shown in SEQ ID NO: 100.

Preferably, VH in the antibody or the antigen binding fragment thereof comprises: VH CDR1 as shown in SEQ ID NO: 249, VH CDR2 as shown in SEQ ID NO: 250, and VH CDR3 as shown in SEQ ID NO: 251; and VL in the antibody or the antigen binding fragment thereof comprises: VL CDR1 as shown in SEQ ID NO: 255, VL CDR2 as shown in SEQ ID NO: 256, and VL CDR3 as shown in SEQ ID NO: 257.

Preferably, VH in the antibody or the antigen binding fragment thereof comprises: VH CDR1 as shown in SEQ ID NO: 252, VH CDR2 as shown in SEQ ID NO: 253, and VH CDR3 as shown in SEQ ID NO: 254; and VL in the antibody or the antigen binding fragment thereof comprises: VL CDR1 as shown in SEQ ID NO: 258, VL CDR2 as shown in SEQ ID NO: 259, and VL CDR3 as shown in SEQ ID NO: 260.

An antibody capable of specifically binding to CD276 protein or an antigen binding fragment thereof is provided, the antibody or the antigen binding fragment thereof comprising:

(a) a heavy chain variable region comprising the following three complementary determining regions: (i) VH CDR1, consisting of the following sequence: SEQ ID NO: 103, 261 or 264, or a sequence having one or more amino acid substitutions, deletions or additions relative to any of them; (ii) VH CDR2, consisting of the following sequence: SEQ ID NO: 104, 262 or 265, or a sequence having one or more amino acid substitutions, deletions or additions relative to any of them; and (iii) VH CDR3, consisting of the following sequence: SEQ ID NO: 105, 263 or 266, or a sequence having one or more amino acid substitutions, deletions or additions relative to any of them; and/or
(b) a light chain variable region comprising the following three complementary determining regions: (iv) VL CDR1, consisting of the following sequence: SEQ ID NO: 108, 267 or 270, or a sequence having one or more amino acid substitutions, deletions or additions relative to any of them; (v) VL CDR2, consisting of the following sequence: SEQ ID NO: 109, 268 or 271, or a sequence having one or more amino acid substitutions, deletions or additions relative to any of them; and (vi) VL CDR3, consisting of the following sequence: SEQ ID NO: 110, 269 or 272, or a sequence having one or more amino acid substitutions, deletions or additions relative to any of them.

Preferably, the substitution in any one of (i) to (vi) is a conservative substitution.

Preferably, VH in the antibody or the antigen binding fragment thereof comprises: VH CDR1 as shown in SEQ ID NO: 103, VH CDR2 as shown in SEQ ID NO: 104, and VH CDR3 as shown in SEQ ID NO: 105; and VL in the antibody or the antigen binding fragment thereof comprises: VL CDR1 as shown in SEQ ID NO: 108, VL CDR2 as shown in SEQ ID NO: 109, and VL CDR3 as shown in SEQ ID NO: 110.

Preferably, VH in the antibody or the antigen binding fragment thereof comprises: VH CDR1 as shown in SEQ ID NO: 261, VH CDR2 as shown in SEQ ID NO: 262, and VH CDR3 as shown in SEQ ID NO: 263; and VL in the antibody or the antigen binding fragment thereof comprises: VL CDR1 as shown in SEQ ID NO: 267, VL CDR2 as shown in SEQ ID NO: 268, and VL CDR3 as shown in SEQ ID NO: 269.

Preferably, VH in the antibody or the antigen binding fragment thereof comprises: VH CDR1 as shown in SEQ ID NO: 264, VH CDR2 as shown in SEQ ID NO: 265, and VH CDR3 as shown in SEQ ID NO: 266; and VL in the antibody or the antigen binding fragment thereof comprises: VL CDR1 as shown in SEQ ID NO: 270, VL CDR2 as shown in SEQ ID NO: 271, and VL CDR3 as shown in SEQ ID NO: 272.

An antibody capable of specifically binding to CD276 protein or an antigen binding fragment thereof is provided, the antibody or the antigen binding fragment thereof comprising:

(a) a heavy chain variable region comprising the following three complementary determining regions: (i) VH CDR1, consisting of the following sequence: SEQ ID NO: 113, 273 or 276, or a sequence having one or more amino acid substitutions, deletions or additions relative to any of them; (ii) VH CDR2, consisting of the following sequence: SEQ ID NO: 114, 274 or 277, or a sequence having one or more amino acid substitutions, deletions or additions relative to any of them; and (iii) VH CDR3, consisting of the following sequence: SEQ ID NO: 115, 275 or 278, or a sequence having one or more amino acid substitutions, deletions or additions relative to any of them; and/or
(b) a light chain variable region comprising the following three complementary determining regions: (iv) VL CDR1, consisting of the following sequence: SEQ ID NO: 118, 279 or 282, or a sequence having one or more amino acid substitutions, deletions or additions relative to any of them; (v) VL CDR2, consisting of the following sequence: SEQ ID NO: 119, 280 or 283, or a sequence having one or more amino acid substitutions, deletions or additions relative to any of them; and (vi) VL CDR3, consisting of the following sequence: SEQ ID NO: 120, 281 or 284, or a sequence having one or more amino acid substitutions, deletions or additions relative to any of them.

Preferably, the substitution in any one of (i) to (vi) is a conservative substitution.

Preferably, VH in the antibody or the antigen binding fragment thereof comprises: VH CDR1 as shown in SEQ ID NO: 113, VH CDR2 as shown in SEQ ID NO: 114, and VH CDR3 as shown in SEQ ID NO: 115; and VL in the antibody or the antigen binding fragment thereof comprises: VL CDR1 as shown in SEQ ID NO: 118, VL CDR2 as shown in SEQ ID NO: 119, and VL CDR3 as shown in SEQ ID NO: 120.

Preferably, VH in the antibody or the antigen binding fragment thereof comprises: VH CDR1 as shown in SEQ ID NO: 273, VH CDR2 as shown in SEQ ID NO: 274, and VH CDR3 as shown in SEQ ID NO: 275; and VL in the antibody or the antigen binding fragment thereof comprises: VL CDR1 as shown in SEQ ID NO: 279, VL CDR2 as shown in SEQ ID NO: 280, and VL CDR3 as shown in SEQ ID NO: 281.

Preferably, VH in the antibody or the antigen binding fragment thereof comprises: VH CDR1 as shown in SEQ ID NO: 276, VH CDR2 as shown in SEQ ID NO: 277, and VH CDR3 as shown in SEQ ID NO: 278; and VL in the antibody or the antigen binding fragment thereof comprises: VL CDR1 as shown in SEQ ID NO: 282, VL CDR2 as shown in SEQ ID NO: 283, and VL CDR3 as shown in SEQ ID NO: 284.

An antibody capable of specifically binding to CD276 protein or an antigen binding fragment thereof is provided, the antibody or the antigen binding fragment thereof comprising:

(a) a heavy chain variable region comprising the following three complementary determining regions: (i) VH CDR1, consisting of the following sequence: SEQ ID NO: 123, 285 or 288, or a sequence having one or more amino acid substitutions, deletions or additions relative to any of them; (ii) VH CDR2, consisting of the following sequence: SEQ ID NO: 124, 286 or 289, or a sequence having one or more amino acid substitutions, deletions or additions relative to any of them; and (iii) VH CDR3, consisting of the following sequence: SEQ ID NO: 125, 287 or 290, or a sequence having one or more amino acid substitutions, deletions or additions relative to any of them; and/or

(b) a light chain variable region comprising the following three complementary determining regions:

(iv) VL CDR1, consisting of the following sequence: SEQ ID NO: 128, 291 or 294, or a sequence having one or more amino acid substitutions, deletions or additions relative to any of them; (v) VL CDR2, consisting of the following sequence: SEQ ID NO: 129, 292 or 295, or a sequence having one or more amino acid substitutions, deletions or additions relative to any of them; and (vi) VL CDR3, consisting of the following sequence: SEQ ID NO: 130, 293 or 296, or a sequence having one or more amino acid substitutions, deletions or additions relative to any of them.

Preferably, the substitution in any one of (i) to (vi) is a conservative substitution.

Preferably, VH in the antibody or the antigen binding fragment thereof comprises: VH CDR1 as shown in SEQ ID NO: 123, VH CDR2 as shown in SEQ ID NO: 124, and VH CDR3 as shown in SEQ ID NO: 125; and VL in the antibody or the antigen binding fragment thereof comprises: VL CDR1 as shown in SEQ ID NO: 128, VL CDR2 as shown in SEQ ID NO: 129, and VL CDR3 as shown in SEQ ID NO: 130.

Preferably, VH in the antibody or the antigen binding fragment thereof comprises: VH CDR1 as shown in SEQ ID NO: 285, VH CDR2 as shown in SEQ ID NO: 286, and VH CDR3 as shown in SEQ ID NO: 287; and VL in the antibody or the antigen binding fragment thereof comprises: VL CDR1 as shown in SEQ ID NO: 291, VL CDR2 as shown in SEQ ID NO: 292, and VL CDR3 as shown in SEQ ID NO: 293.

Preferably, VH in the antibody or the antigen binding fragment thereof comprises: VH CDR1 as shown in SEQ ID NO: 288, VH CDR2 as shown in SEQ ID NO: 289, and VH CDR3 as shown in SEQ ID NO: 290; and VL in the antibody or the antigen binding fragment thereof comprises: VL CDR1 as shown in SEQ ID NO: 294, VL CDR2 as shown in SEQ ID NO: 295, and VL CDR3 as shown in SEQ ID NO: 296.

An antibody capable of specifically binding to CD276 protein or an antigen binding fragment thereof is provided, the antibody or the antigen binding fragment thereof comprising:

(a) a heavy chain variable region comprising the following three complementary determining regions: (i) VH CDR1, consisting of the following sequence: SEQ ID NO: 133, 297 or 300, or a sequence having one or more amino acid substitutions, deletions or additions relative to any of them; (ii) VH CDR2, consisting of the following sequence: SEQ ID NO: 134, 298 or 301, or a sequence having one or more amino acid substitutions, deletions or additions relative to any of them; and (iii) VH CDR3, consisting of the following sequence: SEQ ID NO: 135, 299 or 302, or a sequence having one or more amino acid substitutions, deletions or additions relative to any of them; and/or

(b) a light chain variable region comprising the following three complementary determining regions: (iv) VL CDR1, consisting of the following sequence: SEQ ID NO: 138, 303 or 306, or a sequence having one or more amino acid substitutions, deletions or additions relative to any of them; (v) VL CDR2, consisting of the following sequence: SEQ ID NO: 139, 304 or 307, or a sequence having one or more amino acid substitutions, deletions or additions relative to any of them; and (vi) VL CDR3, consisting of the following sequence: SEQ ID NO: 140, 305 or 308, or a sequence having one or more amino acid substitutions, deletions or additions relative to any of them.

Preferably, the substitution in any one of (i) to (vi) is a conservative substitution.

Preferably, VH in the antibody or the antigen binding fragment thereof comprises: VH CDR1 as shown in SEQ ID NO: 133, VH CDR2 as shown in SEQ ID NO: 134, and VH CDR3 as shown in SEQ ID NO: 135; and VL in the antibody or the antigen binding fragment thereof comprises: VL CDR1 as shown in SEQ ID NO: 138, VL CDR2 as shown in SEQ ID NO: 139, and VL CDR3 as shown in SEQ ID NO: 140.

Preferably, VH in the antibody or the antigen binding fragment thereof comprises: VH CDR1 as shown in SEQ ID NO: 297, VH CDR2 as shown in SEQ ID NO: 298, and VH CDR3 as shown in SEQ ID NO: 299; and VL in the antibody or the antigen binding fragment thereof comprises: VL CDR1 as shown in SEQ ID NO: 303, VL CDR2 as shown in SEQ ID NO: 304, and VL CDR3 as shown in SEQ ID NO: 305.

Preferably, VH in the antibody or the antigen binding fragment thereof comprises: VH CDR1 as shown in SEQ ID NO: 300, VH CDR2 as shown in SEQ ID NO: 301, and VH CDR3 as shown in SEQ ID NO: 302; and VL in the antibody or the antigen binding fragment thereof comprises: VL CDR1 as shown in SEQ ID NO: 306, VL CDR2 as shown in SEQ ID NO: 307, and VL CDR3 as shown in SEQ ID NO: 308.

The present invention further provides an antibody capable of specifically binding to CD276 protein or an antigen binding fragment thereof, the antibody or the antigen binding fragment thereof comprising a heavy chain variable region and a light chain variable region, wherein the heavy chain variable region comprises three CDRs comprised in the heavy chain variable region as shown in any one of SEQ ID NO: 2, 12, 22, 32, 42, 52, 62, 72, 82, 92, 102, 112, 122 or 132; and the light chain variable region comprises three CDRs comprised in the light chain variable region as shown in any one of SEQ ID NO: 7, 17, 27, 37, 47, 57, 67, 77, 87, 97, 107, 117, 127 or 137.

Preferably, the three CDRs comprised in the heavy chain variable region, and/or the three CDRs comprised in the light chain variable region, are defined by the Kabat, Chothia or IMGT numbering system; and preferably, the three CDRs comprised in the heavy chain variable region, and/or the three CDRs comprised in the light chain variable region, are defined by the Kabat, Chothia or IMGT numbering system.

In a preferred embodiment, the antibody or the antigen binding fragment thereof comprises:

(a) a heavy chain variable region, comprising an amino acid sequence selected from the group consisting of: (i) a sequence as shown in SEQ ID NO: 2; (ii) a sequence having one or more amino acid substitutions, deletions or additions relative to the sequence as shown in SEQ ID NO: 2; or (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 2; and/or
(b) a light chain variable region, comprising an amino acid sequence selected from the group consisting of: (iv) a sequence as shown in SEQ ID NO: 7; (v) a sequence having one or more amino acid substitutions, deletions or additions relative to the sequence as shown in SEQ ID NO: 7; or (vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 7.

In a preferred embodiment, the substitution in (ii) or (v) is a conservative substitution.

Preferably, the antibody or the antigen binding fragment thereof comprises: VH having a sequence as shown in SEQ ID NO: 2 and VL having a sequence as shown in SEQ ID NO: 7.

In a preferred embodiment, the antibody or the antigen binding fragment thereof comprises:

(a) a heavy chain variable region, comprising an amino acid sequence selected from the group consisting of: (i) a sequence as shown in SEQ ID NO: 12; (ii) a sequence having one or more amino acid substitutions, deletions or additions relative to the sequence as shown in SEQ ID NO: 12; or (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 12; and/or
(b) a light chain variable region, comprising an amino acid sequence selected from the group consisting of: (iv) a sequence as shown in SEQ ID NO: 17; (v) a sequence having one or more amino acid substitutions, deletions or additions relative to the sequence as shown in SEQ ID NO: 17; or (vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 17.

In a preferred embodiment, the substitution in (ii) or (v) is a conservative substitution.

Preferably, the antibody or the antigen binding fragment thereof comprises: VH having a sequence as shown in SEQ ID NO: 12 and VL having a sequence as shown in SEQ ID NO: 17.

In a preferred embodiment, the antibody or the antigen binding fragment thereof comprises:

(a) a heavy chain variable region, comprising an amino acid sequence selected from the group consisting of: (i) a sequence as shown in SEQ ID NO: 22; (ii) a sequence having one or more amino acid substitutions, deletions or additions relative to the sequence as shown in SEQ ID NO: 22; or (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 22; and/or

(b) a light chain variable region, comprising an amino acid sequence selected from the group consisting of: (iv) a sequence as shown in SEQ ID NO: 27; (v) a sequence having one or more amino acid substitutions, deletions or additions relative to the sequence as shown in SEQ ID NO: 27; or (vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 27.

In a preferred embodiment, the substitution in (ii) or (v) is a conservative substitution.

Preferably, the antibody or the antigen binding fragment thereof comprises: VH having a sequence as shown in SEQ ID NO: 22 and VL having a sequence as shown in SEQ ID NO: 27.

In a preferred embodiment, the antibody or the antigen binding fragment thereof comprises:

(a) a heavy chain variable region, comprising an amino acid sequence selected from the group consisting of: (i) a sequence as shown in SEQ ID NO: 32; (ii) a sequence having one or more amino acid substitutions, deletions or additions relative to the sequence as shown in SEQ ID NO: 32; or (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 32; and/or

(b) a light chain variable region, comprising an amino acid sequence selected from the group consisting of: (iv) a sequence as shown in SEQ ID NO: 37; (v) a sequence having one or more amino acid substitutions, deletions or additions relative to the sequence as shown in SEQ ID NO: 37; or (vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 37.

In a preferred embodiment, the substitution in (ii) or (v) is a conservative substitution.

Preferably, the antibody or the antigen binding fragment thereof comprises: VH having a sequence as shown in SEQ ID NO: 32 and VL having a sequence as shown in SEQ ID NO: 37.

In a preferred embodiment, the antibody or the antigen binding fragment thereof comprises:

(a) a heavy chain variable region, comprising an amino acid sequence selected from the group consisting of: (i) a sequence as shown in SEQ ID NO: 42; (ii) a sequence having one or more amino acid substitutions, deletions or additions relative to the sequence as shown in SEQ ID NO: 42; or (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 42; and/or

(b) a light chain variable region, comprising an amino acid sequence selected from the group consisting of: (iv) a sequence as shown in SEQ ID NO: 47; (v) a sequence having one or more amino acid substitutions, deletions or additions relative to the sequence as shown in SEQ ID NO: 47; or (vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 47.

In a preferred embodiment, the substitution in (ii) or (v) is a conservative substitution.

Preferably, the antibody or the antigen binding fragment thereof comprises: VH having a sequence as shown in SEQ ID NO: 42 and VL having a sequence as shown in SEQ ID NO: 47.

In a preferred embodiment, the antibody or the antigen binding fragment thereof comprises:

(a) a heavy chain variable region, comprising an amino acid sequence selected from the group consisting of: (i) a sequence as shown in SEQ ID NO: 52; (ii) a sequence having one or more amino acid substitutions, deletions or additions relative to the sequence as shown in SEQ ID NO: 52; or (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 52; and/or

(b) a light chain variable region, comprising an amino acid sequence selected from the group consisting of: (iv) a sequence as shown in SEQ ID NO: 57; (v) a sequence having one or more amino acid substitutions, deletions or additions relative to the sequence as shown in SEQ ID NO: 57; or (vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 57.

In a preferred embodiment, the substitution in (ii) or (v) is a conservative substitution.

Preferably, the antibody or the antigen binding fragment thereof comprises: VH having a sequence as shown in SEQ ID NO: 52 and VL having a sequence as shown in SEQ ID NO: 57.

In a preferred embodiment, the antibody or the antigen binding fragment thereof comprises: (a) a heavy chain variable region, comprising an amino acid sequence selected from the group consisting of: (i) a sequence as shown in SEQ ID NO: 62; (ii) a sequence having one or more amino acid substitutions, deletions or additions relative to the sequence as shown in SEQ ID NO: 62; or (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 62; and/or

(b) a light chain variable region, comprising an amino acid sequence selected from the group consisting of: (iv) a sequence as shown in SEQ ID NO: 67; (v) a sequence having one or more amino acid substitutions, deletions or additions relative to the sequence as shown in SEQ ID NO: 67; or (vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 67.

In a preferred embodiment, the substitution in (ii) or (v) is a conservative substitution.

Preferably, the antibody or the antigen binding fragment thereof comprises: VH having a sequence as shown in SEQ ID NO: 62 and VL having a sequence as shown in SEQ ID NO: 67.

In a preferred embodiment, the antibody or the antigen binding fragment thereof comprises:

(a) a heavy chain variable region, comprising an amino acid sequence selected from the group consisting of: (i) a sequence as shown in SEQ ID NO: 72; (ii) a sequence having one or more amino acid substitutions, deletions or additions relative to the sequence as shown in SEQ ID NO: 72; or (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 72; and/or
(b) a light chain variable region, comprising an amino acid sequence selected from the group consisting of: (iv) a sequence as shown in SEQ ID NO: 77; (v) a sequence having one or more amino acid substitutions, deletions or additions relative to the sequence as shown in SEQ ID NO: 77; or (vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 77.

In a preferred embodiment, the substitution in (ii) or (v) is a conservative substitution.

Preferably, the antibody or the antigen binding fragment thereof comprises: VH having a sequence as shown in SEQ ID NO: 72 and VL having a sequence as shown in SEQ ID NO: 77.

In a preferred embodiment, the antibody or the antigen binding fragment thereof comprises:

(a) a heavy chain variable region, comprising an amino acid sequence selected from the group consisting of: (i) a sequence as shown in SEQ ID NO: 82; (ii) a sequence having one or more amino acid substitutions, deletions or additions relative to the sequence as shown in SEQ ID NO: 82; or (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 82; and/or
(b) a light chain variable region, comprising an amino acid sequence selected from the group consisting of: (iv) a sequence as shown in SEQ ID NO: 87; (v) a sequence having one or more amino acid substitutions, deletions or additions relative to the sequence as shown in SEQ ID NO: 87; or (vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 87.

In a preferred embodiment, the substitution in (ii) or (v) is a conservative substitution.

Preferably, the antibody or the antigen binding fragment thereof comprises: VH having a sequence as shown in SEQ ID NO: 82 and VL having a sequence as shown in SEQ ID NO: 87.

In a preferred embodiment, the antibody or the antigen binding fragment thereof comprises:

(a) a heavy chain variable region, comprising an amino acid sequence selected from the group consisting of: (i) a sequence as shown in SEQ ID NO: 92; (ii) a sequence having one or more amino acid substitutions, deletions or additions relative to the sequence as shown in SEQ ID NO: 92; or (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 92; and/or
(b) a light chain variable region, comprising an amino acid sequence selected from the group consisting of: (iv) a sequence as shown in SEQ ID NO: 97; (v) a sequence having one or more amino acid substitutions, deletions or additions relative to the sequence as shown in SEQ ID NO: 97; or (vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 97.

In a preferred embodiment, the substitution in (ii) or (v) is a conservative substitution.

Preferably, the antibody or the antigen binding fragment thereof comprises: VH having a sequence as shown in SEQ ID NO: 92 and VL having a sequence as shown in SEQ ID NO: 97.

In a preferred embodiment, the antibody or the antigen binding fragment thereof comprises:

(a) a heavy chain variable region, comprising an amino acid sequence selected from the group consisting of: (i) a sequence as shown in SEQ ID NO: 102; (ii) a sequence having one or more amino acid substitutions, deletions or additions relative to the sequence as shown in SEQ ID NO: 102; or (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 102; and/or
(b) a light chain variable region, comprising an amino acid sequence selected from the group consisting of: (iv) a sequence as shown in SEQ ID NO: 107; (v) a sequence having one or more amino acid substitutions, deletions or additions relative to the sequence as shown in SEQ ID NO: 107; or (vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 107.

Preferably, the substitution in (ii) or (v) is a conservative substitution.

Preferably, the antibody or the antigen binding fragment thereof comprises: VH having a sequence as shown in SEQ ID NO: 102 and VL having a sequence as shown in SEQ ID NO: 107.

In a preferred embodiment, the antibody or the antigen binding fragment thereof comprises:

(a) a heavy chain variable region, comprising an amino acid sequence selected from the group consisting of: (i) a sequence as shown in SEQ ID NO: 112; (ii) a sequence having one or more amino acid substitutions, deletions or additions relative to the sequence as shown in SEQ ID NO: 112; or (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 112; and/or
(b) a light chain variable region, comprising an amino acid sequence selected from the group consisting of: (iv) a sequence as shown in SEQ ID NO: 117; (v) a sequence having one or more amino acid substitutions, deletions or additions relative to the sequence as shown in SEQ ID NO: 117; or (vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 117.

Preferably, the substitution in (ii) or (v) is a conservative substitution.

Preferably, the antibody or the antigen binding fragment thereof comprises: VH having a sequence as shown in SEQ ID NO: 112 and VL having a sequence as shown in SEQ ID NO: 117.

In a preferred embodiment, the antibody or the antigen binding fragment thereof comprises:

(a) a heavy chain variable region, comprising an amino acid sequence selected from the group consisting of: (i) a sequence as shown in SEQ ID NO: 122; (ii) a sequence having one or more amino acid substitutions, deletions or additions relative to the sequence as shown in SEQ ID NO: 122; or (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 122; and/or
(b) a light chain variable region, comprising an amino acid sequence selected from the group consisting of: (iv) a sequence as shown in SEQ ID NO: 127; (v) a sequence having one or more amino acid substitutions, deletions or additions relative to the sequence as shown in SEQ ID NO: 127; or (vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 127.

Preferably, the substitution in (ii) or (v) is a conservative substitution.

Preferably, the antibody or the antigen binding fragment thereof comprises: VH having a sequence as shown in SEQ ID NO: 122 and VL having a sequence as shown in SEQ ID NO: 127.

In a preferred embodiment, the antibody or the antigen binding fragment thereof comprises:

(a) a heavy chain variable region, comprising an amino acid sequence selected from the group consisting of: (i) a sequence as shown in SEQ ID NO: 132; (ii) a sequence having one or more amino acid substitutions, deletions or additions relative to the sequence as shown in SEQ ID NO: 132; or (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 132; and/or

(b) a light chain variable region, comprising an amino acid sequence selected from the group consisting of: (iv) a sequence as shown in SEQ ID NO: 137; (v) a sequence having one or more amino acid substitutions, deletions or additions relative to the sequence as shown in SEQ ID NO: 137; or (vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 137.

Preferably, the substitution in (ii) or (v) is a conservative substitution.

Preferably, the antibody or the antigen binding fragment thereof comprises: VH having a sequence as shown in SEQ ID NO: 132 and VL having a sequence as shown in SEQ ID NO: 137.

In a preferred embodiment, the antibody or the antigen binding fragment thereof comprises:

(a) a heavy chain constant region of a human immunoglobulin or a variant thereof, the variant having one or more amino acid substitutions, deletions or additions relative to a sequence from which the variant is derived; and
(b) a light chain constant region of a human immunoglobulin or a variant thereof, the variant having conservative substitutions of up to 20 amino acids relative to a sequence from which the variant is derived.

In a preferred embodiment, the heavy chain constant region is an IgG heavy chain constant region, more preferably an IgG1, IgG2, IgG3 or IgG4 heavy chain constant region, further preferably, a human IgG1 or human IgG4 heavy chain constant region.

In a preferred embodiment, the light chain constant region is a κ light chain constant region.

In a preferred embodiment, the antibody or the antigen binding fragment thereof includes an antigen binding fragment which is selected from the group consisting of Fab, Fab', (Fab')$_2$, Fv, disulfide-bonded Fv, scFv, a diabody and a single-domain antibody; and/or, the antibody is a mouse-derived antibody, a chimeric antibody, a humanized antibody, a bispecific antibody or a multispecific antibody; and more preferably, the antibody is a fully humanized antibody.

A second aspect of the present invention provides a chimeric antigen receptor-T cell, comprising the aforementioned antibody or the antigen binding fragment thereof.

In a preferred embodiment, the heavy chain variable region and the light chain variable region in the antibody or the antigen binding fragment thereof are combined in series or in parallel.

A third aspect of the present invention provides an isolated nucleic acid molecule, which encodes the aforementioned antibody or the antigen binding fragment thereof, or a heavy chain variable region and/or a light chain variable region thereof.

Preferably, the nucleic acid molecule sequence encoding the heavy chain variable region is as shown in any one of SEQ ID NO: 1, 11, 21, 31, 41, 51, 61, 71, 81, 91, 101, 111, 121 or 131; and preferably, the nucleic acid molecule sequence encoding the light chain variable region is as shown in any one of SEQ ID NO: 6, 16, 26, 36, 46, 56, 66, 76, 86, 96, 106, 116, 126 or 136.

A fourth aspect of the present invention further provides a vector, comprising the aforementioned isolated nucleic acid molecule.

In a preferred embodiment, the vector is a cloning vector or an expression vector.

More preferably, the vector is a virus.

Further preferably, a virus skeleton of the virus vector is derived from a modified or engineered vaccinia virus Tian Tan strain, a vaccinia virus New York strain, a vaccinia virus Copenhagen strain, a vaccinia virus canary strain, a vaccinia virus Ankara strain, an adenovirus vector, an adeno-associated virus vector, a herpes simplex virus vector, a varicella-zoster virus vector, a respiratory syncytial virus, a Semliki forest virus, an EB virus, a cytomegalovirus, a human Herpesvirus 6, a smallpox virus, a molluscum contagiosum virus, a sheep orf virus, a reovirus, a rotavirus, an enterovirus, a Seneca virus, a poliovirus, a coxsackievirus, a rhinovirus, a hepatitis A virus, a foot and mouth disease virus, a togavirus, an alphavirus, an Eastern equine encephalitis virus, a Sindbis virus, a rubella virus, a coronavirus, a flavivirus, a hepatitis C virus, a Japanese Encephalitis virus, a St. Louis encephalitis virus, a Murray Valley fever virus, a yellow fever virus, a West Nile virus, a Zika virus, a dengue virus, an Ebola virus, a Marburg virus, an arenavirus, a Lassa fever virus, a lymphocytes Choriomeningitis virus, a Pichinde virus, a Junin virus, Machupo virus, a Hantaan virus, a Rift Valley fever virus, a Paramyxovirus, a human parainfluenza virus, a Mumps virus, a simian virus 5, a measles virus, a vesicular stomatitis virus, a rabies virus, a respiratory syncytial virus, an orthomyxovirus, an influenza A virus, an influenza B virus, an influenza C virus, a hepatitis D virus, a simian immunodeficiency virus, a human immunodeficiency virus 1, a human immunodeficiency virus 2, a Rous sarcoma virus, a human T-cell leukemia virus 1, a simian foamy virus, a hepatitis B virus, hepatitis E virus, a human papillomavirus, or a polyomavirus. Still more preferably, the virus vector is a recombinant adenovirus vector, a recombinant poxvirus vector or a recombinant lentivirus vector.

Yet more preferably, the vector is the cloning vector AbVec hIgKappa or the cloning vector AbVec hIgG1.

A fifth aspect of the present invention further provides a host cell, comprising the aforementioned isolated nucleic acid molecule or vector.

In a preferred embodiment, the host cell is prokaryotic or eukaryotic; more preferably, the host cell is selected from an E. coli cell, a yeast cell, a mammalian cell or other cell suitable for the preparation of an antibody or an antigen binding fragment, or a multispecific antibody; further preferably, the host cell is a mammalian cell; still further preferably, the host cell is a cell derived from human beings, mouse, sheep, horse, dog or cat; and most preferably, the host cell is a 293 cell or CHO cell.

A sixth aspect of the present invention provides a preparation method for the aforementioned antibody or the antigen binding fragment thereof, including culturing the aforementioned host cell under a condition that allows the expression of the aforementioned antibody or the antigen binding fragment thereof; and recycling the antibody or the antigen binding fragment thereof from the cultured host cell culture.

A sixth aspect of the present invention provides a bispecific or multispecific molecule, comprising the aforementioned antibody or the antigen binding fragment thereof.

In a preferred embodiment, the bispecific or multispecific molecule specifically binds to CD276 protein and additionally specifically binds to one or more other targets.

In a preferred embodiment, the bispecific or multispecific molecule further comprises at least one molecule, preferably, a second antibody with a second binding specificity for a second target.

In a preferred embodiment, the bispecific or multispecific molecule further comprises other antibody that specifically binds to an epitope of CD276 protein or an antigen binding fragment thereof.

In a preferred embodiment, the bispecific or multispecific molecule is a bispecific T-cell engager.

Preferably, the bispecific T-cell engager comprises an amino acid sequence selected from the group consisting of:

(i) a sequence as shown in any one of SEQ ID NOs: 312, 314, 316, and 318;
(ii) a sequence having one or more amino acid substitutions, deletions or additions relative to the sequence as shown in any one of SEQ ID NOs: 312, 314, 316, and 318; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in any one of SEQ ID NOs: 312, 314, 316, and 318.

A seventh aspect of the present invention provides an immunoconjugate, comprising the aforementioned antibody or the antigen binding fragment thereof, and a therapeutic agent conjugated to the antibody or the antigen binding fragment thereof.

In a preferred embodiment, the therapeutic agent is selected from a cytotoxic agent.

In a preferred embodiment, the therapeutic agent is selected from an alkylating agent, a mitotic inhibitor, an anti-tumor antibiotic, an antimetabolite, a topoisomerase inhibitor, a tyrosine kinase inhibitor, a radionuclide agent, and any combination thereof.

In a preferred embodiment, the immunoconjugate is an antibody-drug conjugate.

An eighth aspect of the present invention provides a pharmaceutical composition, comprising the aforementioned antibody or the antigen binding fragment thereof, the aforementioned bispecific or multispecific molecule or the aforementioned immunoconjugate, and a pharmaceutically acceptable vector and/or excipient.

In a preferred embodiment, the pharmaceutical composition further comprises an additional pharmaceutically active agent.

In a preferred embodiment, the additional pharmaceutically active agent is a drug with anti-tumor activity; more preferably, the pharmaceutically active agent is an alkylating agent, a mitotic inhibitor, an anti-tumor antibiotic, an antimetabolite, a topoisomerase inhibitor, a tyrosine kinase inhibitor, a radionuclide agent, a radiosensitizing agent, an anti-angiogenic agent, a cytokine, a molecularly targeted drug, an immune checkpoint inhibitor or an oncolytic virus.

In a preferred embodiment, the antibody or the antigen binding fragment thereof, the bispecific or multispecific molecule or the immunoconjugate, and the additional pharmaceutically active agent are provided as separate components or as components of the same composition.

A ninth aspect of the present invention provides a kit, containing the aforementioned antibody or the antigen binding fragment thereof.

In a preferred embodiment, the antibody or the antigen binding fragment thereof carries a detectable label, a radionuclide, a fluorescent dye, a luminescent substance or biotin; more preferably, the detectable label is an enzyme, further preferably horseradish peroxidase; and more preferably, the luminescent substance is a chemiluminescent substance.

In a preferred embodiment, the kit further contains a second antibody which specifically recognizes the aforementioned antibody or the antigen binding fragment thereof.

In a preferred embodiment, the second antibody further comprises a detectable label, a radionuclide, a fluorescent dye, a luminescent substance or biotin; more preferably, the detectable label is an enzyme, further preferably horseradish peroxidase; and more preferably, the luminescent substance is a chemiluminescent substance.

A tenth aspect of the present invention provides a chimeric antigen receptor, comprising an antigen binding domain of the aforementioned antibody or the antigen binding fragment thereof.

In a preferred embodiment, the antigen binding domain comprises a heavy chain variable region and a light chain variable region of the aforementioned antibody or the antigen binding fragment thereof.

In a preferred embodiment, the antigen binding domain is scFv.

In a preferred embodiment, the chimeric antigen receptor comprises the antigen binding fragment of the aforementioned antibody.

In a preferred embodiment, the chimeric antigen receptor is expressed by an immune effector cell.

More preferably, the immune effector cell is a T cell.

An eleventh aspect of the present invention provides an isolated nucleic acid molecule, which encodes the aforementioned chimeric antigen receptor.

A twelfth aspect of the present invention provides a vector which comprises the aforementioned isolated nucleic acid molecule; and in a preferred embodiment, the vector is used for the preparation of a chimeric antigen receptor-T cell.

A thirteenth aspect of the present invention further provides a host cell, comprising the aforementioned isolated nucleic acid molecule or the aforementioned vector.

In a preferred embodiment, the host cell is an immune effector cell.

More preferably, the immune effector cell is a T cell or an NK cell.

In a preferred embodiment, the host cell is a chimeric antigen receptor-T cell.

A fourteenth aspect of the present invention further provides use of the aforementioned antibody or the antigen binding fragment thereof, or the aforementioned bispecific or multispecific molecule, or the aforementioned immunoconjugate, or the aforementioned pharmaceutical composition, or the aforementioned chimeric antigen receptor, or the aforementioned host cell in the preparation of a medication for the prevention and/or treatment of a tumor in a subject.

In the preferred embodiment, the subject is human.

In a preferred embodiment, the medication further comprises an additional pharmaceutically active agent.

In a preferred embodiment, the additional pharmaceutically active agent is a drug with anti-tumor activity; more preferably, the pharmaceutically active agent is an alkylating agent, a mitotic inhibitor, an anti-tumor antibiotic, an antimetabolite, a topoisomerase inhibitor, a tyrosine kinase inhibitor, a radionuclide agent, a radiosensitizing agent, an anti-angiogenic agent, a cytokine, a molecularly targeted drug, an immune checkpoint inhibitor or an oncolytic virus.

In a preferred embodiment, the tumor is selected from B-cell lymphoma; T-cell lymphoma; melanoma; prostatic cancer; renal cell carcinoma; sarcoma; glioma, preferably high-grade glioma; blastoma, preferably neuroblastoma; osteosarcoma; plasmacytoma; histiocytoma; pancreatic cancer; breast cancer; lung cancer, preferably small cell lung cancer and non-small cell lung cancer; gastric cancer; liver cancer; colon cancer; rectal cancer; esophageal cancer; colorectal cancer; hematopoietic system cancer; testicular cancer; cervical cancer; ovarian cancer; bladder cancer; squamous cell carcinoma; adenocarcinoma; AIDS-related lymphoma; bladder cancer; brain cancer; nervous system cancer; head and neck cancer; head and neck squamous cell carcinoma; Hodgkin's lymphoma; non-Hodgkin's lymphoma; or a condition predisposing to hematologic malignancies.

A fifteenth aspect of the present invention provides a method for the prevention and/or treatment of a tumor. The method comprises: administering to a subject in need a therapeutically effective amount of the aforementioned antibody or the antigen binding fragment thereof, or the aforementioned bispecific or multispecific molecule, or the aforementioned immunoconjugate, or the aforementioned pharmaceutical composition, or the aforementioned chimeric antigen receptor, or the aforementioned host cell.

In the preferred embodiment, the subject is human.

In the preferred embodiment, the method further comprises: administering to a subject in need an additional pharmaceutically active agent.

In a preferred embodiment, the additional pharmaceutically active agent is a drug with anti-tumor activity; more preferably, the pharmaceutically active agent is an alkylating agent, a mitotic inhibitor, an anti-tumor antibiotic, an antimetabolite, a topoisomerase inhibitor, a tyrosine kinase inhibitor, a radionuclide agent, a radiosensitizing agent, an anti-angiogenic agent, a cytokine, a molecularly targeted drug, an immune checkpoint inhibitor or an oncolytic virus.

In a preferred embodiment, the tumor is selected from B-cell lymphoma; T-cell lymphoma; melanoma; prostatic cancer; renal cell carcinoma; sarcoma; glioma, preferably high-grade glioma; blastoma, preferably neuroblastoma; osteosarcoma; plasmacytoma; histiocytoma; pancreatic cancer; breast cancer; lung cancer, preferably small cell lung cancer and non-small cell lung cancer; gastric cancer; liver cancer; colon cancer; rectal cancer; esophageal cancer; colorectal cancer; hematopoietic system cancer; testicular cancer; cervical cancer; ovarian cancer; bladder cancer;

squamous cell carcinoma; adenocarcinoma; AIDS-related lymphoma; bladder cancer; brain cancer; nervous system cancer; head and neck cancer; head and neck squamous cell carcinoma; Hodgkin's lymphoma; non-Hodgkin's lymphoma; or a condition predisposing to hematologic malignancies.

The present invention has the following beneficial effects: the humanized antibody of the present invention that recognizes and binds to human CD276 has the activity of neutralizing CD276 and compared with the existing CD276 antibody, shows a good tumor cell killing effect in a tumor cell killing assay. Through a target mRNA electroporation experiment, it is found that its mRNA can also be stably expressed in T cells for a long time, and it can also be used for the preparation of CAR-T cells to exert an anti-tumor effect. Therefore, the antibody of the present invention or the antigen binding fragment thereof will effectively improve the anti-tumor efficacy and has important practical significance.

It should be understood that the above various technical features of the present invention and the various technical features specifically described below (e.g., in the examples) may be combined with each other within the scope of the present invention, thereby constituting new or preferred technical solutions. Due to limited space, they will not elaborated herein one by one.

## BRIEF DESCRIPTION OF THE DRAWINGS

The embodiments of the present invention will be described in detail below with reference to the accompanying drawings, in which:

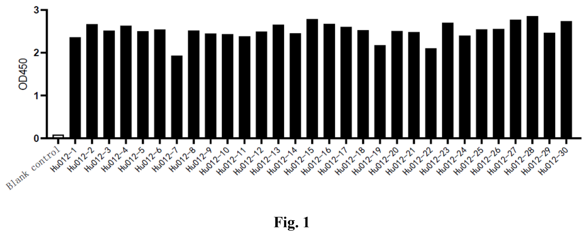

Fig. 1 shows the result of an ELISA-based screening assay for the binding of clone supernatants to antigen CD276. Fig. 1 shows that the 30 clones of the present application can specifically bind to CD276.

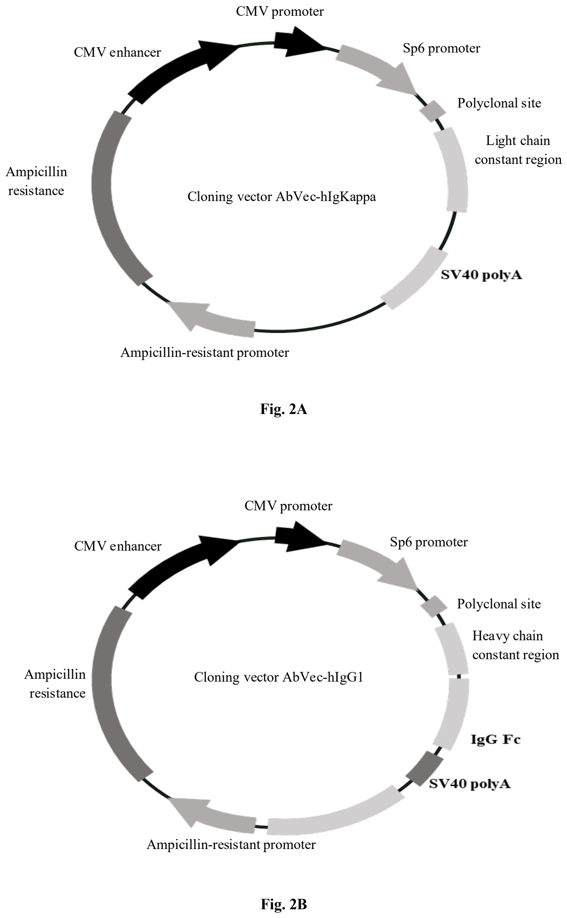

Figs. 2A to 2B are the maps of antibody expression vector constructs used in the present invention, wherein Fig. 2A is the map of an antibody expression vector construct used in the present invention - the cloning vector AbVec-hIgKappa (GenBank: FJ475056.1); and Fig. 2B is the map of an antibody expression vector construct used in the present invention - the cloning vector AbVec-hIgG1 (GenBank: FJ475055.1).

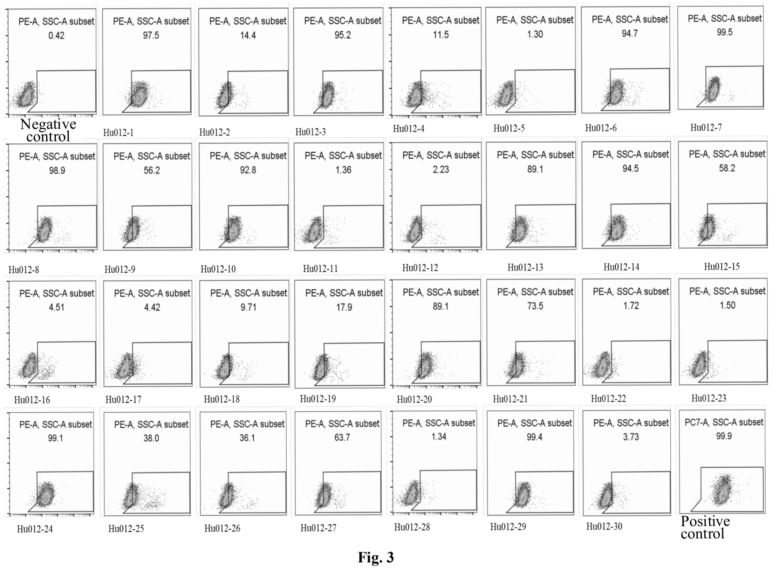

Fig. 3 shows the flow cytometric data of affinity analysis for the binding of anti-CD276 antibodies of the present invention to NCI-H292 cell line. As shown in Fig. 3, the anti-CD276 antibodies Hu012-1, Hu012-3, Hu012-6, Hu012-7, Hu012-8, Hu012-9, Hu012-10, Hu012-13, Hu012-14, Hu012-15, Hu012-20, Hu012-21, Hu012-24, Hu012-27 and Hu012-29 of the present invention may all bind to the target cells, wherein the antibodies Hu012-1, Hu012-7, Hu012-8 and Hu012-24 of the present invention have a high affinity.

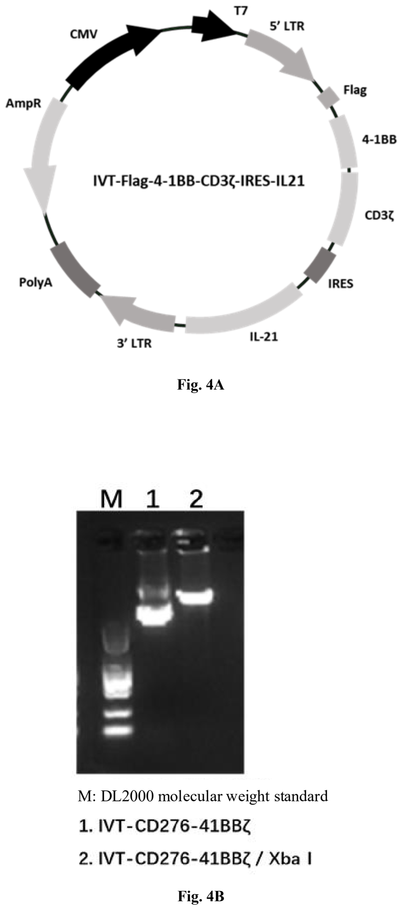

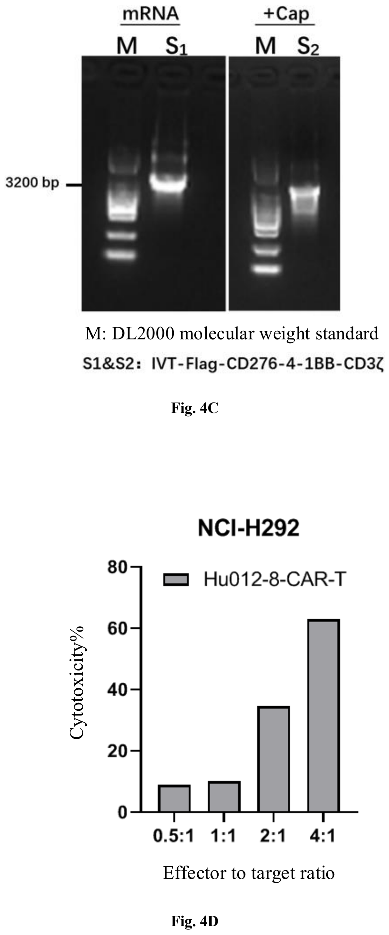

Figs. 4A to 4D shows the test results of *in-vitro* activity for the CAR-T cells prepared from the candidate antibody Hu012-8 of the present invention. Fig. 4A is the map of the eukaryotic expression vector plasmid IVT-Flag-41BB-CD3ζ-IRES-IL21-PolyA as an mRNA transcription template; Fig. 4B is the linearization result of the template vector; Fig. 4C is the electrophoresis analysis result of RNA obtained by *in-vitro* transcription; and Fig. 4D is the *in-vitro* killing result of NCI-H292 tumor cell line by the CAR-T targeting CD276 (the sequence of Hu012-8) prepared by this mRNA electroporation. The CAR-T cells prepared from Hu012-8 have killing activity against NCI-H292 tumor cell line *in vitro,* indicating that they can mediate an anti-tumor effect *in vitro.*

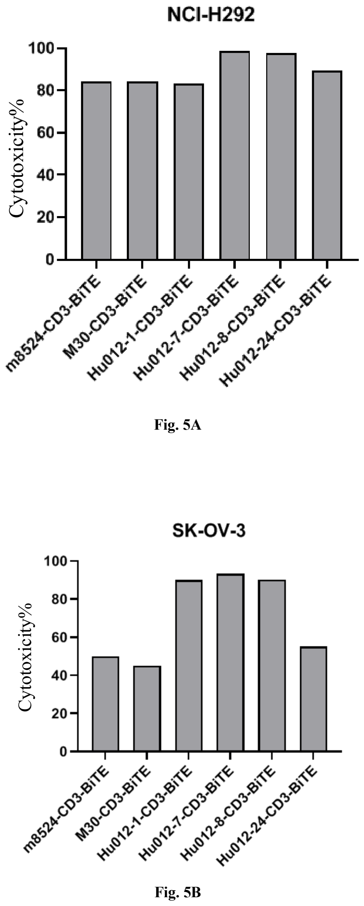

Figs. 5A to 5B show that the BiTE proteins prepared from the sequences of candidate antibodies Hu012-1, Hu012-7, Hu012-8, and Hu012-24 of the present invention exhibit a relatively higher *in-vitro* T cell-mediated broad-spectrum anti-tumor activity, compared to the BiTE proteins prepared from the sequences of m8524 and M30 antibodies as positive controls, wherein Fig. 5A shows the *in-vitro* T cell-mediated killing results for NCI-H292 tumor cell line, and Fig. 5B shows the *in-vitro* T cell-mediated killing results for SK-OV-3 tumor cell line.

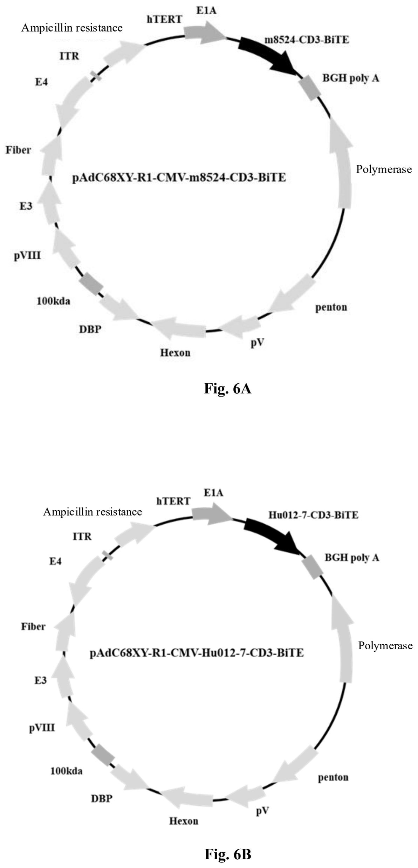

Figs. 6A to 6B are the schematic diagrams of construction of the recombinant adenovirus vectors of the present invention, wherein Fig. 6A is the schematic diagram of construction of the recombinant adenovirus vector pAdC68XY-R1-CMV-m8524-CD3-BiTE of the present invention; and Fig. 6B is the schematic diagram of construction of the recombinant adenovirus vector pAdC68XY-R1-CMV-Hu012-7-CD3-BiTE of the present invention.

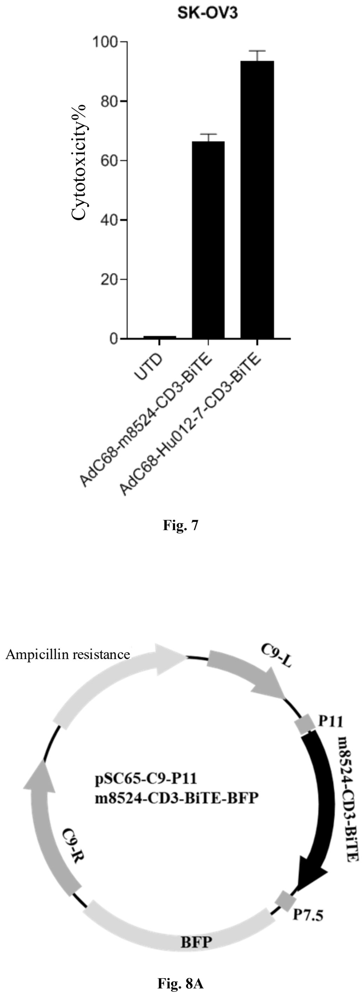

FIG. 7 shows the killing effects of the recombinant adenoviruses of the present invention on human ovarian cancer cells (SKOV3). As shown in Fig. 7, in the human ovarian cancer cells, the recombinant adenovirus carrying P11-Hu012-7-CD3-BiTE, which expresses the αCD276-CD3-BiTE protein, can mediate an efficient killing of tumor cells.

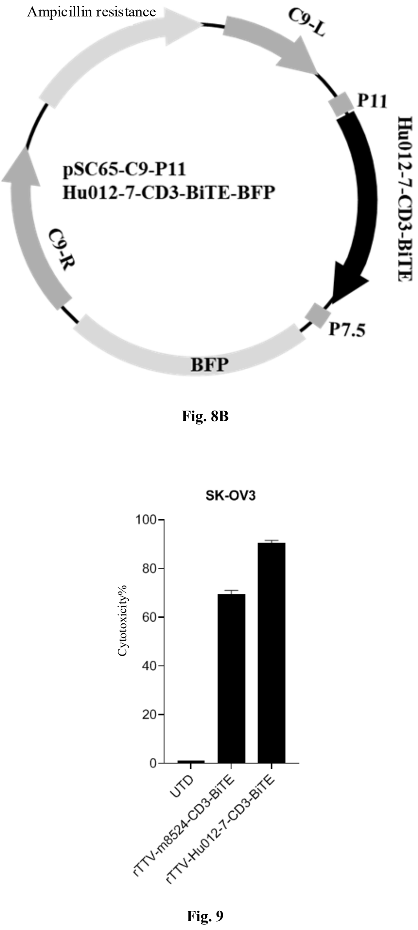

Figs. 8A and 8B are the schematic diagrams of construction of the recombinant poxvirus vectors of the present invention, wherein Fig. 8A is the schematic diagram of construction of the recombinant poxvirus vector pSC65-C9-P11-m8524-CD3-BiTE, and Fig. 8B is the schematic diagram of construction of the recombinant poxvirus vector pSC65-C9-P11-Hu012-7-CD3-BiTE.

Fig. 9 shows the killing effects of the recombinant poxviruses carrying P11-m8524-CD3-BiTE and P11-Hu012-7-CD3-BiTE on human ovarian cancer cells (SKOV3). As shown in FIG. 9, in the human ovarian cancer cells, the recombinant poxvirus carrying P11-Hu012-7-CD3-BiTE, which expresses the $\alpha$CD276-CD3-BiTE protein, can mediate an efficient killing of tumor cells.

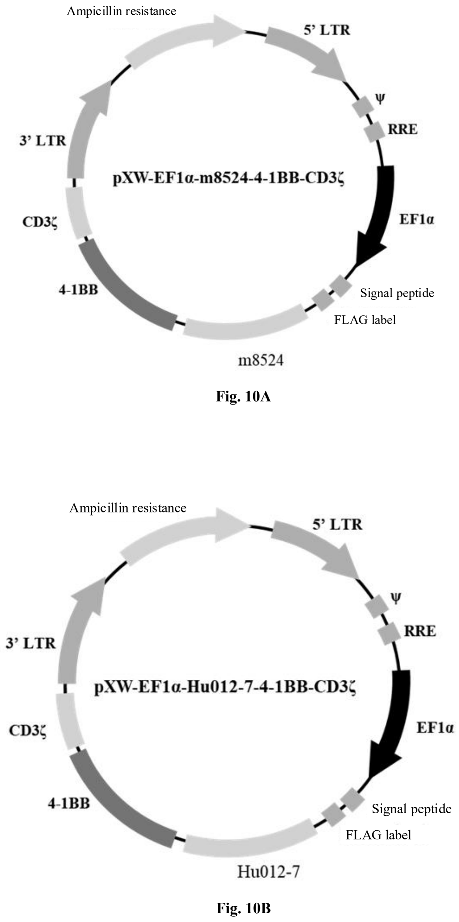

FIG. 10 is the schematic diagrams of construction of the recombinant lentivirus vectors of the present invention, wherein Fig. 10A is the schematic diagram of construction of the recombinant lentivirus vector pXW-EF1$\alpha$-m8524-4-1BB-CD3$\zeta$, and Fig. 10B is the schematic diagram of construction of the recombinant lentivirus vector pXW-EF1$\alpha$-Hu012-7-4-1BB-CD3$\zeta$.

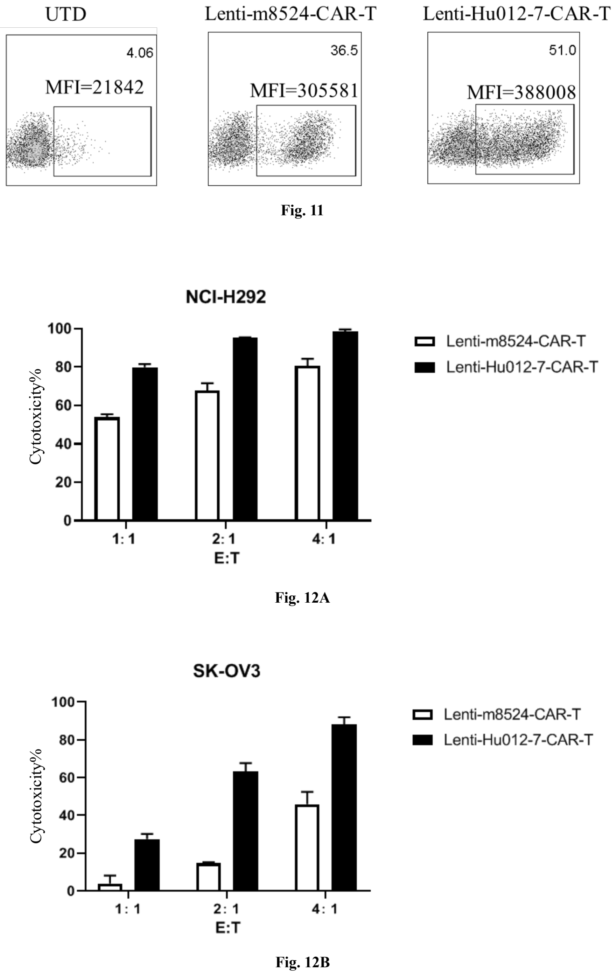

Fig. 11 shows that the chimeric antigen receptor of CD276 can be highly expressed on the cell membranes after the T cells are infected with the lentiviruses carrying EF1$\alpha$-m8524-4-1BB-CD3$\zeta$ and EF1$\alpha$-Hu012-7-4-1BB-CD3$\zeta$.

Figs. 12A and 12B show that the lentivirus CAR T cells carrying EF1$\alpha$-m8524-4-1BB-CD3$\zeta$ and EF1$\alpha$-Hu012-7-4-1BB-CD3$\zeta$ can mediate efficient killing of NCI-H292 and SK-OV3, wherein Fig. 12A shows that the lentivirus CAR T cells carrying EF1$\alpha$-m8524-4-1BB-CD3$\zeta$ and EF1$\alpha$-Hu012-7-4-1BB-CD3$\zeta$ can mediate efficient killing of NCI-H292, and Fig. 12B shows that the lentivirus CAR T cells carrying EF1$\alpha$-m8524-4-1BB-CD3$\zeta$ and EF1$\alpha$-Hu012-7-4-1BB-CD3$\zeta$ can mediate efficient killing of SKOV3.

## PREFERRED MODES FOR CARRYING OUT THE INVENTION

The following examples are used to illustrate the present invention, but not to limit the scope of the present invention.
The following description of the present invention is for the purpose of illustrating a plurality of embodiments of the present application. Therefore, the specific embodiments discussed here should not be construed as limiting the scope of the present application. A person skilled in the art can easily derive a variety of equivalent modalities, variations, and modifications without deviating from the scope of the present application, and it should be understood that such equivalent embodiments are included in the scope of the present invention. All documents cited in the present application, including public publications, patents and patent applications, are incorporated by reference in their entireties.

### Definition and description of terms

The terms "CD276" and "B7-H3" herein may be used interchangeably. CD276 is a type I transmembrane protein as a member of B7 immune co-stimulatory and co-inhibitory family. Its ligand is still unclear.
The term "bispecific T-cell engager (BiTE)" herein represents a class of bispecific antibodies with significant anti-tumor effects, which can target and activate autologous T cells to kill tumor cells. BiTE consists of two single chain variable fragments (scFv) which are connected in series by a flexible fusion linker. One scFv recognizes the T-cell surface protein CD3$\epsilon$, while the other scFv recognizes specific antigens on the surfaces of tumor cells. This structure and specific protein binding ability of BiTE allow it to physically bridge T cells to tumor cells to form a T cell-BiTE-tumor cell complex, induce immune synapse formation, stimulate T-cell activation, and produce cytokines that kill tumors.
The term "chimeric antigen receptor-T cell (CAR-T)" herein is a novel immunotherapy targeting specific antigens on the surfaces of tumor cells.
The term "monospecific" herein refers to having one or more binding sites, each of which binds to the same epitope of the same antigen.
The term "multispecific" herein refers to having at least two antigen binding sites, each of which binds to different epitopes of the same antigen or to different epitopes of different antigens. Thus, the terms such as "bispecific", "trispecific" and "tetraspecific" refer to the number of different epitopes to which an antibody/antigen binding molecule can bind.
The term "Kabat numbering system" herein generally refers to the immunoglobulin alignment and numbering system proposed by Elvin A. Kabat (see, e.g., Kabat et al., Sequences of Proteins of Immunological Interest, 5th Ed. Public Health Service, National Institutes of Health, Bethesda, Md., 1991).
The term "Chothia numbering system" herein generally refers to the immunoglobulin numbering system proposed by Chothia et al., which is a classical rule for identifying the boundaries of CDR regions based on the positions of structural loop regions (see, e.g., Chothia & Lesk (1987) J. Mol. Biol. 196: 901-917; Chothia et al. (1989) Nature 342: 878-883).
The term "IMGT numbering system" herein generally refers to the international ImMunoGeneTics® information system established in 1989 by Marie-Paule Lefranc (University of Montpellier and CNRS). Lefranc, et al. introduced a new standardized numbering system for all protein sequences of the immunoglobulin superfamily, including variable domains from antibody light and heavy chains and T-cell receptor chains from different species. Their numbering scheme is based on the amino acid sequence alignment of germline V genes (see, e.g., MP Lefranc (1999) Nucleic Acids Res. 27: 209-12; MP Lefranc (2015) Nucleic Acids Res. 43: D413-22).

The term "humanized antibody" herein refers to a non-humanized antibody that has been genetically engineered, and its amino acid sequence has been modified to improve the homology with a sequence of the humanized antibody. Generally speaking, all or part of CDR regions of a humanized antibody is derived from a non-humanized antibody (donor antibody), and all or part of non-CDR regions (e.g., a variable region FR and/or constant region) is derived from a humanized immunoglobulin (receptor antibody). The humanized antibody generally retains or partially retains the expected properties of a donor antibody, including but not limited to, antigen specificity, affinity, reactivity, ability to increase the immune cell activity, ability to enhance immune responses, etc.

The term "fully humanized antibody" herein refers to an antibody having a variable region in which both FR and CDR are derived from human germline immunoglobulin sequences. In addition, if an antibody comprises a constant region, the constant region is also derived from human germline immunoglobulin sequences. The fully humanized antibody herein may comprise amino acid residues (e.g., mutations introduced by random or site-specific mutagenesis *in vitro* or by somatic mutations *in vivo*) which are not encoded by human germline immunoglobulin sequences. However, the term "fully humanized antibody" herein is not intended to include antibodies in which CDR sequences derived from a germline of another mammalian species (e.g., mouse) have been transplanted to human framework sequences.

The term "conservative amino acids" herein generally refers to amino acids that belong to the same class or have similar characteristics (e.g., charge, side chain size, hydrophobicity, hydrophilicity, main chain conformation, and rigidity). The following six groups are the examples of amino acids that are considered to be conservative substitutions for each other:

1) alanine (A), serine (S), and threonine (T);
2) aspartic acid (D), and glutamic acid (E);
3) asparagine (N), and glutamine (Q);
4) arginine (R), lysine (K), and histidine (H);
5) isoleucine (I), leucine (L), methionine (M), and valine (V); and
6) phenylalanine (F), tyrosine (Y), and tryptophan (W).

In the present invention, "sequence identity" between two polypeptide sequences generally indicates a percentage of identical amino acids between the sequences. "Sequence identity" indicates a percentage of amino acids substituted with the same amino acids. Methods for evaluating the degree of sequence identity between amino acids or nucleotides are known to those skilled in the art. For example, the amino acid sequence identity is generally measured using sequence analysis software. For example, the BLAST program of the NCBI database may be used to determine the identity.

**Example 1: Construction and screening of native human antibody phage display library**

CD276 antigen: the human recombinant CD276 protein was purchased from Beijing Sino Biological Technology Co., Ltd., catalog number: 11188-H08H-B.

The screening of the CD276 fully humanized antibody was commissioned by Chengdu Renyu Biotechnology Co., Ltd.

A total of three rounds of screening were performed, and the clones obtained in the third round were selected for ELISA to screen for ELISA positive clones. After sequencing analysis and ELISA binding, 30 positive clones that could bind to CD276 protein were screened. The sequences of 14 clones were selected to construct full-length antibodies for further experiments. The specific implementation method was as follows:

1.1 Sequencing and analysis of positive clones

After the initial screening, 96 positive clones that could bind to CD276 protein were numbered, 2 $\mu$L of bacterial solution was pipetted into 2 mL of 2 $\times$ YT medium (17 g of peptone, 10 g of yeast extract, and 5 g of sodium chloride, added with water to 1000 mL), and cultured at 37°C, 220 rpm overnight; and the plasmids were extracted for the first-generation sequencing. Antibody gene-consensus fasta files were generated from the sequencing results through integration of the original AB1 files, and alignment and removal of non-antibody gene sequences using SeqMan. Subsequently, the DNA sequences were translated into amino acid sequences through MEGA6, terminator and unconventional sequences, etc. were found through the amino acid sequences, and the fasta files of the amino acid sequences were derived to obtain the antibody light chain and heavy chain sequences of the positive antibody clones. After the analysis and alignment, the sequences were brand new antibody sequences.

## 1.2 ELISA screening of binding affinity of clone supernatants to antigen CD276

2 μg/mL CD276 protein was coated in an ELISA plate at 4°C for 12 h; on the next day, the coating solution was discarded from the ELISA plate, and the ELISA plate was blotted dry on a sterile absorbent paper, washed 3 times with 0.05% PBST, then added with 350 μL of blocking solution, and placed at 37°C for 1 h; the clones from the third round of screening were picked into a 96-well deep-well plate containing 300 μL of 2-YT medium, and cultured at 37°C overnight, in which the supernatant contained expressed Fab; the supernatant was taken and subjected to gradient dilution prior to removal of the blocking solution, and the plate was blotted dry on a sterile absorbent paper, and washed 3 times with 0.05% PBST; the centrifuged monoclone supernatant and 2% skimmed milk powder were added to the corresponding ELISA wells in a ratio of 1: 1 at 100 μL/well, and the plate was allowed to stand at 37°C for 1 h; and the supernatant was removed, and the plate was washed 5 times with 0.1% PBST, followed by detection by using HRP-labeled goat anti-human Fab as a second antibody (goat anti-human-HRP, ThermoFisher, 31482, with a dilution of 1: 6000). The higher the OD450 signal value was, the stronger the affinity was. The results were shown in Fig. 1. In the ELISA assay, the Fabs of the 30 antibodies all showed a good affinity activity. There were 29 antibodies having OD450 values higher than 2.0, which were more than 20 times compared to that of the background, showing a higher affinity activity.

## Example 2 Construction, expression, and purification of full-length antibodies

### 2.1 Plasmid preparation

From the strains containing the antibody obtained by the screening, PCR amplification was used to obtain the light and heavy chain variable region fragments of the antibodies. The cloning vector AbVec-hIgKappa (GenBank: FJ475056.1) or the cloning vector AbVee-hIgG1 (GenBank: FJ475055.1), i.e., the eukaryotic expression vector plasmids containing the light and heavy chain constant region fragments were constructed respectively by a homologous recombination method. The maps of the plasmids were shown in Fig. 2A and Fig. 2B, respectively. The complete antibody light and heavy chain full-length genes were formed.

The constructed vectors containing the antibody light and heavy chain full-length genes were transformed into *E. coli* TOP10 (Weidi, catalog number: DL1010S) respectively, and cultured at 37°C overnight. The endotoxin-free antibody light and heavy chain plasmids were obtained for eukaryotic expression by performing plasmid extraction with an endotoxin-free plasmid extraction kit (OMEGA, catalog number: D6950-01).

### 2.2 Expression of candidate antibodies

The candidate antibodies Hu012-1, Hu012-2, Hu012-3, Hu012-4, Hu012-5, Hu012-6, Hu012-7, Hu012-8, Hu012-9, Hu012-10, Hu012-11, Hu012-12, Hu012-13, Hu012-14, Hu012-15, Hu012-16, Hu012-17, Hu012-18, Hu012-19, Hu012-20, Hu012-21, Hu012-22, Hu012-23, Hu012-24, Hu012-25, Hu012-26, Hu012-27, Hu012-28, Hu012-29, and Hu012-30 and the control antibodies M30 (having a sequence as shown in SEQ ID NO: 309, described in U.S. patent application publication US2013/0078234A1, which is incorporated herein by reference in its entirety) and m8524 (having a sequence as shown in SEQ ID NO: 310, described in U.S. patent application publication US2017/0369585A1, which is incorporated herein by reference in its entity) were expressed by an Expi293 transient expression system (Thermo Fisher, Catalog No. A1435101).

The specific method was as follows: on the day of transfection, the cell density was confirmed to be around 7E6 to 1E7 viable cells/mL and the cell viability rate was greater than 98%, at which point the cells were adjusted to a final concentration of $6 \times 10E6$ cells/mL with a fresh Expi293 expression medium which was pre-warmed to 37°C. The target plasmids were diluted with Expi293™ Expression Medium which was pre-cooled to 4°C (10 μg of plasmids were added to 1 mL of the medium), and meanwhile, a FectoPro (Polyplus, Catalog number: PT-116-001) transfection reagent was diluted with Expi293TM Expression Medium. They were then mixed in equal volumes, and homogenized by pipetting gently to prepare an Expi293™ Expression Medium/plasmid DNA mixed solution, which was incubated for 15 min at room temperature, slowly added to a prepared cell suspension with gentle shaking, and finally placed in a cell culture shaker under a condition of 37°C and 5% $CO_2$. After 18 to 22 h of transfection, 0.6 μL/mL FectoPRO booster was added to the culture solution. The shake flask was placed on a shaker for further culture under a condition of 37°C and 5% $CO_2$. On Day 5 after the transfection, Expi293™ Expression Medium of the same volume was added slowly and the cell suspension was mixed gently and uniformly at the same time. After 7 to 15 days of transfection, the cell culture supernatant in which the target protein was expressed was centrifuged at a high speed of 4000 g for 10 min to remove cell debris. Accordingly, an antibody expression supernatant was obtained.

2.3 Purification of candidate antibodies

A Protein G agarose column (GE, Catalog number: 28903134) was used to perform affinity purification on the antibodies in the expression supernatants.

The specific method was as follows: centrifugation at 200 g was performed for 20 seconds to remove a protective solution (20% ethanol) in the Protein G agarose column, and 600 $\mu$L of PBS buffer was then added, and centrifugation at 200 g was performed for 20 seconds to wash the residual protective solution in the Protein G agarose column. 600 $\mu$L of PBS buffer was added, and mixed well. The mixed Protein G agarose was added to the expression supernatant, and incubated by overturning in a four-dimensional shaker at 4°C for 6 h. At end of the incubation, the expression supernatant was removed by centrifugation at 4°C, 1000 g for 5 min. The Protein G agarose was transferred to the column, and added with 600 $\mu$L of PBS buffer to wash the Protein G agarose followed by removal of PBS buffer by centrifugation at 200 g for 20 seconds. A total of 3 washes were performed. A target protein was eluted with 400 $\mu$L of 100 mM glycine (pH2.9), and the eluate was centrifuged at 200 g for 20 seconds. The eluate was then neutralized with 10 $\mu$L of 1 M Tris-HCl (pH9.0). The neutralization was repeated once. The neutralized eluate was ultrafiltered through a 30 kD ultrafiltration concentration tube (Millipore, catalog number: UFC901096). The resulting protein was exchanged into PBS buffer with a dilution factor of > 1000.

2.4 Determination of antibody concentrations

The relative molecular weights and purities of the 30 candidate antibodies Hu012-1, Hu012-2, Hu012-3, Hu012-4, Hu012-5, Hu012-6, Hu012-7, Hu012-8, Hu012-9, Hu012-10, Hu012-11, Hu012-12, Hu012-13, Hu012-14, Hu012-15, Hu012-16, Hu012-17, Hu012-18, Hu012-19, Hu012-20, Hu012-21, Hu012-22, Hu012-23, Hu012-24, Hu012-25, Hu012-26, Hu012-27, Hu012-28, Hu012-29, and Hu012-30 and the control antibodies M30 and m8524 were detected by using SDS-PAGE. The concentrations of the purified antibody proteins were determined using a validated ultra-micro-volume spectrophotometer (Thermo Fisher, NanoDrop One C). A value obtained by dividing the determined A280 value by a theoretical extinction coefficient of the antibody was used as an antibody concentration value for the follow-up study. After passing the quality inspection, these antibodies were aliquoted and stored at -80°C.

**Example 3: Testing for specific binding of candidate antibodies to CD276-expressing target cells**

The affinity of specific binding of the candidate antibodies to CD276-expressing target cells was detected by flow cytometry. The flow cytometry was performed using the fluorescently labeled antibody PE/Cyanine7 anti-human CD276 (B7-H3) (BioLegend, Catalog Number: 351007). NCI-H292 cells highly expressed CD276 protein, so these cells were set as the target cells.

The specific method was as follows:

3.1 Culture of NCI-H292 cells in cell culture flask

The medium was an RPMI medium (comprising 10% FBS, and 1% penicillin/streptomycin). After being cultured in a 37°C, 5% $CO_2$ incubator for 48 h, the supernatant was discarded. The cells were collected by digestion with 0.25% trypsin. The cells were then centrifuged at 800 g for 3 min.

3.2 CD276 antibody-specific flow cytometric staining

The supernatant was discarded, and the cells were resuspended in 2% FBS washing buffer (1E6/mL). The number of cells in each group was 1E5. The positive control fluorescent antibody PE/Cyanine7 anti-human CD276 (B7-H3) and the candidate antibody of the present invention were added respectively, and incubated at room temperature for 15 min. The supernatant was discarded after centrifugation at 800 g for 3 min, and the cells were washed with 200 $\mu$L of 2% FBS washing buffer. The supernatant was discarded after centrifugation. 200 $\mu$L of 2% FBS washing buffer was added to the control group for resuspension. A 200-fold diluted fluorescently labeled second antibody IgG-Fc-PE (BioLegend, Catalog number: 409304) was added to the candidate antibody group, with a final volume of 50 $\mu$L, and the cells were continued to be incubated at room temperature in the dark for 15 min. The supernatant was discarded after centrifugation at 800 g for 3 min. The cells were resuspended with 200 $\mu$L of 2% FBS washing buffer, and then detected on the instrument together with the control group. The higher the signal value was, the stronger the affinity was.

The results of specific binding of the candidate antibodies to CD276-expressing target cells were shown in Fig. 3. The candidate antibodies Hu012-1, Hu012-3, Hu012-6, Hu012-7, Hu012-8, Hu012-9, Hu012-10, Hu012-13, Hu012-14, Hu012-15, Hu012-20, Hu012-21, Hu012-24, Hu012-27 and Hu012-29 may all bind to the target cells, among which the antibodies Hu012-1, Hu012-7, Hu012-8 and Hu012-24 of the present invention bound to the target cells with a positive rate close to 100%, and had a high affinity. The antibodies were of IgG1 subtype. Further, Table 1 gave information on these antibodies.

Table 1 Antibody information

| Antibody No. | Encoding nucleotide sequence | Amino acid sequence | Analysis mode | CDR1 | CDR2 | CDR3 |
|---|---|---|---|---|---|---|
| Hu012-1 | VH-DNA | VH-AA | IMGT | VH-CDR1 | VH-CDR2 | VH-CDR3 |
| | | | | SEQ ID NO: 3 | SEQ ID NO: 4 | SEQ ID NO: 5 |
| | | | Kabat | VH-CDR1 | VH-CDR2 | VH-CDR3 |
| | | | | SEQ ID NO: 141 | SEQ ID NO: 142 | SEQ ID NO: 143 |
| | SEQ ID NO: 1 | SEQ ID NO: 2 | Chothia | VH-CDR1 | VH-CDR2 | VH-CDR3 |
| | | | | SEQ ID NO: 144 | SEQ ID NO: 145 | SEQ ID NO: 146 |
| | VL-DNA | VL-AA | IMGT | VL-CDR1 | VL-CDR2 | VL-CDR3 |
| | | | | SEQ ID NO: 8 | SEQ ID NO: 9 | SEQ ID NO: 10 |
| | | | Kabat | VL-CDR1 | VL-CDR2 | VL-CDR3 |
| | | | | SEQ ID NO: 147 | SEQ ID NO: 148 | SEQ ID NO: 149 |
| | SEQ ID NO: 6 | SEQ ID NO: 7 | Chothia | VL-CDR1 | VL-CDR2 | VL-CDR3 |
| | | | | SEQ ID NO: 150 | SEQ ID NO: 151 | SEQ ID NO: 152 |
| Hu012-3 | VH-DNA | VH-AA | IMGT | VH-CDR1 | VH-CDR2 | VH-CDR3 |
| | | | | SEQ ID NO: 13 | SEQ ID NO: 14 | SEQ ID NO: 15 |
| | | | Kabat | VH-CDR1 | VH-CDR2 | VH-CDR3 |
| | | | | SEQ ID NO: 153 | SEQ ID NO: 154 | SEQ ID NO: 155 |
| | SEQ ID NO: 11 | SEQ ID NO: 12 | Chothia | VH-CDR1 | VH-CDR2 | VH-CDR3 |
| | | | | SEQ ID NO: 156 | SEQ ID NO: 157 | SEQ ID NO: 158 |
| | VL-DNA | VL-AA | IMGT | VL-CDR1 | VL-CDR2 | VL-CDR3 |
| | | | | SEQ ID NO: 18 | SEQ ID NO: 19 | SEQ ID NO: 20 |
| | | | Kabat | VL-CDR1 | VL-CDR2 | VL-CDR3 |
| | | | | SEQ ID NO: 159 | SEQ ID NO: 160 | SEQ ID NO: 161 |
| | SEQ ID NO: 16 | SEQ ID NO: 17 | Chothia | VL-CDR1 | VL-CDR2 | VL-CDR3 |
| | | | | SEQ ID NO: 162 | SEQ ID NO: 163 | SEQ ID NO: 164 |
| Hu012-6 | VH-DNA | VH-AA | IMGT | VH-CDR1 | VH-CDR2 | VH-CDR3 |
| | | | | SEQ ID NO: 23 | SEQ ID NO: 24 | SEQ ID NO: 25 |
| | | | Kabat | VH-CDR1 | VH-CDR2 | VH-CDR3 |
| | | | | SEQ ID NO: 165 | SEQ ID NO: 166 | SEQ ID NO: 167 |
| | SEQ ID NO: 21 | SEQ ID NO: 22 | Chothia | VH-CDR1 | VH-CDR2 | VH-CDR3 |
| | | | | SEQ ID NO: 168 | SEQ ID NO: 169 | SEQ ID NO: 170 |
| | VL-DNA | VL-AA | IMGT | VL-CDR1 | VL-CDR2 | VL-CDR3 |
| | | | | SEQ ID NO: 28 | SEQ ID NO: 29 | SEQ ID NO: 30 |
| | | | Kabat | VL-CDR1 | VL-CDR2 | VL-CDR3 |
| | | | | SEQ ID NO: 171 | SEQ ID NO: 172 | SEQ ID NO: 173 |
| | SEQ ID NO: 26 | SEQ ID NO: 27 | Chothia | VL-CDR1 | VL-CDR2 | VL-CDR3 |
| | | | | SEQ ID NO: 174 | SEQ ID NO: 175 | SEQ ID NO: 176 |

(continued)

| Antibody No. | Encoding nucleotide sequence | Amino acid sequence | Analysis mode | CDR1 | CDR2 | CDR3 |
|---|---|---|---|---|---|---|
| Hu012-7 | VH-DNA | VH-AA | IMGT | VH-CDR1 | VH-CDR2 | VH-CDR3 |
| | | | | SEQ ID NO: 33 | SEQ ID NO: 34 | SEQ ID NO: 35 |
| | | | Kabat | VH-CDR1 | VH-CDR2 | VH-CDR3 |
| | | | | SEQ ID NO: 177 | SEQ ID NO: 178 | SEQ ID NO: 179 |
| | SEQ ID NO: 31 | SEQ ID NO: 32 | Chothia | VH-CDR1 | VH-CDR2 | VH-CDR3 |
| | | | | SEQ ID NO: 180 | SEQ ID NO: 181 | SEQ ID NO: 182 |
| | VL-DNA | VL-AA | IMGT | VL-CDR1 | VL-CDR2 | VL-CDR3 |
| | | | | SEQ ID NO: 38 | SEQ ID NO: 39 | SEQ ID NO: 40 |
| | | | Kabat | VL-CDR1 | VL-CDR2 | VL-CDR3 |
| | | | | SEQ ID NO: 183 | SEQ ID NO: 184 | SEQ ID NO: 185 |
| | SEQ ID NO: 36 | SEQ ID NO: 37 | Chothia | VL-CDR1 | VL-CDR2 | VL-CDR3 |
| | | | | SEQ ID NO: 186 | SEQ ID NO: 187 | SEQ ID NO: 188 |
| Hu012- | VH-DNA | VH-AA | IMGT | VH-CDR1 | VH-CDR2 | VH-CDR3 |
| | | | | SEQ ID NO: 43 | SEQ ID NO: 44 | SEQ ID NO: 45 |
| | | | Kabat | VH-CDR1 | VH-CDR2 | VH-CDR3 |
| | | | | SEQ ID NO: 189 | SEQ ID NO: 190 | SEQ ID NO: 191 |
| | SEQ ID NO: 41 | SEQ ID NO: 42 | Chothia | VH-CDR1 | VH-CDR2 | VH-CDR3 |
| | | | | SEQ ID NO: 192 | SEQ ID NO: 193 | SEQ ID NO: 194 |
| | VL-DNA | VL-AA | IMGT | VL-CDR1 | VL-CDR2 | VL-CDR3 |
| | | | | SEQ ID NO: 48 | SEQ ID NO: 49 | SEQ ID NO: 50 |
| | | | Kabat | VL-CDR1 | VL-CDR2 | VL-CDR3 |
| | | | | SEQ ID NO: 195 | SEQ ID NO: 196 | SEQ ID NO: 197 |
| | SEQ ID NO: 46 | SEQ ID NO: 47 | Chothia | VL-CDR1 | VL-CDR2 | VL-CDR3 |
| | | | | SEQ ID NO: 198 | SEQ ID NO: 199 | SEQ ID NO: 200 |
| Hu012-9 | VH-DNA | VH-AA | IMGT | VH-CDR1 | VH-CDR2 | VH-CDR3 |
| | | | | SEQ ID NO: 53 | SEQ ID NO: 54 | SEQ ID NO: 55 |
| | | | Kabat | VH-CDR1 | VH-CDR2 | VH-CDR3 |
| | | | | SEQ ID NO: 201 | SEQ ID NO: 202 | SEQ ID NO: 203 |
| | SEQ ID NO: 51 | SEQ ID NO: 52 | Chothia | VH-CDR1 | VH-CDR2 | VH-CDR3 |
| | | | | SEQ ID NO: 204 | SEQ ID NO: 205 | SEQ ID NO: 206 |
| | VL-DNA | VL-AA | IMGT | VL-CDR1 | VL-CDR2 | VL-CDR3 |
| | | | | SEQ ID NO: 58 | SEQ ID NO: 59 | SEQ ID NO: 60 |
| | | | Kabat | VL-CDR1 | VL-CDR2 | VL-CDR3 |
| | | | | SEQ ID NO: 207 | SEQ ID NO: 208 | SEQ ID NO: 209 |
| | SEQ ID NO: 56 | SEQ ID NO: 57 | Chothia | VL-CDR1 | VL-CDR2 | VL-CDR3 |
| | | | | SEQ ID NO: 210 | SEQ ID NO: 211 | SEQ ID NO: 212 |

(continued)

| Antibody No. | Encoding nucleotide sequence | Amino acid sequence | Analysis mode | CDR1 | CDR2 | CDR3 |
|---|---|---|---|---|---|---|
| Hu012-10 | VH-DNA | VH-AA | IMGT | VH-CDR1 | VH-CDR2 | VH-CDR3 |
| | | | | SEQ ID NO: 63 | SEQ ID NO: 64 | SEQ ID NO: 65 |
| | | | Kabat | VH-CDR1 | VH-CDR2 | VH-CDR3 |
| | | | | SEQ ID NO: 213 | SEQ ID NO: 214 | SEQ ID NO: 215 |
| | SEQ ID NO: 61 | SEQ ID NO: 62 | Chothia | VH-CDR1 | VH-CDR2 | VH-CDR3 |
| | | | | SEQ ID NO: 216 | SEQ ID NO: 217 | SEQ ID NO: 218 |
| | VL-DNA | VL-AA | IMGT | VL-CDR1 | VL-CDR2 | VL-CDR3 |
| | | | | SEQ ID NO: 68 | SEQ ID NO: 69 | SEQ ID NO: 70 |
| | | | Kabat | VL-CDR1 | VL-CDR2 | VL-CDR3 |
| | | | | SEQ ID NO: 219 | SEQ ID NO: 220 | SEQ ID NO: 221 |
| | SEQ ID NO: 66 | SEQ ID NO: 67 | Chothia | VL-CDR1 | VL-CDR2 | VL-CDR3 |
| | | | | SEQ ID NO: 222 | SEQ ID NO: 223 | SEQ ID NO: 224 |
| Hu012-13 | VH-DNA | VH-AA | IMGT | VH-CDR1 | VH-CDR2 | VH-CDR3 |
| | | | | SEQ ID NO: 73 | SEQ ID NO: 74 | SEQ ID NO: 75 |
| | | | Kabat | VH-CDR1 | VH-CDR2 | VH-CDR3 |
| | | | | SEQ ID NO: 225 | SEQ ID NO: 226 | SEQ ID NO: 227 |
| | SEQ ID NO: 71 | SEQ ID NO: 72 | Chothia | VH-CDR1 | VH-CDR2 | VH-CDR3 |
| | | | | SEQ ID NO: 228 | SEQ ID NO: 229 | SEQ ID NO: 230 |
| | VL-DNA | VL-AA | IMGT | VL-CDR1 | VL-CDR2 | VL-CDR3 |
| | | | | SEQ ID NO: 78 | SEQ ID NO: 79 | SEQ ID NO: 80 |
| | | | Kabat | VL-CDR1 | VL-CDR2 | VL-CDR3 |
| | | | | SEQ ID NO: 231 | SEQ ID NO: 232 | SEQ ID NO: 233 |
| | SEQ ID NO: 76 | SEQ ID NO: 77 | Chothia | VL-CDR1 | VL-CDR2 | VL-CDR3 |
| | | | | SEQ ID NO: 234 | SEQ ID NO: 235 | SEQ ID NO: 236 |
| Hu012-14 | VH-DNA | VH-AA | IMGT | VH-CDR1 | VH-CDR2 | VH-CDR3 |
| | | | | SEQ ID NO: 83 | SEQ ID NO: 84 | SEQ ID NO: 85 |
| | | | Kabat | VH-CDR1 | VH-CDR2 | VH-CDR3 |
| | | | | SEQ ID NO: 237 | SEQ ID NO: 238 | SEQ ID NO: 239 |
| | SEQ ID NO: 81 | SEQ ID NO: 82 | Chothia | VH-CDR1 | VH-CDR2 | VH-CDR3 |
| | | | | SEQ ID NO: 240 | SEQ ID NO: 241 | SEQ ID NO: 242 |
| | VL-DNA | VL-AA | IMGT | VL-CDR1 | VL-CDR2 | VL-CDR3 |
| | | | | SEQ ID NO: 88 | SEQ ID NO: 89 | SEQ ID NO: 90 |
| | | | Kabat | VL-CDR1 | VL-CDR2 | VL-CDR3 |
| | | | | SEQ ID NO: 243 | SEQ ID NO: 244 | SEQ ID NO: 245 |
| | SEQ ID NO: 86 | SEQ ID NO: 87 | Chothia | VL-CDR1 | VL-CDR2 | VL-CDR3 |
| | | | | SEQ ID NO: 246 | SEQ ID NO: 247 | SEQ ID NO: 248 |

(continued)

| Antibody No. | Encoding nucleotide sequence | Amino acid sequence | Analysis mode | CDR1 | CDR2 | CDR3 |
|---|---|---|---|---|---|---|
| Hu012-20 | VH-DNA | VH-AA | IMGT | VH-CDR1 | VH-CDR2 | VH-CDR3 |
| | | | | SEQ ID NO: 93 | SEQ ID NO: 94 | SEQ ID NO: 95 |
| | | | Kabat | VH-CDR1 | VH-CDR2 | VH-CDR3 |
| | SEQ ID NO: 91 | SEQ ID NO: 92 | | SEQ ID NO: 249 | SEQ ID NO: 250 | SEQ ID NO: 251 |
| | | | Chothia | VH-CDR1 | VH-CDR2 | VH-CDR3 |
| | | | | SEQ ID NO: 252 | SEQ ID NO: 253 | SEQ ID NO: 254 |
| | VL-DNA | VL-AA | IMGT | VL-CDR1 | VL-CDR2 | VL-CDR3 |
| | | | | SEQ ID NO: 98 | SEQ ID NO: 99 | SEQ ID NO: 100 |
| | | | Kabat | VL-CDR1 | VL-CDR2 | VL-CDR3 |
| | SEQ ID NO: 96 | SEQ ID NO: 97 | | SEQ ID NO: 255 | SEQ ID NO: 256 | SEQ ID NO: 257 |
| | | | Chothia | VL-CDR1 | VL-CDR2 | VL-CDR3 |
| | | | | SEQ ID NO: 258 | SEQ ID NO: 259 | SEQ ID NO: 260 |
| Hu012-21 | VH-DNA | VH-AA | IMGT | VH-CDR1 | VH-CDR2 | VH-CDR3 |
| | | | | SEQ ID NO: 103 | SEQ ID NO: 104 | SEQ ID NO: 105 |
| | | | Kabat | VH-CDR1 | VH-CDR2 | VH-CDR3 |
| | SEQ ID NO: 101 | SEQ ID NO: 102 | | SEQ ID NO: 261 | SEQ ID NO: 262 | SEQ ID NO: 263 |
| | | | Chothia | VH-CDR1 | VH-CDR2 | VH-CDR3 |
| | | | | SEQ ID NO: 264 | SEQ ID NO: 265 | SEQ ID NO: 266 |
| | VL-DNA | VL-AA | IMGT | VL-CDR1 | VL-CDR2 | VL-CDR3 |
| | | | | SEQ ID NO: 108 | SEQ ID NO: 109 | SEQ ID NO: 110 |
| | | | Kabat | VL-CDR1 | VL-CDR2 | VL-CDR3 |
| | SEQ ID NO: 106 | SEQ ID NO: 107 | | SEQ ID NO: 267 | SEQ ID NO: 268 | SEQ ID NO: 269 |
| | | | Chothia | VL-CDR1 | VL-CDR2 | VL-CDR3 |
| | | | | SEQ ID NO: 270 | SEQ ID NO: 271 | SEQ ID NO: 272 |
| Hu012-24 | VH-DNA | VH-AA | IMGT | VH-CDR1 | VH-CDR2 | VH-CDR3 |
| | | | | SEQ ID NO: 113 | SEQ ID NO: 114 | SEQ ID NO: 115 |
| | | | Kabat | VH-CDR1 | VH-CDR2 | VH-CDR3 |
| | SEQ ID NO: 111 | SEQ ID NO: 112 | | SEQ ID NO: 273 | SEQ ID NO: 274 | SEQ ID NO: 275 |
| | | | Chothia | VH-CDR1 | VH-CDR2 | VH-CDR3 |
| | | | | SEQ ID NO: 276 | SEQ ID NO: 277 | SEQ ID NO: 278 |
| | VL-DNA | VL-AA | IMGT | VL-CDR1 | VL-CDR2 | VL-CDR3 |
| | | | | SEQ ID NO: 118 | SEQ ID NO: 119 | SEQ ID NO: 120 |
| | | | Kabat | VL-CDR1 | VL-CDR2 | VL-CDR3 |
| | SEQ ID NO: 116 | SEQ ID NO: 117 | | SEQ ID NO: 279 | SEQ ID NO: 280 | SEQ ID NO: 281 |
| | | | Chothia | VL-CDR1 | VL-CDR2 | VL-CDR3 |
| | | | | SEQ ID NO: 282 | SEQ ID NO: 283 | SEQ ID NO: 284 |

(continued)

| Antibody No. | Encoding nucleotide sequence | Amino acid sequence | Analysis mode | CDR1 | CDR2 | CDR3 |
|---|---|---|---|---|---|---|
| Hu012-27 | VH-DNA | VH-AA | IMGT | VH-CDR1 | VH-CDR2 | VH-CDR3 |
| | | | | SEQ ID NO: 123 | SEQ ID NO: 124 | SEQ ID NO: 125 |
| | | | Kabat | VH-CDR1 | VH-CDR2 | VH-CDR3 |
| | | | | SEQ ID NO: 285 | SEQ ID NO: 286 | SEQ ID NO: 287 |
| | SEQ ID NO: 121 | SEQ ID NO: 122 | Chothia | VH-CDR1 | VH-CDR2 | VH-CDR3 |
| | | | | SEQ ID NO: 288 | SEQ ID NO: 289 | SEQ ID NO: 290 |
| | VL-DNA | VL-AA | IMGT | VL-CDR1 | VL-CDR2 | VL-CDR3 |
| | | | | SEQ ID NO: 128 | SEQ ID NO: 129 | SEQ ID NO: 130 |
| | | | Kabat | VL-CDR1 | VL-CDR2 | VL-CDR3 |
| | | | | SEQ ID NO: 291 | SEQ ID NO: 292 | SEQ ID NO: 293 |
| | SEQ ID NO: 126 | SEQ ID NO: 127 | Chothia | VL-CDR1 | VL-CDR2 | VL-CDR3 |
| | | | | SEQ ID NO: 294 | SEQ ID NO: 295 | SEQ ID NO: 296 |
| Hu012-29 | VH-DNA | VH-AA | IMGT | VH-CDR1 | VH-CDR2 | VH-CDR3 |
| | | | | SEQ ID NO: 133 | SEQ ID NO: 134 | SEQ ID NO: 135 |
| | | | Kabat | VH-CDR1 | VH-CDR2 | VH-CDR3 |
| | | | | SEQ ID NO: 297 | SEQ ID NO: 298 | SEQ ID NO: 299 |
| | SEQ ID NO: 131 | SEQ ID NO: 132 | Chothia | VH-CDR1 | VH-CDR2 | VH-CDR3 |
| | | | | SEQ ID NO: 300 | SEQ ID NO: 301 | SEQ ID NO: 302 |
| | VL-DNA | VL-AA | IMGT | VL-CDR1 | VL-CDR2 | VL-CDR3 |
| | | | | SEQ ID NO: 138 | SEQ ID NO: 139 | SEQ ID NO: 140 |
| | | | Kabat | VL-CDR1 | VL-CDR2 | VL-CDR3 |
| | | | | SEQ ID NO: 303 | SEQ ID NO: 304 | SEQ ID NO: 305 |
| | SEQ ID NO: 136 | SEQ ID NO: 137 | Chothia | VL-CDR1 | VL-CDR2 | VL-CDR3 |
| | | | | SEQ ID NO: 306 | SEQ ID NO: 307 | SEQ ID NO: 308 |

**Example 4: *In-vitro* activity test for CART cells prepared from antibody Hu012-8**

4.1 Construction of CAR vector targeting CD276 (the sequence of candidate antibody Hu012-8)

Using the constructed eukaryotic expression vector containing the antibody light and heavy chains as a template, PCR amplification was performed to obtain the antibody light and heavy chain variable region fragments. Through a homologous recombination method, the eukaryotic expression vector plasmid IVT-Flag-4-1BB-CD3ζ-IRES-IL21-PolyA containing T7 promoter and the light and heavy chain constant region fragments was constructed as shown in Fig. 4A.
The constructed vector which contained the antibody light and heavy chains and could be transcribed *in vitro* was transformed into *E. coli* Stbl3 (Weidi, Catalog number: DL1046M) and cultured at 37°C overnight. The plasmids containing the antibody light and heavy chains were extracted by an endotoxin-free plasmid extraction kit (OMEGA, D6950-01) for later use. Positive clones were picked on an LB plate and numbered. 3 μL of bacterial solution was pipetted into 3 mL of a LB anti-ampicillin medium, and cultured at 37°C, 220 rpm overnight. The plasmids were extracted for sequencing. After the sequencing results were analyzed and aligned successfully, the subsequent experiments were carried out.

4.2 *In-vitro* transcription of mRNA of CAR targeting CD276 (the sequence of candidate antibody Hu012-8)

The constructed vector was linearized by a restriction endonuclease, and the result was shown in Fig. 4B. The linearized plasmids ensured the blunt ends of double strands or overhang at 5' end. The components except T7 RNA Polymerase Mix (Novoprotein, Catalog Number: E131-01A) were shaken and mixed well, centrifuged briefly, and collected at the bottom of the tube, and stored on ice for later use.

The components shown in Table 2 were added at room temperature:

Table 2

| Components | Volume |
|---|---|
| 10×transcription buffer | 2 μL |
| Mix ATP/GTP/CTP/UTP | 1.5 μL each |
| Template DNA | X μL |
| Enzyme mixture | 1 μL |
| RNase-free water | To 20 μL |

The components were mixed gently well with a pipette, centrifuged briefly and collected, and incubated at 37°C for 2-3 h. 1 μL of DNase (TAKARA, Catalog Number: 2270A) was added to the reaction system, and incubated at 37°C for 15 min to digest the transcribed DNA template. The synthesized RNA was subjected to electrophoresis analysis, and the purification result is shown in Fig. 4C, which can be used for downstream experiments.

4.3 Purification and modification of mRNA product of CAR targeting CD276 (the sequence of candidate antibody Hu012-8)

20 μL of the product in step 4.2 was taken, and then added with 30 μL of lithium chloride solution (7.5 M) and 30 μL of RNase-free water (a final concentration of lithium chloride was kept at 2.5-2.8 M), respectively. After mixing well, the mixture was then placed at -20°C for at least 30 min. A supernatant was removed after centrifugation at 12000 rpm for 15 min, and the precipitate was collected. The precipitate was washed three times with pre-cooled 70% ethanol. The precipitate was redissolved in RNase-free water for testing.

The mRNA modification step was a capping reaction. An appropriate amount of RNA was diluted to 14 μL with RNase-free water (all the reagents used in this step were from Novoprotein, catalog number: M062-YH01). The RNA was heated at 65°C for 5 minutes, and then placed on ice for 5 minutes. 32 mM SAM was diluted to 4 mM. The reaction was performed at 37°C for 30 min. After the RNA capping was completed, the RNA was directly purified and can be used for the subsequent experiments. The components were shown in Table 3 below:

Table 3

| Components | 20 μL |
|---|---|
| Partially purified RNA | 15 μL |
| 10×capping buffer | 2 μL |
| GTP (10 mM) | 1 μL |
| SAM (4 mM) | 1 μL |
| Cap2 methyltransferase | 1 μL |
| Vaccinia capping enzyme (10 U/μL) | 1 μL |

4.4 Preparation and *in-vitro* anti-tumor activity experiment of CAR-T targeting CD276 (the sequence of candidate antibody Hu012-8)

The above mRNA concentration was adjusted to 2 μg/μL. The activated T cells were resuspended with Electroporation Buffer (Maxcyte®) at a density of 1E8/mL. The T-cell suspension was added to an OC-25×3 electroporation cup (Maxcyte®), at 20 μL/well. 1 μL of mRNA having a concentration of 2 μg/μL was added to each well. At the same time, a blank control group was set, that is, by adding only T cells, without adding mRNA. The bubbles were removed by mixing well. A program was set on an ATx™ electroporator (Maxcyte®) for electroporation. The CAR-T cells expressing a protein targeting CD276 and blank control T cells that were mocked-electroporated were obtained.

After 24 h of electroporation, an *in vitro* anti-tumor (human lung cancer cell NCI-H292, carrying a reporter gene encoding luciferase) killing activity experiment was performed. All the reagents and instruments used were purchased from Promega unless otherwise specified. The specific method was as follows: the tumor cells were plated in a 96-well plate (1E4/well), and cultured at 37°C, 5% $CO_2$ for 24 h; the medium was then removed; and the CAR-T cells and mocked-electroporated T cells after 24 h of electroporation were added at the effector to target ratios (CAR-T: target cell) of 0.5: 1, 1: 1, 2: 1, and 4: 1. After a further culture at 37°C, 5% $CO_2$ for 24 h, a supernatant was removed. 50 μL of 1x cell lysis buffer (Catalog Number: E1531) was added to each well, and shaken and incubated at room temperature for 30 min. 30 μL of luciferase assay substrate (Catalog Number: E151A) was added to each well, and color-developed for 30 seconds. The fluorescence value was detected on the instrument (GloMax®Navigator Microplate Luminometer). A cell-specific killing rate was calculated by using the reading of the mocked-electroporated T cell group as a control.

The result was shown in Fig. 4D. For the NCI-H292 tumor cell line, the targeting CD276-CAR-T, which was prepared by the electroporation using the mRNA prepared by the sequence of the candidate antibody Hu012-8 of the present invention, had a significant killing effect, which was positively correlated with the effector to target ratio. When the effector to target ratio was 4: 1, the killing efficiency was as high as more than 60%, and the mRNA-CAR-T cells showed a high killing activity.

All the cells, reagents and instruments used in Example 4 were purchased from Promega unless otherwise specified.

**Example 5: Experiments of *in-vitro* broad-spectrum anti-tumor activity mediated by BiTE prepared from candidate antibodies Hu012-1, Hu012-7, Hu012-8 and Hu012-24**

The present invention conducted an experimental validation on the *in-vitro* killing effects of activated T cells for solid tumor cells (human ovarian adenoma cells SK-OV-3 and human lung cancer cells NCI-H292 tumors, both carrying a reporter gene encoding luciferase) mediated by BiTE proteins (labeled as CD276(Hu012-1)BiTE, CD276(Hu012-7)BiTE, CD276(Hu012-8)BiTE, and CD276(Hu012-24)BiTE, respectively) prepared from the candidate antibodies Hu012-1, Hu012-7, Hu012-8 and Hu012-24 .

The CDS sequence of CD276(Hu012-1)BiTE was as shown in SEQ ID NO: 311, and the amino acid sequence thereof was as shown in SEQ ID NO: 312. The CDS sequence of CD276(Hu012-7)BiTE was as shown in SEQ ID NO: 313, and the amino acid sequence thereof was as shown in SEQ ID NO: 314. The CDS sequence of CD276(Hu012-8)BiTE was shown in SEQ ID NO: 315, and the amino acid sequence thereof was shown in SEQ ID NO: 316. The CDS sequence of CD276(Hu012-24)BiTE was as shown in SEQ ID NO: 317, and the amino acid sequence thereof was as shown in SEQ ID NO: 318.

All the reagents and instruments used were purchased from Promega unless otherwise specified. The specific method was as follows: the tumor cells were plated in a 96-well plate (1E4/well), and cultured at 37°C, 5% $CO_2$ for 24 h, and then the medium was removed; and the BiTE proteins prepared from the candidate antibodies Hu012-1, Hu012-7, Hu012-8 and Hu012-24 of the present invention were added at a dose of 2 μg/mL. The activated T cells were added to each well, wherein a ratio of the T cells to NCI-H292 cells was 1: 1, and a ratio of the T cells to SK-OV-3 cells was 4: 1. A blank control group and a non-specific killing group (only T cells were added) were set. After a further culture at 37°C, 5% $CO_2$ for 24 h, the supernatant was removed. 50 μL of 1x cell lysis buffer ((Promega, Catalog Number: E1531) was added to each well, and shaken and incubated at room temperature for 30 min. Then, 30 μL of luciferase assay substrate (Promega, Catalog Number: E151A) was added to each well and color-developed for 30 seconds. A fluorescence value was detected on the instrument (GloMax® Navigator Microplate Luminometer). The specific killing data was calculated using the value of the non-specific killing group with only T cells added as a control.

The results were shown in Fig. 5A and Fig. 5B. For these two tumor cell lines, the BiTE proteins prepared from the candidate sequences Hu012-1, Hu012-7, Hu012-8 and Hu012-24 of the present invention can mediate a high T-cell killing activity *in vitro,* and had a significantly enhanced killing effect as compared with the BiTE prepared from the control antibodies M30 and m8524. Hu012-7 and Hu0127-8 had 2.1-fold and 2.0-fold increase in killing the SK-OV3 tumor cell line, respectively, and 1.2-fold and 1.2-fold increase in killing the NCI-H292 tumor cell line, respectively, indicating that the anti-CD276 antibody sequences of the present invention have a broad-spectrum activity of mediating tumor-killing *in vitro.*

All the cells, reagents and instruments used in Example 5 were purchased from Promega unless otherwise specified.

**Example 6: Construction of recombinant adenovirus of αCD276-CD3-BiTE**

The DNA sequences of artificially synthesized CMV-m8524-CD3-BiTE and CMV-Hu012-7-CD3-BiTE were as shown in SEQ ID NO: 319 (i.e., CMV-m8524-CD3-BiTE, with a corresponding amino acid sequence as shown in SEQ ID NO: 320) and SEQ ID NO: 321 (i.e., CMV-Hu012-7-CD3-BiTE, with a corresponding amino acid sequence as shown in SEQ ID NO: 322). The chimpanzee adenovirus AdC68XY-R1 plasmid was enzymatically digested with PI-SceI (NEB, Catalog number: R0696S) at 37°C for 4 h and inactivated at 65°C for 20 min; an exogenous sequence was ligated to an enzymatically digested linearized vector (Vazyme, Catalog number: c112-02) by means of seamless cloning; and the exogenous sequence was constructed behind the E1A gene in the pAdC68 vector. The ligated product was transformed into *E. coli* Stbl2 (Weidi, Catalog number: DL1045) and grown overnight on an ampicilin-containing culture plate. Single colonies were randomly picked for PCR sequencing using specific primers. After verifying that all the sequences were correct, the endotoxin-free pAdC68XY-R1-CMV-m8524-CD3-BiTE recombinant adenovirus vector plasmid and pAdC68-XY-R1-CMV-Hu012-7-CD3-BiTE recombinant adenovirus vector plasmid were extracted after a bacterial shaking culture. The construction maps of the two plasmids were shown in Fig. 6A and Fig. 6B, respectively.

**Example 7: Packaging and amplification of recombinant oncolytic adenovirus**

7.1 Virus packaging: 2.5 $\mu$g of the two recombinant adenovirus plasmids obtained in Example 6 were linearized with PacI digestion, i.e., enzymatically digested at 37°C for 4 h, and inactivated at 65°C for 20 min. According to the instructions of Lipofectamine 3000 transfection kit, the two linearized recombinant plasmids were transfected into HEK293 cells with a confluence of 60-70% in a 6-well plate. A DNA/liposome complex was added for transfection. After 6 h of transfection, the medium was replaced with a DMEM medium containing 5% FBS and 1% diabody. The cytopathic effects were observed under a microscope every day until a large number of plaques appeared in HEK293 cells at Day 5 to Day 10, and P0-generation viruses were collected and stored in a -80°C refrigerator. Finally, the recombinant adenoviruses AdC68-m8524-CD3-BiTE and AdC68-Hu012-7-CD3-BiTE carrying CMV-m8524-CD3-BiTE and CMV-Hu012-7-CD3-BiTE were obtained.

7.2 Virus amplification: the P0-generation viruses were harvested, and repeatedly frozen and thawed three times. After centrifugation at 5000 g for 5 min, an appropriate amount of the frozen and thawed virus supernatant was taken to infect HEK293 cells, followed by continuous passage culture. The virus $TCID_{50}$ (50% tissue culture infectious dose) was measured in the HEK293 cells and calculated using the Reed-Muench method.

**Example 8: Detection for killing of SKOV3 by recombinant adenovirus of $\alpha$CD276-CD3-BiTE**

8.1 293T cells were plated in 24-well plates, at $1 \times 10^5$ cells/well. After cultured for about 24 h, when the cells adhered to the wall and spread over about 80%-90% of the entire bottom surface, AdC68-m8524-CD3-BiTE and AdC68-Hu012-7-CD3-BiTE were used to respectively infect the cells at an MOI of 0.3 for 2 h, and the 24-well plates were then placed in an incubator for further culture. The supernatants of the cells were collected after 24 h, and centrifuged at 12000 g for 5 min. The supernatants were transferred to new EP tubes and placed in a -80°C refrigerator for detection.

8.2 SK-OV3 cell plating: SK-OV3-Luc (Shanghai Sinobay Biotechnology Co., Ltd.) is a Luciferase-expressing cell line. The cells were plated in a 96-well plate at a density of $1 \times 10^4$ cells/well and cultured at 37°C overnight till cell adherence.

8.3 After 24 h, the medium was removed, and Tcells, the infection supernatants of AdC68-m8524-CD3-BiTE and AdC68-Hu012-7-CD3-BiTE and T cells, and a control without T cells were added, respectively.

8.4 Culture was performed at 37°C overnight for killing. After 24 h, the supernatant was removed, 50 $\mu$L of cell lysis buffer (Promega, Catalog number: E1531) was added to each well, and shaken and incubated for 30 min at room temperature. 30 $\mu$L of luciferase substrate (Promega, Catalog number: E151A) was added to each well. The detection was performed on the instrument (GloMax Navigator Microplate Luminometer, Promega, Steady-Glo protocol).

A formula for calculating the cell killing rate was as follows:

Cell killing rate (%) = (luciferase activity value of target cell group - luciferase activity value of experimental group)/luciferase activity value of target cell group $\times$ 100

The results were shown in Fig. 7. Both the AdC68-m8524-CD3-BiTE and AdC68-Hu012-7-CD3-BiTE recombinant adenoviruses can well mediate an *in-vitro* anti-human ovarian cancer cell effect of Tcells. AdC68-Hu012-7-CD3-BiTE mediated killing of more than 93% of tumor cells, while the control antibody adenovirus AdC68-m8524-CD3-BiTE only mediated killing of 66% of tumor cells. The killing effect mediated by AdC68-Hu012-7-CD3-BiTE exceeded the control by as much as 1.4 times.

**Example 9: Construction of recombinant poxvirus of $\alpha$CD276-CD3-BiTE**

9.1 Construction of pSC65 vectors carrying P11-m8524-CD3-BiTE and P11-Hu012-7-CD3-BiTE target genes

The DNA sequences of artificially synthesized P11-m8524-CD3-BiTE and P11-Hu012-7-CD3-BiTE were as shown in SEQ ID NO: 323 (i.e., P11-m8524-CD3-BiTE, with a corresponding amino acid sequence as shown in SEQ ID NO: 324) and SEQ ID NO: 325 (i.e., P11-Hu012-7-CD3-BiTE, with a corresponding amino acid sequence as shown in SEQ ID NO: 326), and cloned and constructed into shuttle plasmids at the vaccinia virus C9 gene site. The plasmid construction maps were shown in Fig. 8A and Fig. 8B.

9.2 Construction of recombinant vaccinia virus

9.2.1 Cell preparation: 143TK-cells were plated in a 6-well plate, at about $1 \times 10^6$ cells/well. After cultured for about 24 h, when the cells are adherent and spread over the entire bottom surface, the next operation was performed.

9.2.2 Vaccinia virus incubation: the cells were infected with wild-type poxvirus having a titer of viral solution of 0.0125/3 (virus PFU/cell count, PFU: plaque-forming unit), incubated in a 37°C incubator for 1 h, and then taken out; a supernatant was aspirated away; and the cells were rinsed with 1 mL of PBS and then added into 1 mL of complete medium.

9.2.3 Plasmid transfection: 143TK-cells were transfected with the above shuttle plasmids pSC65-C9-P11-m8524-CD3-BiTE and pSC65-C9-P11-Hu012-7-CD3-BiTE. The cells were cultured in a 37°C incubator for about 48 h, and the specific time depended on the condition of cytopathic effect.

9.2.4 A $2 \times$ DMEM maintenance medium (containing 2% PS and 4% FBS) was prepared for virus plaque plating, added with 2% pre-warmed low-melting-point agarose and then added with X-gal (a final concentration of 200 $\mu$g/mL).

9.2.5 The supernatant was aspirated away from the 6-well plate and the mixture for plaque plating was added to the 6-well plate, with 1 mL per well. Then, the 6-well plate was carefully placed in a 4°C refrigerator to promote solidification. The low-melting-point agarose was solidified, and then transferred to a 37°C incubator and invertedly cultured.

9.2.6 The recombinant vaccinia viruses rTTV-C9-P11-m8524-CD3-BiTE and rTTV-C9-P11-Hu012-7-CD3-BiTE which have blue florescence were picked and added into a 500 $\mu$L complete medium. The viruses were repeatedly frozen and thawed at -80°C for more than three times to release as much virus as possible.

9.2.7 143TK-cells were plated in a 6-well plate, at about $1 \times 10^6$ cells/well. After cultured for about 24 h, the cells were adherent and spread over the entire bottom surface.

9.2.8 The virus solution was pipetted in an EP tube repeatedly so that it was scattered completely. 9.2.9 The complete medium was replaced with a maintenance medium, then added with a virus solution having the blue fluorescence and incubated in a 37°C incubator for 3-4 h.

9.2.10. Applying screening pressure: the virus solution was placed and incubated in a 37°C incubator for about 48 h with a BrdU working concentration of 50 $\mu$g/mL, and plaque plating was performed according to the virus plaque formation condition. This purification process needs to be performed at least 5 times.

9.2.11 A small scale amplification of recombinant vaccinia virus was then performed. 143TK-cells were plated on the 6-well plate, at $1 \times 10^6$ cells/well, and the cells covered approximately 100% of the bottom area of the well plate when in use.

9.2.12 The medium in the wells was replaced with 2 mL of maintenance medium before virus inoculation. The purified virus solution having blue fluorescence was pipetted repeatedly until the virus solution was scattered. About 100 $\mu$L of virus solution was added to each well, and incubated in a 37°C incubator for about 48 h, and the sample was collected according to the virus plaque formation condition.

9.2.13 Sample collection: 1 mL of medium supernatant was pipetted from the wells carefully. The remaining 1 mL of medium was used to sufficiently pipette the cells down and the cells were collected in an EP tube for subsequent genome extraction and used as virus seed for amplification. 9.3 Amplification and purification of recombinant vaccinia virus

9.3.1 VERO cell plating: each 10 cm dish was plated with $5 \times 10^6$ cells to ensure that the cell density reached 100% when a vaccinia virus was inoculated on the next day.

9.3.2 Before virus inoculation, a complete medium needs to be replaced with 8 mL of a maintenance medium (DMEM medium + 2% FBS + 1% PS), and the virus was inoculated into the cells in the maintenance medium, with an inoculation amount of about 0.02 MOI (MOI = virus titer PFU/cell count). A further culture was performed in a 37°C, 5% $CO_2$ incubator for about 48 h, and the sample was collected according to the virus plaque formation condition.

9.3.3 Vaccinia virus collection: 8 mL medium in the dish was discarded, 2 mL of maintenance medium was taken to pipette the remaining cells down, and the cells were collected in a 15 mL centrifuge tube.

9.3.4 After frozen storage for 24 h, the collected virus solution was frozen and thawed repeatedly twice, was subject a density gradient centrifugation with 36% sucrose solution, and centrifuged at 16000 g, 4°C for 90 min; a supernatant was carefully poured off; and the virus precipitate in the centrifuge tube was dissolved with PBS buffer, and aliquoted and stored at -80°C for the determination of the virus titer.

9.4.4 Titer determination of recombinant vaccinia virus

9.4.1 Preparation of 143TK-cells: 143TK-cells were plated in a 24-well plate, at about $2 \times 10^5$ cells/well, and the cell density reached 100% of bottom area of the 24-well plate when in use. 9.4.2 Virus dilution: the vaccinia virus solution was diluted with a maintenance medium, i.e., a 10-fold serial dilution was started at 1: 100 to a final volume of 1100 $\mu$L.

9.4.3 The complete medium in the 24-well plate was discarded, 500 $\mu$L of diluted virus solution was added to the wells, and two duplicate wells were set. The incubation was performed in a 37°C, 5% $CO_2$ incubator for about 48 h, and the plaque plating time was determined according to the virus plaque formation condition.

9.4.4 Plaque plating method: 8 mL of plaque plating medium containing 2×DMEM medium+4% FBS+2% PS and 8 mL of low-melting-point agarose which was placed also in a 37°C water bath after being melt in a boiling water bath, were mixed to obtain a mixture, and X-gal was added to the mixture to achieve a final concentration of 200 $\mu$g/mL for later use.

9.4.5 The supernatant was aspirated away from the 24-well plate. The plaque plating mixture in step 9.4.4 was intermediately added to the 24-well plate at 500 $\mu$L per well. Then, the 24-well plate was carefully placed in a 4°C refrigerator to promote solidification. The low-melting-point agarose was solidified, and then transferred to a 37°C incubator and invertedly cultured.

9.4.6 Virus plaque counting: firstly, the quantity of virus plaques was observed to determine if it showed a 10-fold decreasing trend, the quantity of blue fluorescent foci in a single-digit range in the two duplicate wells inoculated with the virus was counted subsequently, and a sum of the quantities of the blue fluorescence value in the two wells was obtained and multiplied by a reciprocal value of the corresponding dilution of the wells to obtain the titer of 1 mL of the virus solution. Finally, the recombinant poxviruses rTTV-P11-m8524-CD3-BiTE and rTTV-P11-Hu012-7-CD3-BiTE carrying P11-m8524-CD3-BiTE and P11-Hu012-7-CD3-BiTE target genes were obtained.

## Example 10: Effect of recombinant vaccinia virus of $\alpha$CD276-CD3-BiTE against ovarian cancer cells

10.1. TK143-cells were plated in a 12-well plate at $2\times10^5$ cells/well and transfected the next day after cell adherence. The cells were infected with the rTTV-P11-m8524-CD3-BiTE and rTTV-P11-Hu012-7-CD3-BiTE viruses at a multiplicity of infection (MOI) of 0.02. After the infection, the cells were placed in an incubator and cultured for 24 h, respectively. The cell supernatants were collected after the culture was completed, and the expressions of killing factors were detected.

10.2. The tumor cell killing efficiency was evaluated by a luciferase-based cytotoxicity assay. First, $1\times10^4$ SKOV3-Luc (firefly luciferase gene-modified human ovarian cancer cells) were inoculated on a 96-well flat-bottom black plate with 100 $\mu$L of medium per well, and cultured in a 37°C, 5% $CO_2$ cell incubator for 18 h. On the second day, the culture supernatant in step 10.1 was added to the corresponding wells, and the luciferase activity value of the target cells was detected using the GloMax®96 microplate luminescence detector after the co-culture was completed.

A formula for calculating the cell killing rate was as follows:

Cell killing rate (%) = (luciferase activity value of control virus infection supernatant group - luciferase activity value of experimental group)/luciferase activity value of control virus infection supernatant group $\times$ 100

The result was shown in Fig. 9. Both the recombinant vaccinia virus rTTV-P11-m8524-CD3-BiTE and the recombinant vaccinia virus rTTV-P11-Hu012-7-CD3-BiTE can well mediate *in-vitro* anti-human ovarian cancer cell effect of T cells, wherein rTTV-P11-Hu012-7-CD3-BiTE mediated killing of more than 90% of tumor cells, while the adenovirus rTTV-P11-m8524-CD3-BiTE as the control antibody mediated killing of less than 70% of tumor cells, the former was 1.3 times better than the control group.

## Example 11: Construction of lentivirus expression plasmids of CD276 chimeric antigen receptor

The nucleic acid sequences as shown in SEQ NO: 327 (i.e., EF1$\alpha$-m8524-4-1BB-CD3$\zeta$, with a corresponding amino acid sequence as shown in SEQ ID NO: 328) and SEQ ID NO: 329 (i.e., EF1$\alpha$-Hu012-7-4-1BB-CD3$\zeta$, with a corresponding amino acid sequence as shown in SEQ ID NO: 330) were synthesized by Shanghai Tsingke Biotechnology Co., Ltd. and cloned into the blank lentivirus expression plasmid pXW-EF1$\alpha$-MCS to obtain pXW-EF1$\alpha$-m8524-4-1BB-CD3$\zeta$ and pXW-EF1$\alpha$-Hu012-7-4-1BB-CD3$\zeta$ recombinant lentivirus expression plasmids. The plasmid maps were shown in Fig. 10A and Fig. 10B.

## Example 12: Packaging, concentration and titer determination of lentivirus

12.1 Packaging of lentivirus

HEK293T cell treatment: 24 h before the transfection, HEK293T cells in the logarithmic growth phase were collected, and inoculated in a 10 cm cell culture dish ($6\times10^6$ to $8\times10^6$ cells); and the cells grew in 10 mL of complete DMEM medium, and were cultured in a 37°C, 5% $CO_2$ cell incubator for 18-24 h, followed by plasmid transfection till the cell density reached 70-90%.

HEK293T cell transfection: 1 mL of basal DMEM medium was added to a 15 mL centrifge tube, and a mixed solution for transfection was prepared according to a mass ratio of lentivirus expression plasmids to packaging plasmids to envelope plasmids of 1: 3: 1, with a total plasmid amount of 15 $\mu$g/dish. 30 $\mu$L of the TurboFect transfection reagent was added according to a ratio of plasmids ($\mu$g) to a transfection reagent ($\mu$L) of 1: 2, incubated at room temperature for 15-20 min, then added to a dish plated with HEK293T cells, and continued to be cultured in a 37°C, 5% $CO_2$ cell incubator for 48 h; a virus supernatant was then collected, and centrifuged at 1000×g, 4°C for 10 min; the precipitate at the bottom of the tube was discarded; and a virus supernatant was collected.

## 12.2 Concentration of lentivirus

A 0.45 $\mu$m filter was used to further filter the viral supernatant collected by centrifugation, and a Lenti-X lentivirus concentration reagent (Clontech, Catalog number: 631232) at 1/3 of the volume of the viral supernatant was added, inverted and mixed well several times, incubated at 4°C overnight, and centrifuged at 2000×g, 4°C for 45 min. A white precipitate can be seen at the bottom of the centrifuge tube, which was the concentrated virus particles. The supernatant was discarded carefully, and the white precipitate was resuspended with a blank RPMI1640 medium at 1/50-1/100 of the volume of the original virus supernatant, aliquoted and freeze-stored at -80°C for later use.

## 12.3 Lentivirus titer determination

Jurkat T cells were inoculated at $1\times10^5$ cells/well into a 96-well U-bottom plate, and the collected lentivirus concentrate was diluted in 10-fold increments. 100 $\mu$L of virus diluent was added to the corresponding wells, added with an infection enhancer, i.e., protamine sulfate, which was adjusted to the concentration to 10 $\mu$g/mL. Centrifugation was performed at 1000×g, 32°C for 90 min for infection. After an overnight culture, the medium was replaced with a fresh RPMI1640 complete medium, followed by a further culture for 48 h; and the proportion of fluorescence-positive cells was detected by a flow cytometry. A virus titer was calculated using the following formula:

Viral titer (TU/mL) = 1×105×proportion of fluorescence-positive cells / 100 × 1000 × corresponding dilution factor.

## Example 13: Detection of expression of CD276 chimeric antigen receptor

The lentivirus expression plasmids in Example 11 and the lentiviruses carrying EF1-m8524-4-1BB-CD3$\zeta$ and EF1$\alpha$-Hu012-7-4-1BB-CD3$\zeta$ prepared by the lentivirus vector preparation method of Example 12 were added to a 48-well flat-bottom plate plated with $1\times10^6$ preactivated peripheral blood mononuclear cells at a ratio of MOI=5, and added with an infection enhancer, i.e., protamine sulfate, which was adjusted to a working concentration to 10 $\mu$g/mL. Centrifugations were performed at 1000×g, 32°C for 90 min for infection. After an overnight culture, the media were replaced with a fresh T cell growth medium for a further culture. The fresh T cell growth media were added every 2-3 days, and the cell density was adjusted to 0.5 to $2\times10^6$ cells/mL. After 6-7 days of infection, immunomagnetic beads of activated T cells were removed, the genetically engineered T cells were continued to be cultured and expanded, and the subsequent functional experiments could be performed until the cells entered a resting state (9-14 days after removal of the beads). The cells were collected after the above operations and eluted with FACS buffer once, added with 2 $\mu$g/mL of the flow cytometry antibody PE-anti-DYKDDDDK, incubated at room temperature in the dark for 20 min, eluted twice with FACS buffer after the incubation, and resuspended and mixed well with 300 $\mu$L of FACS buffer; and then, the expression of $\alpha$CD276 chimeric antigen receptor molecules were detected by a flow cytometer.

The results were shown in Fig. 11. The flow cytometry results showed that compared with the control group, Lenti-m8524-CAR-T and Lenti-Hu012-7-CAR-T were highly expressed on the T cell membranes, with a positive rate of 51% which was 1.4 times higher than 36.5% of the control group; and the mean fluorescence intensity (MFI) was also 1.3 times higher.

## Example 14: Killing of tumor cells by CD276 chimeric antigen receptors carried by lentivirus vectors

The tumor cell killing efficiency was evaluated by a luciferase-based cytotoxicity assay. First, $1\times10^4$ SK-OV3-Luc (firefly luciferase gene-modified human ovarian cancer cells) or NCI-H292-Luc (firefly luciferase gene-modified human lung cancer cells) was inoculated into a 96-well flat-bottom black plate with 100 $\mu$L of medium per well, and cultured in a 37°C, 5% $CO_2$ cell incubator for 18 h. On the second day, the genetically engineered T cells in Example 13 and non-transduced T cells cultured at the same time were added to the wells containing the target cells at a ratio of effector cells to target cells of 1: 1, 2: 1 and 4: 1, and continued to be cultured for 20 h; and a luciferase activity value of the target cells was detected by the GloMax®96 microplate luminescence detector after co-culture.

A formula for calculating the cell killing rate was as follows:

Cell killing rate (%) = (luciferase activity value of non-transduced T cell group - luciferase activity value of experimental group)/ luciferase activity value of non-transduced T cell group $\times$ 100

The results were shown in Fig. 12A and Fig. 12B. Both the control Lenti-m8524-CAR-T and Lenti-Hu012-7-CAR-T can efficiently kill the tumor cells. Under the condition that the ratio of effector cells to target cells was 4: 1, the killing activity of Lenti-Hu012-7-CAR-T was better than that of the control Lenti-m8524-CAR-T. However, when the effector to target ratio (E: T) was reduced, Lenti-Hu012-7-CAR-T still maintained a higher killing ability, especially for the SKOV3 cell line. Under the condition of low effector to target ratio of 2: 1, it still had a killing efficiency of more than 60%, while the killing efficiency of Lenti-m8524-CAR-T was less than 20%. Under the condition of low effector to target ratio of 1: 1, Lenti-Hu012-7-CAR-T still had a killing efficiency of nearly 30%, while the killing efficiency of Lenti-m8524-CAR-T was almost zero (FIG. 12B).

The above examples are exemplary, and should not be construed as limiting the present invention. A person of ordinary skill in the art can make changes, modifications, substitutions and variations to the above examples within the scope of the present invention.

## Claims

1. An antibody capable of specifically binding to CD276 protein or an antigen binding fragment thereof, the antibody or the antigen binding fragment thereof comprising:

   (a) a heavy chain variable region comprising the following three complementary determining regions:

   (i) VH CDR1, consisting of the following sequence: SEQ ID NO: 3, 141 or 144, or a sequence having one or more amino acid substitutions, deletions or additions relative to any of them;
   (ii) VH CDR2, consisting of the following sequence: SEQ ID NO: 4, 142 or 145, or a sequence having one or more amino acid substitutions, deletions or additions relative to any of them; and
   (iii) VH CDR3, consisting of the following sequence: SEQ ID NO: 5, 143 or 146, or a sequence having one or more amino acid substitutions, deletions or additions relative to any of them; and/or

   (b) a light chain variable region comprising the following three complementary determining regions:

   (iv) VL CDR1, consisting of the following sequence: SEQ ID NO: 8, 147 or 150, or a sequence having one or more amino acid substitutions, deletions or additions relative to any of them;
   (v) VL CDR2, consisting of the following sequence: SEQ ID NO: 9, 148 or 151, or a sequence having one or more amino acid substitutions, deletions or additions relative to any of them; and
   (vi) VL CDR3, consisting of the following sequence: SEQ ID NO: 10, 149 or 152, or a sequence having one or more amino acid substitutions, deletions or additions relative to any of them; preferably, the substitution in any one of (i) to (vi) is a conservative substitution;
   preferably, VH in the antibody or the antigen binding fragment thereof comprises: VH CDR1 as shown in SEQ ID NO: 3, VH CDR2 as shown in SEQ ID NO: 4, and VH CDR3 as shown in SEQ ID NO: 5; and VL in the antibody or the antigen binding fragment thereof comprises: VL CDR1 as shown in SEQ ID NO: 8, VL CDR2 as shown in SEQ ID NO: 9, and VL CDR3 as shown in SEQ ID NO: 10; or
   VH in the antibody or the antigen binding fragment thereof comprises: VH CDR1 as shown in SEQ ID NO: 141, VH CDR2 as shown in SEQ ID NO: 142, and VH CDR3 as shown in SEQ ID NO: 143; and VL in the antibody or the antigen binding fragment thereof comprises: VL CDR1 as shown in SEQ ID NO: 147, VL CDR2 as shown in SEQ ID NO: 148, and VL CDR3 as shown in SEQ ID NO: 149; or
   VH in the antibody or the antigen binding fragment thereof comprises: VH CDR1 as shown in SEQ ID NO: 144, VH CDR2 as shown in SEQ ID NO: 145, and VH CDR3 as shown in SEQ ID NO: 146; and VL in the antibody or the antigen binding fragment thereof comprises: VL CDR1 as shown in SEQ ID NO: 150, VL CDR2 as shown in SEQ ID NO: 151, and VL CDR3 as shown in SEQ ID NO: 152.

2. An antibody capable of specifically binding to CD276 protein or an antigen binding fragment thereof, the antibody or the antigen binding fragment thereof comprising:

(a) a heavy chain variable region comprising the following three complementary determining regions:

(i) VH CDR1, consisting of the following sequence: SEQ ID NO: 13, 153 or 156, or a sequence having one or more amino acid substitutions, deletions or additions relative to any of them;
(ii) VH CDR2, consisting of the following sequence: SEQ ID NO: 14, 154 or 157, or a sequence having one or more amino acid substitutions, deletions or additions relative to any of them; and
(iii) VH CDR3, consisting of the following sequence: SEQ ID NO: 15, 155 or 158, or a sequence having one or more amino acid substitutions, deletions or additions relative to any of them; and/or

(b) a light chain variable region comprising the following three complementary determining regions:

(iv) VL CDR1, consisting of the following sequence: SEQ ID NO: 18, 159 or 162, or a sequence having one or more amino acid substitutions, deletions or additions relative to any of them;
(v) VL CDR2, consisting of the following sequence: SEQ ID NO: 19, 160 or 163, or a sequence having one or more amino acid substitutions, deletions or additions relative to any of them; and
(vi) VL CDR3, consisting of the following sequence: SEQ ID NO: 20, 161 or 164, or a sequence having one or more amino acid substitutions, deletions or additions relative to any of them; preferably, the substitution in any one of (i) to (vi) is a conservative substitution;
preferably, VH in the antibody or the antigen binding fragment thereof comprises: VH CDR1 as shown in SEQ ID NO: 13, VH CDR2 as shown in SEQ ID NO: 14, and VH CDR3 as shown in SEQ ID NO: 15; and VL in the antibody or the antigen binding fragment thereof comprises: VL CDR1 as shown in SEQ ID NO: 18, VL CDR2 as shown in SEQ ID NO: 19, and VL CDR3 as shown in SEQ ID NO: 20; or
VH in the antibody or the antigen binding fragment thereof comprises: VH CDR1 as shown in SEQ ID NO: 153, VH CDR2 as shown in SEQ ID NO: 154, and VH CDR3 as shown in SEQ ID NO: 155; and VL in the antibody or the antigen binding fragment thereof comprises: VL CDR1 as shown in SEQ ID NO: 159, VL CDR2 as shown in SEQ ID NO: 160, and VL CDR3 as shown in SEQ ID NO: 161; or
VH in the antibody or the antigen binding fragment thereof comprises: VH CDR1 as shown in SEQ ID NO: 156, VH CDR2 as shown in SEQ ID NO: 157, and VH CDR3 as shown in SEQ ID NO: 158; and VL in the antibody or the antigen binding fragment thereof comprises: VL CDR1 as shown in SEQ ID NO: 162, VL CDR2 as shown in SEQ ID NO: 163, and VL CDR3 as shown in SEQ ID NO: 164.

3. An antibody capable of specifically binding to CD276 protein or an antigen binding fragment thereof, the antibody or the antigen binding fragment thereof comprising:

(a) a heavy chain variable region comprising the following three complementary determining regions:

(i) VH CDR1, consisting of the following sequence: SEQ ID NO: 23, 165 or 168, or a sequence having one or more amino acid substitutions, deletions or additions relative to any of them;
(ii) VH CDR2, consisting of the following sequence: SEQ ID NO: 24, 166 or 169, or a sequence having one or more amino acid substitutions, deletions or additions relative to any of them; and
(iii) VH CDR3, consisting of the following sequence: SEQ ID NO: 25, 167 or 170, or a sequence having one or more amino acid substitutions, deletions or additions relative to any of them; and/or

(b) a light chain variable region comprising the following three complementary determining regions:

(iv) VL CDR1, consisting of the following sequence: SEQ ID NO: 28, 171 or 174, or a sequence having one or more amino acid substitutions, deletions or additions relative to any of them;
(v) VL CDR2, consisting of the following sequence: SEQ ID NO: 29, 172 or 175, or a sequence having one or more amino acid substitutions, deletions or additions relative to any of them; and
(vi) VL CDR3, consisting of the following sequence: SEQ ID NO: 30, 173 or 176, or a sequence having one or more amino acid substitutions, deletions or additions relative to any of them; preferably, the substitution in any one of (i) to (vi) is a conservative substitution;
preferably, VH in the antibody or the antigen binding fragment thereof comprises: VH CDR1 as shown in SEQ ID NO: 23, VH CDR2 as shown in SEQ ID NO: 24, and VH CDR3 as shown in SEQ ID NO: 25; and VL in the antibody or the antigen binding fragment thereof comprises: VL CDR1 as shown in SEQ ID NO: 28, VL CDR2 as shown in SEQ ID NO: 29, and VL CDR3 as shown in SEQ ID NO: 30; or
VH in the antibody or the antigen binding fragment thereof comprises: VH CDR1 as shown in SEQ ID NO: 165, VH CDR2 as shown in SEQ ID NO: 166, and VH CDR3 as shown in SEQ ID NO: 167; and VL in the antibody or the antigen binding fragment thereof comprises: VL CDR1 as shown in SEQ ID NO: 171, VL CDR2 as shown in SEQ ID NO: 172, and VL CDR3 as shown in SEQ ID NO: 173; or

VH in the antibody or the antigen binding fragment thereof comprises: VH CDR1 as shown in SEQ ID NO: 168, VH CDR2 as shown in SEQ ID NO: 169, and VH CDR3 as shown in SEQ ID NO: 170; and VL in the antibody or the antigen binding fragment thereof comprises: VL CDR1 as shown in SEQ ID NO: 174, VL CDR2 as shown in SEQ ID NO: 175, and VL CDR3 as shown in SEQ ID NO: 176.

4. An antibody capable of specifically binding to CD276 protein or an antigen binding fragment thereof, the antibody or the antigen binding fragment thereof comprising:

(a) a heavy chain variable region comprising the following three complementary determining regions:

(i) VH CDR1, consisting of the following sequence: SEQ ID NO: 33, 177 or 180, or a sequence having one or more amino acid substitutions, deletions or additions relative to any of them;
(ii) VH CDR2, consisting of the following sequence: SEQ ID NO: 34, 178 or 181, or a sequence having one or more amino acid substitutions, deletions or additions relative to any of them; and
(iii) VH CDR3, consisting of the following sequence: SEQ ID NO: 35, 179 or 182, or a sequence having one or more amino acid substitutions, deletions or additions relative to any of them; and/or

(b) a light chain variable region comprising the following three complementary determining regions:

(iv) VL CDR1, consisting of the following sequence: SEQ ID NO: 38, 183 or 186, or a sequence having one or more amino acid substitutions, deletions or additions relative to any of them;
(v) VL CDR2, consisting of the following sequence: SEQ ID NO: 39, 184 or 187, or a sequence having one or more amino acid substitutions, deletions or additions relative to any of them; and
(vi) VL CDR3, consisting of the following sequence: SEQ ID NO: 40, 185 or 188, or a sequence having one or more amino acid substitutions, deletions or additions relative to any of them; preferably, the substitution in any one of (i) to (vi) is a conservative substitution;
preferably, VH in the antibody or the antigen binding fragment thereof comprises: VH CDR1 as shown in SEQ ID NO: 33, VH CDR2 as shown in SEQ ID NO: 34, and VH CDR3 as shown in SEQ ID NO: 35; and VL in the antibody or the antigen binding fragment thereof comprises: VL CDR1 as shown in SEQ ID NO: 38, VL CDR2 as shown in SEQ ID NO: 39, and VL CDR3 as shown in SEQ ID NO: 40; or
VH in the antibody or the antigen binding fragment thereof comprises: VH CDR1 as shown in SEQ ID NO: 177, VH CDR2 as shown in SEQ ID NO: 178, and VH CDR3 as shown in SEQ ID NO: 179; and VL in the antibody or the antigen binding fragment thereof comprises: VL CDR1 as shown in SEQ ID NO: 183, VL CDR2 as shown in SEQ ID NO: 184, and VL CDR3 as shown in SEQ ID NO: 185; or
VH in the antibody or the antigen binding fragment thereof comprises: VH CDR1 as shown in SEQ ID NO: 180, VH CDR2 as shown in SEQ ID NO: 181, and VH CDR3 as shown in SEQ ID NO: 182; and VL in the antibody or the antigen binding fragment thereof comprises: VL CDR1 as shown in SEQ ID NO: 186, VL CDR2 as shown in SEQ ID NO: 187, and VL CDR3 as shown in SEQ ID NO: 188.

5. An antibody capable of specifically binding to CD276 protein or an antigen binding fragment thereof, the antibody or the antigen binding fragment thereof comprising:

(a) a heavy chain variable region comprising the following three complementary determining regions:

(i) VH CDR1, consisting of the following sequence: SEQ ID NO: 43, 189 or 192, or a sequence having one or more amino acid substitutions, deletions or additions relative to any of them;
(ii) VH CDR2, consisting of the following sequence: SEQ ID NO: 44, 190 or 193, or a sequence having one or more amino acid substitutions, deletions or additions relative to any of them; and
(iii) VH CDR3, consisting of the following sequence: SEQ ID NO: 45, 191 or 194, or a sequence having one or more amino acid substitutions, deletions or additions relative to any of them; and/or

(b) a light chain variable region comprising the following three complementary determining regions:

(iv) VL CDR1, consisting of the following sequence: SEQ ID NO: 48, 195 or 198, or a sequence having one or more amino acid substitutions, deletions or additions relative to any of them;
(v) VL CDR2, consisting of the following sequence: SEQ ID NO: 49, 196 or 199, or a sequence having one or more amino acid substitutions, deletions or additions relative to any of them; and
(vi) VL CDR3, consisting of the following sequence: SEQ ID NO: 50, 197 or 200, or a sequence having one or more amino acid substitutions, deletions or additions relative to any of them; preferably, the substitution in any one of (i) to (vi) is a conservative substitution;

preferably, VH in the antibody or the antigen binding fragment thereof comprises: VH CDR1 as shown in SEQ ID NO: 43, VH CDR2 as shown in SEQ ID NO: 44, and VH CDR3 as shown in SEQ ID NO: 45; and VL in the antibody or the antigen binding fragment thereof comprises: VL CDR1 as shown in SEQ ID NO: 48, VL CDR2 as shown in SEQ ID NO: 49, and VL CDR3 as shown in SEQ ID NO: 50; or

VH in the antibody or the antigen binding fragment thereof comprises: VH CDR1 as shown in SEQ ID NO: 189, VH CDR2 as shown in SEQ ID NO: 190, and VH CDR3 as shown in SEQ ID NO: 191; and VL in the antibody or the antigen binding fragment thereof comprises: VL CDR1 as shown in SEQ ID NO: 195, VL CDR2 as shown in SEQ ID NO: 196, and VL CDR3 as shown in SEQ ID NO: 197; or

VH in the antibody or the antigen binding fragment thereof comprises: VH CDR1 as shown in SEQ ID NO: 192, VH CDR2 as shown in SEQ ID NO: 193, and VH CDR3 as shown in SEQ ID NO: 194; and VL in the antibody or the antigen binding fragment thereof comprises: VL CDR1 as shown in SEQ ID NO: 198, VL CDR2 as shown in SEQ ID NO: 199, and VL CDR3 as shown in SEQ ID NO: 200.

6.  An antibody capable of specifically binding to CD276 protein or an antigen binding fragment thereof, the antibody or the antigen binding fragment thereof comprising:

    (a) a heavy chain variable region comprising the following three complementary determining regions:

    (i) VH CDR1, consisting of the following sequence: SEQ ID NO: 53, 201 or 204, or a sequence having one or more amino acid substitutions, deletions or additions relative to any of them;
    (ii) VH CDR2, consisting of the following sequence: SEQ ID NO: 54, 202 or 205, or a sequence having one or more amino acid substitutions, deletions or additions relative to any of them; and
    (iii) VH CDR3, consisting of the following sequence: SEQ ID NO: 55, 203 or 206, or a sequence having one or more amino acid substitutions, deletions or additions relative to any of them; and/or

    (b) a light chain variable region comprising the following three complementary determining regions:

    (iv) VL CDR1, consisting of the following sequence: SEQ ID NO: 58, 207 or 210, or a sequence having one or more amino acid substitutions, deletions or additions relative to any of them;
    (v) VL CDR2, consisting of the following sequence: SEQ ID NO: 59, 208 or 211, or a sequence having one or more amino acid substitutions, deletions or additions relative to any of them; and
    (vi) VL CDR3, consisting of the following sequence: SEQ ID NO: 60, 209 or 212, or a sequence having one or more amino acid substitutions, deletions or additions relative to any of them; preferably, the substitution in any one of (i) to (vi) is a conservative substitution;

    preferably, VH in the antibody or the antigen binding fragment thereof comprises: VH CDR1 as shown in SEQ ID NO: 53, VH CDR2 as shown in SEQ ID NO: 54, and VH CDR3 as shown in SEQ ID NO: 55; and VL in the antibody or the antigen binding fragment thereof comprises: VL CDR1 as shown in SEQ ID NO: 58, VL CDR2 as shown in SEQ ID NO: 59, and VL CDR3 as shown in SEQ ID NO: 60; or

    VH in the antibody or the antigen binding fragment thereof comprises: VH CDR1 as shown in SEQ ID NO: 201, VH CDR2 as shown in SEQ ID NO: 202, and VH CDR3 as shown in SEQ ID NO: 203; and VL in the antibody or the antigen binding fragment thereof comprises: VL CDR1 as shown in SEQ ID NO: 207, VL CDR2 as shown in SEQ ID NO: 208, and VL CDR3 as shown in SEQ ID NO: 209; or

    VH in the antibody or the antigen binding fragment thereof comprises: VH CDR1 as shown in SEQ ID NO: 204, VH CDR2 as shown in SEQ ID NO: 205, and VH CDR3 as shown in SEQ ID NO: 206; and VL in the antibody or the antigen binding fragment thereof comprises: VL CDR1 as shown in SEQ ID NO: 210, VL CDR2 as shown in SEQ ID NO: 211, and VL CDR3 as shown in SEQ ID NO: 212.

7.  An antibody capable of specifically binding to CD276 protein or an antigen binding fragment thereof, the antibody or the antigen binding fragment thereof comprising:

    (a) a heavy chain variable region comprising the following three complementary determining regions:

    (i) VH CDR1, consisting of the following sequence: SEQ ID NO: 63, 213 or 216, or a sequence having one or more amino acid substitutions, deletions or additions relative to any of them;

(ii) VH CDR2, consisting of the following sequence: SEQ ID NO: 64, 214 or 217, or a sequence having one or more amino acid substitutions, deletions or additions relative to any of them; and
(iii) VH CDR3, consisting of the following sequence: SEQ ID NO: 65, 215 or 218, or a sequence having one or more amino acid substitutions, deletions or additions relative to any of them; and/or

(b) a light chain variable region comprising the following three complementary determining regions:

(iv) VL CDR1, consisting of the following sequence: SEQ ID NO: 68, 219 or 222, or a sequence having one or more amino acid substitutions, deletions or additions relative to any of them;
(v) VL CDR2, consisting of the following sequence: SEQ ID NO: 69, 220 or 223, or a sequence having one or more amino acid substitutions, deletions or additions relative to any of them; and
(vi) VL CDR3, consisting of the following sequence: SEQ ID NO: 70, 221 or 224, or a sequence having one or more amino acid substitutions, deletions or additions relative to any of them; preferably, the substitution in any one of (i) to (vi) is a conservative substitution;
preferably, VH in the antibody or the antigen binding fragment thereof comprises: VH CDR1 as shown in SEQ ID NO: 63, VH CDR2 as shown in SEQ ID NO: 64, and VH CDR3 as shown in SEQ ID NO: 65; and VL in the antibody or the antigen binding fragment thereof comprises: VL CDR1 as shown in SEQ ID NO: 68, VL CDR2 as shown in SEQ ID NO: 69, and VL CDR3 as shown in SEQ ID NO: 70; or
VH in the antibody or the antigen binding fragment thereof comprises: VH CDR1 as shown in SEQ ID NO: 213, VH CDR2 as shown in SEQ ID NO: 214, and VH CDR3 as shown in SEQ ID NO: 215; and VL in the antibody or the antigen binding fragment thereof comprises: VL CDR1 as shown in SEQ ID NO: 219, VL CDR2 as shown in SEQ ID NO: 220, and VL CDR3 as shown in SEQ ID NO: 221; or
VH in the antibody or the antigen binding fragment thereof comprises: VH CDR1 as shown in SEQ ID NO: 216, VH CDR2 as shown in SEQ ID NO: 217, and VH CDR3 as shown in SEQ ID NO: 218; and VL in the antibody or the antigen binding fragment thereof comprises: VL CDR1 as shown in SEQ ID NO: 222, VL CDR2 as shown in SEQ ID NO: 223, and VL CDR3 as shown in SEQ ID NO: 224.

8. An antibody capable of specifically binding to CD276 protein or an antigen binding fragment thereof, the antibody or the antigen binding fragment thereof comprising:

(a) a heavy chain variable region comprising the following three complementary determining regions:

(i) VH CDR1, consisting of the following sequence: SEQ ID NO: 73, 225 or 228, or a sequence having one or more amino acid substitutions, deletions or additions relative to any of them;
(ii) VH CDR2, consisting of the following sequence: SEQ ID NO: 74, 226 or 229, or a sequence having one or more amino acid substitutions, deletions or additions relative to any of them; and
(iii) VH CDR3, consisting of the following sequence: SEQ ID NO: 75, 227 or 230, or a sequence having one or more amino acid substitutions, deletions or additions relative to any of them; and/or

(b) a light chain variable region comprising the following three complementary determining regions:

(iv) VL CDR1, consisting of the following sequence: SEQ ID NO: 78, 231 or 234, or a sequence having one or more amino acid substitutions, deletions or additions relative to any of them;
(v) VL CDR2, consisting of the following sequence: SEQ ID NO: 79, 232 or 235, or a sequence having one or more amino acid substitutions, deletions or additions relative to any of them; and
(vi) VL CDR3, consisting of the following sequence: SEQ ID NO: 80, 233 or 236, or a sequence having one or more amino acid substitutions, deletions or additions relative to any of them; preferably, the substitution in any one of (i) to (vi) is a conservative substitution;
preferably, VH in the antibody or the antigen binding fragment thereof comprises: VH CDR1 as shown in SEQ ID NO: 73, VH CDR2 as shown in SEQ ID NO: 74, and VH CDR3 as shown in SEQ ID NO: 75; and VL in the antibody or the antigen binding fragment thereof comprises: VL CDR1 as shown in SEQ ID NO: 78, VL CDR2 as shown in SEQ ID NO: 79, and VL CDR3 as shown in SEQ ID NO: 80; or
VH in the antibody or the antigen binding fragment thereof comprises: VH CDR1 as shown in SEQ ID NO: 225, VH CDR2 as shown in SEQ ID NO: 226, and VH CDR3 as shown in SEQ ID NO: 227; and VL in the antibody or the antigen binding fragment thereof comprises: VL CDR1 as shown in SEQ ID NO: 231, VL CDR2 as shown in SEQ ID NO: 232, and VL CDR3 as shown in SEQ ID NO: 233; or
VH in the antibody or the antigen binding fragment thereof comprises: VH CDR1 as shown in SEQ ID NO: 228, VH CDR2 as shown in SEQ ID NO: 229, and VH CDR3 as shown in SEQ ID NO: 230; and VL in the antibody or the antigen binding fragment thereof comprises: VL CDR1 as shown in SEQ ID NO: 234, VL CDR2 as shown in SEQ ID NO: 235, and VL CDR3 as shown in SEQ ID NO: 236.

9. An antibody capable of specifically binding to CD276 protein or an antigen binding fragment thereof, the antibody or the antigen binding fragment thereof comprising:

(a) a heavy chain variable region comprising the following three complementary determining regions:

(i) VH CDR1, consisting of the following sequence: SEQ ID NO: 83, 237 or 240, or a sequence having one or more amino acid substitutions, deletions or additions relative to any of them;
(ii) VH CDR2, consisting of the following sequence: SEQ ID NO: 84, 238 or 241, or a sequence having one or more amino acid substitutions, deletions or additions relative to any of them; and
(iii) VH CDR3, consisting of the following sequence: SEQ ID NO: 85, 239 or 242, or a sequence having one or more amino acid substitutions, deletions or additions relative to any of them; and/or

(b) a light chain variable region comprising the following three complementary determining regions:

(iv) VL CDR1, consisting of the following sequence: SEQ ID NO: 88, 243 or 246, or a sequence having one or more amino acid substitutions, deletions or additions relative to any of them;
(v) VL CDR2, consisting of the following sequence: SEQ ID NO: 89, 244 or 247, or a sequence having one or more amino acid substitutions, deletions or additions relative to any of them; and
(vi) VL CDR3, consisting of the following sequence: SEQ ID NO: 90, 245 or 248, or a sequence having one or more amino acid substitutions, deletions or additions relative to any of them; preferably, the substitution in any one of (i) to (vi) is a conservative substitution;
preferably, VH in the antibody or the antigen binding fragment thereof comprises: VH CDR1 as shown in SEQ ID NO: 83, VH CDR2 as shown in SEQ ID NO: 84, and VH CDR3 as shown in SEQ ID NO: 85; and VL in the antibody or the antigen binding fragment thereof comprises: VL CDR1 as shown in SEQ ID NO: 88, VL CDR2 as shown in SEQ ID NO: 89, and VL CDR3 as shown in SEQ ID NO: 90; or
VH in the antibody or the antigen binding fragment thereof comprises: VH CDR1 as shown in SEQ ID NO: 237, VH CDR2 as shown in SEQ ID NO: 238, and VH CDR3 as shown in SEQ ID NO: 239; and VL in the antibody or the antigen binding fragment thereof comprises: VL CDR1 as shown in SEQ ID NO: 243, VL CDR2 as shown in SEQ ID NO: 244, and VL CDR3 as shown in SEQ ID NO: 245; or
VH in the antibody or the antigen binding fragment thereof comprises: VH CDR1 as shown in SEQ ID NO: 240, VH CDR2 as shown in SEQ ID NO: 241, and VH CDR3 as shown in SEQ ID NO: 242; and VL in the antibody or the antigen binding fragment thereof comprises: VL CDR1 as shown in SEQ ID NO: 246, VL CDR2 as shown in SEQ ID NO: 247, and VL CDR3 as shown in SEQ ID NO: 248.

10. An antibody capable of specifically binding to CD276 protein or an antigen binding fragment thereof, the antibody or the antigen binding fragment thereof comprising:

(a) a heavy chain variable region comprising the following three complementary determining regions:

(i) VH CDR1, consisting of the following sequence: SEQ ID NO: 93, 249 or 252, or a sequence having one or more amino acid substitutions, deletions or additions relative to any of them;
(ii) VH CDR2, consisting of the following sequence: SEQ ID NO: 94, 250 or 253, or a sequence having one or more amino acid substitutions, deletions or additions relative to any of them; and
(iii) VH CDR3, consisting of the following sequence: SEQ ID NO: 95, 251 or 254, or a sequence having one or more amino acid substitutions, deletions or additions relative to any of them; and/or

(b) a light chain variable region comprising the following three complementary determining regions:

(iv) VL CDR1, consisting of the following sequence: SEQ ID NO: 98, 255 or 258, or a sequence having one or more amino acid substitutions, deletions or additions relative to any of them;
(v) VL CDR2, consisting of the following sequence: SEQ ID NO: 99, 256 or 259, or a sequence having one or more amino acid substitutions, deletions or additions relative to any of them; and
(vi) VL CDR3, consisting of the following sequence: SEQ ID NO: 100, 257 or 260, or a sequence having one or more amino acid substitutions, deletions or additions relative to any of them; preferably, the substitution in any one of (i) to (vi) is a conservative substitution;

preferably, VH in the antibody or the antigen binding fragment thereof comprises: VH CDR1 as shown in SEQ ID NO: 93, VH CDR2 as shown in SEQ ID NO: 94, and VH CDR3 as shown in SEQ ID NO: 95; and VL in the antibody or the antigen binding fragment thereof comprises: VL CDR1 as shown in SEQ ID NO: 98, VL CDR2 as shown in SEQ ID NO: 99, and VL CDR3 as shown in SEQ ID NO: 100; or

VH in the antibody or the antigen binding fragment thereof comprises: VH CDR1 as shown in SEQ ID NO: 249, VH CDR2 as shown in SEQ ID NO: 250, and VH CDR3 as shown in SEQ ID NO: 251; and VL in the antibody or the antigen binding fragment thereof comprises: VL CDR1 as shown in SEQ ID NO: 255, VL CDR2 as shown in SEQ ID NO: 256, and VL CDR3 as shown in SEQ ID NO: 257; or

VH in the antibody or the antigen binding fragment thereof comprises: VH CDR1 as shown in SEQ ID NO: 252, VH CDR2 as shown in SEQ ID NO: 253, and VH CDR3 as shown in SEQ ID NO: 254; and VL in the antibody or the antigen binding fragment thereof comprises: VL CDR1 as shown in SEQ ID NO: 258, VL CDR2 as shown in SEQ ID NO: 259, and VL CDR3 as shown in SEQ ID NO: 260.

11. An antibody capable of specifically binding to CD276 protein or an antigen binding fragment thereof, the antibody or the antigen binding fragment thereof comprising:

(a) a heavy chain variable region comprising the following three complementary determining regions:

(i) VH CDR1, consisting of the following sequence: SEQ ID NO: 103, 261 or 264, or a sequence having one or more amino acid substitutions, deletions or additions relative to any of them;
(ii) VH CDR2, consisting of the following sequence: SEQ ID NO: 104, 262 or 265, or a sequence having one or more amino acid substitutions, deletions or additions relative to any of them; and
(iii) VH CDR3, consisting of the following sequence: SEQ ID NO: 105, 263 or 266, or a sequence having one or more amino acid substitutions, deletions or additions relative to any of them; and/or

(b) a light chain variable region comprising the following three complementary determining regions:

(iv) VL CDR1, consisting of the following sequence: SEQ ID NO: 108, 267 or 270, or a sequence having one or more amino acid substitutions, deletions or additions relative to any of them;
(v) VL CDR2, consisting of the following sequence: SEQ ID NO: 109, 268 or 271, or a sequence having one or more amino acid substitutions, deletions or additions relative to any of them; and
(vi) VL CDR3, consisting of the following sequence: SEQ ID NO: 110, 269 or 272, or a sequence having one or more amino acid substitutions, deletions or additions relative to any of them; preferably, the substitution in any one of (i) to (vi) is a conservative substitution;
preferably, VH in the antibody or the antigen binding fragment thereof comprises: VH CDR1 as shown in SEQ ID NO: 103, VH CDR2 as shown in SEQ ID NO: 104, and VH CDR3 as shown in SEQ ID NO: 105; and VL in the antibody or the antigen binding fragment thereof comprises: VL CDR1 as shown in SEQ ID NO: 108, VL CDR2 as shown in SEQ ID NO: 109, and VL CDR3 as shown in SEQ ID NO: 110; or
VH in the antibody or the antigen binding fragment thereof comprises: VH CDR1 as shown in SEQ ID NO: 261, VH CDR2 as shown in SEQ ID NO: 262, and VH CDR3 as shown in SEQ ID NO: 263; and VL in the antibody or the antigen binding fragment thereof comprises: VL CDR1 as shown in SEQ ID NO: 267, VL CDR2 as shown in SEQ ID NO: 268, and VL CDR3 as shown in SEQ ID NO: 269; or
VH in the antibody or the antigen binding fragment thereof comprises: VH CDR1 as shown in SEQ ID NO: 264, VH CDR2 as shown in SEQ ID NO: 265, and VH CDR3 as shown in SEQ ID NO: 266; and VL in the antibody or the antigen binding fragment thereof comprises: VL CDR1 as shown in SEQ ID NO: 270, VL CDR2 as shown in SEQ ID NO: 271, and VL CDR3 as shown in SEQ ID NO: 272.

12. An antibody capable of specifically binding to CD276 protein or an antigen binding fragment thereof, the antibody or the antigen binding fragment thereof comprising:

(a) a heavy chain variable region comprising the following three complementary determining regions:

(i) VH CDR1, consisting of the following sequence: SEQ ID NO: 113, 273 or 276, or a sequence having one or more amino acid substitutions, deletions or additions relative to any of them;
(ii) VH CDR2, consisting of the following sequence: SEQ ID NO: 114, 274 or 277, or a sequence having one or more amino acid substitutions, deletions or additions relative to any of them; and
(iii) VH CDR3, consisting of the following sequence: SEQ ID NO: 115, 275 or 278, or a sequence having one or more amino acid substitutions, deletions or additions relative to any of them; and/or

(b) a light chain variable region comprising the following three complementary determining regions:

(iv) VL CDR1, consisting of the following sequence: SEQ ID NO: 118, 279 or 282, or a sequence having one or more amino acid substitutions, deletions or additions relative to any of them;
(v) VL CDR2, consisting of the following sequence: SEQ ID NO: 119, 280 or 283, or a sequence having one or more amino acid substitutions, deletions or additions relative to any of them; and
(vi) VL CDR3, consisting of the following sequence: SEQ ID NO: 120, 281 or 284, or a sequence having one or more amino acid substitutions, deletions or additions relative to any of them; preferably, the substitution in any one of (i) to (vi) is a conservative substitution;
preferably, VH in the antibody or the antigen binding fragment thereof comprises: VH CDR1 as shown in SEQ ID NO: 113, VH CDR2 as shown in SEQ ID NO: 114, and VH CDR3 as shown in SEQ ID NO: 115; and VL in the antibody or the antigen binding fragment thereof comprises: VL CDR1 as shown in SEQ ID NO: 118, VL CDR2 as shown in SEQ ID NO: 119, and VL CDR3 as shown in SEQ ID NO: 120; or
VH in the antibody or the antigen binding fragment thereof comprises: VH CDR1 as shown in SEQ ID NO: 273, VH CDR2 as shown in SEQ ID NO: 274, and VH CDR3 as shown in SEQ ID NO: 275; and VL in the antibody or the antigen binding fragment thereof comprises: VL CDR1 as shown in SEQ ID NO: 279, VL CDR2 as shown in SEQ ID NO: 280, and VL CDR3 as shown in SEQ ID NO: 281; or
VH in the antibody or the antigen binding fragment thereof comprises: VH CDR1 as shown in SEQ ID NO: 276, VH CDR2 as shown in SEQ ID NO: 277, and VH CDR3 as shown in SEQ ID NO: 278; and VL in the antibody or the antigen binding fragment thereof comprises: VL CDR1 as shown in SEQ ID NO: 282, VL CDR2 as shown in SEQ ID NO: 283, and VL CDR3 as shown in SEQ ID NO: 284.

13. An antibody capable of specifically binding to CD276 protein or an antigen binding fragment thereof, the antibody or the antigen binding fragment thereof comprising:

(a) a heavy chain variable region comprising the following three complementary determining regions:

(i) VH CDR1, consisting of the following sequence: SEQ ID NO: 123, 285 or 288, or a sequence having one or more amino acid substitutions, deletions or additions relative to any of them;
(ii) VH CDR2, consisting of the following sequence: SEQ ID NO: 124, 286 or 289, or a sequence having one or more amino acid substitutions, deletions or additions relative to any of them; and
(iii) VH CDR3, consisting of the following sequence: SEQ ID NO: 125, 287 or 290, or a sequence having one or more amino acid substitutions, deletions or additions relative to any of them; and/or

(b) a light chain variable region comprising the following three complementary determining regions:

(iv) VL CDR1, consisting of the following sequence: SEQ ID NO: 128, 291 or 294, or a sequence having one or more amino acid substitutions, deletions or additions relative to any of them;
(v) VL CDR2, consisting of the following sequence: SEQ ID NO: 129, 292 or 295, or a sequence having one or more amino acid substitutions, deletions or additions relative to any of them; and
(vi) VL CDR3, consisting of the following sequence: SEQ ID NO: 130, 293 or 296, or a sequence having one or more amino acid substitutions, deletions or additions relative to any of them; preferably, the substitution in any one of (i) to (vi) is a conservative substitution;
preferably, VH in the antibody or the antigen binding fragment thereof comprises: VH CDR1 as shown in SEQ ID NO: 123, VH CDR2 as shown in SEQ ID NO: 124, and VH CDR3 as shown in SEQ ID NO: 125; and VL in the antibody or the antigen binding fragment thereof comprises: VL CDR1 as shown in SEQ ID NO: 128, VL CDR2 as shown in SEQ ID NO: 129, and VL CDR3 as shown in SEQ ID NO: 130; or
VH in the antibody or the antigen binding fragment thereof comprises: VH CDR1 as shown in SEQ ID NO: 285, VH CDR2 as shown in SEQ ID NO: 286, and VH CDR3 as shown in SEQ ID NO: 287; and VL in the antibody or the antigen binding fragment thereof comprises: VL CDR1 as shown in SEQ ID NO: 291, VL CDR2 as shown in SEQ ID NO: 292, and VL CDR3 as shown in SEQ ID NO: 293; or
VH in the antibody or the antigen binding fragment thereof comprises: VH CDR1 as shown in SEQ ID NO: 288, VH CDR2 as shown in SEQ ID NO: 289, and VH CDR3 as shown in SEQ ID NO: 290; and VL in the antibody or the antigen binding fragment thereof comprises: VL CDR1 as shown in SEQ ID NO: 294, VL CDR2 as shown in SEQ ID NO: 295, and VL CDR3 as shown in SEQ ID NO: 296.

14. An antibody capable of specifically binding to CD276 protein or an antigen binding fragment thereof, the antibody or the antigen binding fragment thereof comprising:

(a) a heavy chain variable region comprising the following three complementary determining regions:

(i) VH CDR1, consisting of the following sequence: SEQ ID NO: 133, 297 or 300, or a sequence having one or more amino acid substitutions, deletions or additions relative to any of them;
(ii) VH CDR2, consisting of the following sequence: SEQ ID NO: 134, 298 or 301, or a sequence having one or more amino acid substitutions, deletions or additions relative to any of them; and
(iii) VH CDR3, consisting of the following sequence: SEQ ID NO: 135, 299 or 302, or a sequence having one or more amino acid substitutions, deletions or additions relative to any of them; and/or

(b) a light chain variable region comprising the following three complementary determining regions:

(iv) VL CDR1, consisting of the following sequence: SEQ ID NO: 138, 303 or 306, or a sequence having one or more amino acid substitutions, deletions or additions relative to any of them;
(v) VL CDR2, consisting of the following sequence: SEQ ID NO: 139, 304 or 307, or a sequence having one or more amino acid substitutions, deletions or additions relative to any of them; and
(vi) VL CDR3, consisting of the following sequence: SEQ ID NO: 140, 305 or 308, or a sequence having one or more amino acid substitutions, deletions or additions relative to any of them; preferably, the substitution in any one of (i) to (vi) is a conservative substitution;
preferably, VH in the antibody or the antigen binding fragment thereof comprises: VH CDR1 as shown in SEQ ID NO: 133, VH CDR2 as shown in SEQ ID NO: 134, and VH CDR3 as shown in SEQ ID NO: 135; and VL in the antibody or the antigen binding fragment thereof comprises: VL CDR1 as shown in SEQ ID NO: 138, VL CDR2 as shown in SEQ ID NO: 139, and VL CDR3 as shown in SEQ ID NO: 140; or
VH in the antibody or the antigen binding fragment thereof comprises: VH CDR1 as shown in SEQ ID NO: 297, VH CDR2 as shown in SEQ ID NO: 298, and VH CDR3 as shown in SEQ ID NO: 299; and VL in the antibody or the antigen binding fragment thereof comprises: VL CDR1 as shown in SEQ ID NO: 303, VL CDR2 as shown in SEQ ID NO: 304, and VL CDR3 as shown in SEQ ID NO: 305; or
VH in the antibody or the antigen binding fragment thereof comprises: VH CDR1 as shown in SEQ ID NO: 300, VH CDR2 as shown in SEQ ID NO: 301, and VH CDR3 as shown in SEQ ID NO: 302; and VL in the antibody or the antigen binding fragment thereof comprises: VL CDR1 as shown in SEQ ID NO: 306, VL CDR2 as shown in SEQ ID NO: 307, and VL CDR3 as shown in SEQ ID NO: 308.

15. An antibody capable of specifically binding to CD276 protein or an antigen binding fragment thereof, the antibody or the antigen binding fragment thereof comprising a heavy chain variable region and a light chain variable region, wherein the heavy chain variable region comprising three CDRs comprised in the heavy chain variable region as shown in any one of SEQ ID NO: 2, 12, 22, 32, 42, 52, 62, 72, 82, 92, 102, 112, 122 or 132; and the light chain variable region comprises three CDRs comprised in the light chain variable region as shown in any one of SEQ ID NO: 7, 17, 27, 37, 47, 57, 67, 77, 87, 97, 107, 117, 127 or 137;
preferably, the three CDRs comprised in the heavy chain variable region, and/or the three CDRs comprised in the light chain variable region, are defined by the Kabat, Chothia or IMGT numbering system.

16. The antibody or the antigen binding fragment thereof according to claim 1, wherein the antibody or the antigen binding fragment thereof comprises:

(a) a heavy chain variable region comprising an amino acid sequence selected from the group consisting of:

(i) a sequence as shown in SEQ ID NO: 2;
(ii) a sequence having one or more amino acid substitutions, deletions or additions relative to the sequence as shown in SEQ ID NO: 2; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 2;
and/or,

(b) a light chain variable region comprising an amino acid sequence selected from the group consisting of:

(iv) a sequence as shown in SEQ ID NO: 7;
(v) a sequence having one or more amino acid substitutions, deletions or additions relative to the sequence as shown in SEQ ID NO: 7; or

(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 7;

preferably, the substitution in (ii) or (v) is a conservative substitution;

preferably, the antibody or the antigen binding fragment thereof comprises: VH having a sequence as shown in SEQ ID NO: 2 and VL having a sequence as shown in SEQ ID NO: 7.

17. The antibody or the antigen binding fragment thereof according to claim 2, wherein the antibody or the antigen binding fragment thereof comprises:

(a) a heavy chain variable region comprising an amino acid sequence selected from the group consisting of:

(i) a sequence as shown in SEQ ID NO: 12;
(ii) a sequence having one or more amino acid substitutions, deletions or additions relative to the sequence as shown in SEQ ID NO: 12; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 12;
and/or,

(b) a light chain variable region comprising an amino acid sequence selected from the group consisting of:

(iv) a sequence as shown in SEQ ID NO: 17;
(v) a sequence having one or more amino acid substitutions, deletions or additions relative to the sequence as shown in SEQ ID NO: 17; or
(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 17;
preferably, the substitution in (ii) or (v) is a conservative substitution;
preferably, the antibody or the antigen binding fragment thereof comprises: VH having a sequence as shown in SEQ ID NO: 12 and VL having a sequence as shown in SEQ ID NO: 17.

18. The antibody or the antigen binding fragment thereof according to claim 3, wherein the antibody or the antigen binding fragment thereof comprises:

(a) a heavy chain variable region comprising an amino acid sequence selected from the group consisting of:

(i) a sequence as shown in SEQ ID NO: 22;
(ii) a sequence having one or more amino acid substitutions, deletions or additions relative to the sequence as shown in SEQ ID NO: 22; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 22;
and/or,

(b) a light chain variable region comprising an amino acid sequence selected from the group consisting of:

(iv) a sequence as shown in SEQ ID NO: 27;
(v) a sequence having one or more amino acid substitutions, deletions or additions relative to the sequence as shown in SEQ ID NO: 27; or
(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 27;
preferably, the substitution in (ii) or (v) is a conservative substitution;
preferably, the antibody or the antigen binding fragment thereof comprises: VH having a sequence as shown in SEQ ID NO: 22 and VL having a sequence as shown in SEQ ID NO: 27.

19. The antibody or the antigen binding fragment thereof according to claim 4, wherein the antibody or the antigen binding fragment thereof comprises:

(a) a heavy chain variable region comprising an amino acid sequence selected from the group consisting of:

(i) a sequence as shown in SEQ ID NO: 32;
(ii) a sequence having one or more amino acid substitutions, deletions or additions relative to the sequence as shown in SEQ ID NO: 32; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 32;
and/or,

(b) a light chain variable region comprising an amino acid sequence selected from the group consisting of:

(iv) a sequence as shown in SEQ ID NO: 37;
(v) a sequence having one or more amino acid substitutions, deletions or additions relative to the sequence as shown in SEQ ID NO: 37; or
(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 37;
preferably, the substitution in (ii) or (v) is a conservative substitution;
preferably, the antibody or the antigen binding fragment thereof comprises: VH having a sequence as shown in SEQ ID NO: 32 and VL having a sequence as shown in SEQ ID NO: 37.

20. The antibody or the antigen binding fragment thereof according to claim 5, wherein the antibody or the antigen binding fragment thereof comprises:

(a) a heavy chain variable region comprising an amino acid sequence selected from the group consisting of:

(i) a sequence as shown in SEQ ID NO: 42;
(ii) a sequence having one or more amino acid substitutions, deletions or additions relative to the sequence as shown in SEQ ID NO: 42; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 42;
and/or,

(b) a light chain variable region comprising an amino acid sequence selected from the group consisting of:

(iv) a sequence as shown in SEQ ID NO: 47;
(v) a sequence having one or more amino acid substitutions, deletions or additions relative to the sequence as shown in SEQ ID NO: 47; or
(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 47;
preferably, the substitution in (ii) or (v) is a conservative substitution;
preferably, the antibody or the antigen binding fragment thereof comprises: VH having a sequence as shown in SEQ ID NO: 42 and VL having a sequence as shown in SEQ ID NO: 47.

21. The antibody or the antigen binding fragment thereof according to claim 6, wherein the antibody or the antigen binding fragment thereof comprises:

(a) a heavy chain variable region comprising an amino acid sequence selected from the group consisting of:

(i) a sequence as shown in SEQ ID NO: 52;
(ii) a sequence having one or more amino acid substitutions, deletions or additions relative to the sequence as shown in SEQ ID NO: 52; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 52; and/or,

(b) a light chain variable region comprising an amino acid sequence selected from the group consisting of:

(iv) a sequence as shown in SEQ ID NO: 57;
(v) a sequence having one or more amino acid substitutions, deletions or additions relative to the sequence as shown in SEQ ID NO: 57; or
(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 57;
preferably, the substitution in (ii) or (v) is a conservative substitution;
preferably, the antibody or the antigen binding fragment thereof comprises: VH having a sequence as shown in SEQ ID NO: 52 and VL having a sequence as shown in SEQ ID NO: 57.

22. The antibody or the antigen binding fragment thereof according to claim 7, wherein the antibody or the antigen binding fragment thereof comprises:

(a) a heavy chain variable region comprising an amino acid sequence selected from the group consisting of:

(i) a sequence as shown in SEQ ID NO: 62;
(ii) a sequence having one or more amino acid substitutions, deletions or additions relative to the sequence as shown in SEQ ID NO: 62; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 62; and/or,

(b) a light chain variable region comprising an amino acid sequence selected from the group consisting of:

(iv) a sequence as shown in SEQ ID NO: 67;
(v) a sequence having one or more amino acid substitutions, deletions or additions relative to the sequence as shown in SEQ ID NO: 67; or
(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 67;
preferably, the substitution in (ii) or (v) is a conservative substitution;
preferably, the antibody or the antigen binding fragment thereof comprises: VH having a sequence as shown in SEQ ID NO: 62 and VL having a sequence as shown in SEQ ID NO: 67.

23. The antibody or the antigen binding fragment thereof according to claim 8, wherein the antibody or the antigen binding fragment thereof comprises:

(a) a heavy chain variable region comprising an amino acid sequence selected from the group consisting of:

(i) a sequence as shown in SEQ ID NO: 72;
(ii) a sequence having one or more amino acid substitutions, deletions or additions relative to the sequence as shown in SEQ ID NO: 72; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 72; and/or,

(b) a light chain variable region comprising an amino acid sequence selected from the group consisting of:

(iv) a sequence as shown in SEQ ID NO: 77;
(v) a sequence having one or more amino acid substitutions, deletions or additions relative to the sequence as shown in SEQ ID NO: 77; or

(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 77;

preferably, the substitution in (ii) or (v) is a conservative substitution;

preferably, the antibody or the antigen binding fragment thereof comprises: VH having a sequence as shown in SEQ ID NO: 72 and VL having a sequence as shown in SEQ ID NO: 77.

24. The antibody or the antigen binding fragment thereof according to claim 9, wherein the antibody or the antigen binding fragment thereof comprises:

(a) a heavy chain variable region comprising an amino acid sequence selected from the group consisting of:

(i) a sequence as shown in SEQ ID NO: 82;

(ii) a sequence having one or more amino acid substitutions, deletions or additions relative to the sequence as shown in SEQ ID NO: 82; or

(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 82;

and/or,

(b) a light chain variable region comprising an amino acid sequence selected from the group consisting of:

(iv) a sequence as shown in SEQ ID NO: 87;

(v) a sequence having one or more amino acid substitutions, deletions or additions relative to the sequence as shown in SEQ ID NO: 87; or

(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 87;

preferably, the substitution in (ii) or (v) is a conservative substitution;

preferably, the antibody or the antigen binding fragment thereof comprises: VH having a sequence as shown in SEQ ID NO: 82 and VL having a sequence as shown in SEQ ID NO: 87.

25. The antibody or the antigen binding fragment thereof according to claim 10, wherein the antibody or the antigen binding fragment thereof comprises:

(a) a heavy chain variable region comprising an amino acid sequence selected from the group consisting of:

(i) a sequence as shown in SEQ ID NO: 92;

(ii) a sequence having one or more amino acid substitutions, deletions or additions relative to the sequence as shown in SEQ ID NO: 92; or

(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 92;

and/or,

(b) a light chain variable region comprising an amino acid sequence selected from the group consisting of:

(iv) a sequence as shown in SEQ ID NO: 97;

(v) a sequence having one or more amino acid substitutions, deletions or additions relative to the sequence as shown in SEQ ID NO: 97; or

(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 97;

preferably, the substitution in (ii) or (v) is a conservative substitution;

preferably, the antibody or the antigen binding fragment thereof comprises: VH having a sequence as shown in SEQ ID NO: 92 and VL having a sequence as shown in SEQ ID NO: 97.

26. The antibody or the antigen binding fragment thereof according to claim 11, wherein the antibody or the antigen binding fragment thereof comprises:

(a) a heavy chain variable region comprising an amino acid sequence selected from the group consisting of:

(i) a sequence as shown in SEQ ID NO: 102;
(ii) a sequence having one or more amino acid substitutions, deletions or additions relative to the sequence as shown in SEQ ID NO: 102; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 102;
and/or,

(b) a light chain variable region comprising an amino acid sequence selected from the group consisting of:

(iv) a sequence as shown in SEQ ID NO: 107;
(v) a sequence having one or more amino acid substitutions, deletions or additions relative to the sequence as shown in SEQ ID NO: 107; or
(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 107;
preferably, the substitution in (ii) or (v) is a conservative substitution;
preferably, the antibody or the antigen binding fragment thereof comprises: VH having a sequence as shown in SEQ ID NO: 102 and VL having a sequence as shown in SEQ ID NO: 107.

27. The antibody or the antigen binding fragment thereof according to claim 12, wherein the antibody or the antigen binding fragment thereof comprises:

(a) a heavy chain variable region comprising an amino acid sequence selected from the group consisting of:

(i) a sequence as shown in SEQ ID NO: 112;
(ii) a sequence having one or more amino acid substitutions, deletions or additions relative to the sequence as shown in SEQ ID NO: 112; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 112;
and/or,

(b) a light chain variable region comprising an amino acid sequence selected from the group consisting of:

(iv) a sequence as shown in SEQ ID NO: 117;
(v) a sequence having one or more amino acid substitutions, deletions or additions relative to the sequence as shown in SEQ ID NO: 117; or
(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 117;
preferably, the substitution in (ii) or (v) is a conservative substitution;
preferably, the antibody or the antigen binding fragment thereof comprises: VH having a sequence as shown in SEQ ID NO: 112 and VL having a sequence as shown in SEQ ID NO: 117.

28. The antibody or the antigen binding fragment thereof according to claim 13, wherein the antibody or the antigen binding fragment thereof comprises:

(a) a heavy chain variable region comprising an amino acid sequence selected from the group consisting of:

(i) a sequence as shown in SEQ ID NO: 122;
(ii) a sequence having one or more amino acid substitutions, deletions or additions relative to the sequence as shown in SEQ ID NO: 122; or

(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 122;
and/or,

(b) a light chain variable region comprising an amino acid sequence selected from the group consisting of:

(iv) a sequence as shown in SEQ ID NO: 127;
(v) a sequence having one or more amino acid substitutions, deletions or additions relative to the sequence as shown in SEQ ID NO: 127; or
(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 127;
preferably, the substitution in (ii) or (v) is a conservative substitution;
preferably, the antibody or the antigen binding fragment thereof comprises: VH having a sequence as shown in SEQ ID NO: 122 and VL having a sequence as shown in SEQ ID NO: 127.

29. The antibody or the antigen binding fragment thereof according to claim 14, wherein the antibody or the antigen binding fragment thereof comprises:

(a) a heavy chain variable region comprising an amino acid sequence selected from the group consisting of:

(i) a sequence as shown in SEQ ID NO: 132;
(ii) a sequence having one or more amino acid substitutions, deletions or additions relative to the sequence as shown in SEQ ID NO: 132; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 132;
and/or,

(b) a light chain variable region comprising an amino acid sequence selected from the group consisting of:

(iv) a sequence as shown in SEQ ID NO: 137;
(v) a sequence having one or more amino acid substitutions, deletions or additions relative to the sequence as shown in SEQ ID NO: 137; or
(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in SEQ ID NO: 137;
preferably, the substitution in (ii) or (v) is a conservative substitution;
preferably, the antibody or the antigen binding fragment thereof comprises: VH having a sequence as shown in SEQ ID NO: 132 and VL having a sequence as shown in SEQ ID NO: 137.

30. The antibody or the antigen binding fragment thereof according to any one of claims 1 to 29, wherein the antibody or the antigen binding fragment thereof further comprises:

(a) a heavy chain constant region of a human immunoglobulin or a variant thereof, the variant having one or more amino acid substitutions, deletions or additions relative to a sequence from which the variant is derived; and
(b) a light chain constant region of a human immunoglobulin or a variant thereof, the variant having conservative substitutions of up to 20 amino acids relative to a sequence from which the variant is derived;

preferably, the heavy chain constant region is an IgG heavy chain constant region, more preferably an IgG1, IgG2, IgG3 or IgG4 heavy chain constant region, further preferably, a human IgG1 or human IgG4 heavy chain constant region;
preferably, the light chain constant region is a $\kappa$ light chain constant region.

31. The antibody or the antigen binding fragment thereof according to any one of claims 1 to 30, wherein the antigen binding fragment is selected from the group consisting of Fab, Fab', (Fab')$_2$, Fv, disulfide-bonded Fv, scFv, a diabody and a single-domain antibody; and/or, the antibody is a mouse-derived antibody, a chimeric antibody, a humanized antibody, a bispecific antibody or a multispecific antibody; and more preferably, the antibody is a fully humanized antibody.

32. A chimeric antigen receptor-T cell, comprising the antibody or antigen binding fragment thereof according to any one of claims 1 to 31;
preferably, the heavy chain variable region and the light chain variable region in the antibody or the antigen binding fragment thereof are combined in series or in parallel.

33. An isolated nucleic acid molecule, which encodes the antibody or the antigen binding fragment thereof according to any one of claims 1 to 31, or a heavy chain variable region and/or a light chain variable region thereof;

preferably, a nucleic acid molecule sequence encoding the heavy chain variable region is as shown in any one of SEQ ID NO: 1, 11, 21, 31, 41, 51, 61, 71, 81, 91, 101, 111, 121 or 131;
preferably, a nucleic acid molecule sequence encoding the light chain variable region is as shown in any one of SEQ ID NO: 6, 16, 26, 36, 46, 56, 66, 76, 86, 96, 106, 116, 126 or 136.

34. A vector, comprising the isolated nucleic acid molecule according to claim 33;

preferably, the vector is a cloning vector or an expression vector;
more preferably, the vector is a virus;
further preferably, a virus skeleton of the virus vector is derived from a modified or engineered vaccinia virus Tian Tan strain, a vaccinia virus New York strain, a vaccinia virus Copenhagen strain, a vaccinia virus canary strain, a vaccinia virus Ankara strain, an adenovirus vector, an adeno-associated virus vector, a herpes simplex virus vector, a varicella-zoster virus vector, a respiratory syncytial virus, a Semliki forest virus, an EB virus, a cytomegalovirus, a human Herpesvirus 6, a smallpox virus, a molluscum contagiosum virus, a sheep orf virus, a reovirus, a rotavirus, an enterovirus, a Seneca virus, a poliovirus, a coxsackievirus, a rhinovirus, a hepatitis A virus, a foot and mouth disease virus, a togavirus, an alphavirus, an Eastern equine encephalitis virus, a Sindbis virus, a rubella virus, a coronavirus, a flavivirus, a hepatitis C virus, a Japanese Encephalitis virus, a St. Louis encephalitis virus, a Murray Valley fever virus, a yellow fever virus, a West Nile virus, a Zika virus, a dengue virus, an Ebola virus, a Marburg virus, an arenavirus, a Lassa fever virus, a lymphocytes Choriomeningitis virus, a Pichinde virus, a Junin virus, Machupo virus, a Hantaan virus, a Rift Valley fever virus, a Paramyxovirus, a human parainfluenza virus, a Mumps virus, a simian virus 5, a measles virus, a vesicular stomatitis virus, a rabies virus, a respiratory syncytial virus, an orthomyxovirus, an influenza A virus, an influenza B virus, an influenza C virus, a hepatitis D virus, a simian immunodeficiency virus, a human immunodeficiency virus 1, a human immunodeficiency virus 2, a Rous sarcoma virus, a human T-cell leukemia virus 1, a simian foamy virus, a hepatitis B virus, hepatitis E virus, a human papillomavirus, or a polyomavirus; more preferably, the virus vector is a recombinant adenovirus vector, a recombinant poxvirus vector or a recombinant lentivirus vector;
still more preferably, the vector is the cloning vector AbVec hIgKappa or the cloning vector AbVec hIgG1.

35. A host cell, comprising the isolated nucleic acid molecule according to claim 33 or the vector according to claim 34;
preferably, the host cell is prokaryotic or eukaryotic; more preferably, the host cell is selected from an *E. coli* cell, a yeast cell, a mammalian cell or other cell suitable for the preparation of an antibody or an antigen binding fragment, or a multispecific antibody; further preferably, the host cell is a mammalian cell; still further preferably, the host cell is a cell derived from human beings, mouse, sheep, horse, dog or cat; and most preferably, the host cell is a 293 cell or a CHO cell.

36. A preparation method for the antibody or the antigen binding fragment thereof according to any one of claims 1 to 31, comprising culturing the host cell according to claim 35 under a condition that allows the expression of the antibody or the antigen binding fragment thereof according to any one of claims 1 to 31; and recycling the antibody or the antigen binding fragment thereof from the cultured host cell culture.

37. A bispecific or multispecific molecule, comprising the antibody or antigen binding fragment thereof according to any one of claims 1 to 31;

preferably, the bispecific or multispecific molecule specifically binds to CD276 protein and additionally specifically binds to one or more other targets;
preferably, the bispecific or multispecific molecule further comprises at least one molecule, preferably a second antibody, with a second binding specificity for a second target;

preferably, the bispecific or multispecific molecule further comprises other antibody that specifically binds to an epitope of CD276 protein or an antigen binding fragment thereof; preferably, the bispecific or multispecific molecule is a bispecific T-cell engager;

preferably, the bispecific T-cell engager comprises an amino acid sequence selected from the group consisting of:

(i) a sequence as shown in any one of SEQ ID NOs: 312, 314, 316 and 318;
(ii) a sequence having one or more amino acid substitutions, deletions or additions relative to the sequence as shown in any one of SEQ ID NOs: 312, 314, 316 and 318; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as shown in any one of SEQ ID NOs: 312, 314, 316 and 318.

38. An immunoconjugate, comprising the antibody or the antigen binding fragment thereof according to any one of claims 1 to 31 and a therapeutic agent conjugated to the antibody or the antigen binding fragment thereof;

preferably, the therapeutic agent is selected from a cytotoxic agent;
preferably, the therapeutic agent is selected from an alkylating agent, a mitotic inhibitor, an anti-tumor antibiotic, an antimetabolite, a topoisomerase inhibitor, a tyrosine kinase inhibitor, a radionuclide agent, and any combination thereof;
preferably, the immunoconjugate is an antibody-drug conjugate.

39. A pharmaceutical composition, comprising the antibody or the antigen binding fragment thereof according to any one of claims 1 to 31, the bispecific or multispecific molecule according to claim 37 or the immunoconjugate according to claim 38, and a pharmaceutically acceptable vector and/or excipient;

preferably, the pharmaceutical composition further comprises an additional pharmaceutically active agent;
preferably, the additional pharmaceutically active agent is a drug with anti-tumor activity; more preferably, the pharmaceutically active agent is an alkylating agent, a mitotic inhibitor, an anti-tumor antibiotic, an antimetabolite, a topoisomerase inhibitor, a tyrosine kinase inhibitor, a radionuclide agent, a radiosensitizing agent, an anti-angiogenic agent, a cytokine, a molecularly targeted drug, an immune checkpoint inhibitor or an oncolytic virus;
preferably, the antibody or the antigen binding fragment thereof, and the bispecific or multispecific molecule or the immunoconjugate, and the additional pharmaceutically active agent are provided as separate components or as components of the same composition.

40. A kit, comprising the antibody or antigen binding fragment thereof according to any one of claims 1 to 31;

preferably, the antibody or antigen binding fragment thereof carries a detectable label, a radionuclide, a fluorescent dye, a luminescent substance or biotin; more preferably, the detectable label is an enzyme, further preferably horseradish peroxidase; and more preferably, the luminescent substance is a chemiluminescent substance;
preferably, the kit further contains a second antibody which specifically recognizes the antibody or the antigen binding fragment thereof according to any one of claims 1 to 31;
preferably, the second antibody further comprises a detectable label, a radionuclide, a fluorescent dye, a luminescent substance or biotin; more preferably, the detectable label is an enzyme, further preferably horseradish peroxidase; and more preferably, the luminescent substance is a chemiluminescent substance.

41. A chimeric antigen receptor, comprising an antigen binding domain of the antibody or antigen binding fragment thereof according to any one of claims 1 to 31;

preferably, the antigen binding domain comprises a heavy chain variable region and a light chain variable region of the antibody or the antigen binding fragment thereof according to any one of claims 1 to 31;
preferably, the antigen binding domain is scFv;
preferably, the chimeric antigen receptor comprises the antigen binding fragment of the antibody according to any one of claims 1 to 31;
preferably, the chimeric antigen receptor is expressed by an immune effector cell;
more preferably, the immune effector cell is a T cell.

42. An isolated nucleic acid molecule, which encodes the chimeric antigen receptor according to claim 41.

43. A vector, comprising the isolated nucleic acid molecule according to claim 42, and preferably, the vector is used for the preparation of a chimeric antigen receptor-T cell.

44. A host cell, comprising the isolated nucleic acid molecule according to claim 42 or the vector according to claim 43;

   preferably, the host cell is an immune effector cell; more particularly, the host cell is a T cell or NK cell;
   preferably, the host cell is a chimeric antigen receptor-T cell.

45. Use of the antibody or the antigen binding fragment thereof according to any one of the claims 1 to 31, or the bispecific or multispecific molecule according to claim 37, or the immunoconjugate according to claim 38, or the pharmaceutical composition according to claim 39, or the chimeric antigen receptor according to claim 41, or the host cell according to claim 44 in the preparation of a medication for the prevention and/or treatment of a tumor in a subject;

   preferably, the subject is human;
   preferably, the medication further comprises an additional pharmaceutically active agent; preferably, the additional pharmaceutically active agent is a drug with anti-tumor activity; more preferably, the pharmaceutically active agent is an alkylating agent, a mitotic inhibitor, an anti-tumor antibiotic, an antimetabolite, a topoisomerase inhibitor, a tyrosine kinase inhibitor, a radionuclide agent, a radiosensitizing agent, an anti-angiogenic agent, a cytokine, a molecularly targeted drug, an immune checkpoint inhibitor or an oncolytic virus;
   preferably, the tumor is selected from B-cell lymphoma; T-cell lymphoma; melanoma; prostatic cancer; renal cell carcinoma; sarcoma; glioma, preferably high-grade glioma; blastoma, preferably neuroblastoma; osteosarcoma; plasmacytoma; histiocytoma; pancreatic cancer; breast cancer; lung cancers, preferably small cell lung cancer and non-small cell lung cancer; gastric cancer; liver cancer; colon cancer; rectal cancer; esophageal cancer; colorectal cancer; hematopoietic system cancer; testicular cancer; cervical cancer; ovarian cancer; bladder cancer; squamous cell carcinoma; adenocarcinoma; AIDS-related lymphoma; bladder cancer; brain cancer; nervous system cancer; head and neck cancer; head and neck squamous cell carcinoma; Hodgkin's lymphoma; non-Hodgkin's lymphoma; or a condition predisposing to hematologic malignancies.

**Fig. 1**

**Fig. 2A**

**Fig. 2B**

Fig. 3

**Fig. 4A**

M: DL2000 molecular weight standard

1. IVT-CD276-41BBζ

2. IVT-CD276-41BBζ / Xba I

**Fig. 4B**

M: DL2000 molecular weight standard

S1&S2: IVT-Flag-CD276-4-1BB-CD3ζ

**Fig. 4C**

Effector to target ratio

**Fig. 4D**

**NCI-H292**

**Fig. 5A**

**SK-OV-3**

**Fig. 5B**

**Fig. 6A**

**Fig. 6B**

**SK-OV3**

Fig. 7

Fig. 8A

Ampicillin resistance

C9-L

P11

Hu012-7-CD3-BiTE

pSC65-C9-P11
Hu012-7-CD3-BiTE-BFP

C9-R

P7.5

BFP

**Fig. 8B**

SK-OV3

Cytotoxicity%

UTD
rTTV-m8524-CD3-BiTE
rTTV-Hu012-7-CD3-BiTE

**Fig. 9**

**Fig. 10A**

**Fig. 10B**

**Fig. 11**

**Fig. 12A**

**Fig. 12B**

## INTERNATIONAL SEARCH REPORT

| International application No. |
|---|
| **PCT/CN2024/090299** |

| A. | CLASSIFICATION OF SUBJECT MATTER |
|---|---|

C07K16/28(2006.01)i; C12N5/10(2006.01)i; A61K39/00(2006.01)i; C12N15/13(2006.01)i; A61K39/395(2006.01)i; A61P35/00(2006.01)i

According to International Patent Classification (IPC) or to both national classification and IPC

| B. | FIELDS SEARCHED |
|---|---|

Minimum documentation searched (classification system followed by classification symbols)

IPC: C07K C12N A61K A61P

Documentation searched other than minimum documentation to the extent that such documents are included in the fields searched

Electronic data base consulted during the international search (name of data base and, where practicable, search terms used)

CNABS, DWPI, CNTXT, USTXT, EPTXT, WOTXT, JPTXT, CNKI, Web of Science, 万方数据检索系统, Wanfang Data Search System, pubmed, patentics, 中国专利生物序列检索系统, China Patent Biological Sequence Search System, GENBANK, STN: SEQ ID NOs: 1-10, 31-50, 111-120, 141-152, 177-200, 273-284, 312, 314, 316 and 318, CD276, B7H3, B7-H3, 抗体, antibody, McAb, Mab, CAR-T, chimeric antigen receptor-T cell, 免疫缀合物, ADC, bispecific

| C. | DOCUMENTS CONSIDERED TO BE RELEVANT |
|---|---|

| Category* | Citation of document, with indication, where appropriate, of the relevant passages | Relevant to claim No. |
|---|---|---|
| A | WO 2023060922 A1 (HRAIN BIOTECHNOLOGY CO., LTD.) 20 April 2023 (2023-04-20) claims 1-21 | 1, 4-5 (in part), 12 (in part), 15 (in part), 16, 19-20, 27, 30-45 (in part) |
| A | WO 2022151960 A1 (PERSONGEN BIOTHERAPEUTICS (SUZHOU) CO., LTD.) 21 July 2022 (2022-07-21) claims 1-10, and description, paragraph 40 | 1, 4-5 (in part), 12 (in part), 15 (in part), 16, 19-20, 27, 30-45 (in part) |
| A | CN 113621068 A (HRAIN BIOTECHNOLOGY CO., LTD.) 09 November 2021 (2021-11-09) claims 1-21 | 1, 4-5 (in part), 12 (in part), 15 (in part), 16, 19-20, 27, 30-45 (in part) |

☑ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **29 July 2024** | **06 August 2024** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2024/090299**

### C. DOCUMENTS CONSIDERED TO BE RELEVANT

| Category* | Citation of document, with indication, where appropriate, of the relevant passages | Relevant to claim No. |
|---|---|---|
| A | US 2016053017 A1 (THE UNITED STATES OF AMERICA, AS REPRESENTED BY THE SECRETARY, DEPARTMENT OF HEALTH AND HUMAN SERV et al.) 25 February 2016 (2016-02-25)<br>claims 1-29 | 1, 4-5 (in part), 12 (in part), 15 (in part), 16, 19-20, 27, 30-45 (in part) |
| A | WO 2022237820 A1 (ANTENGENE BIOLOGICS LIMITED) 17 November 2022 (2022-11-17)<br>claims 1-67 | 1, 4-5 (in part), 12 (in part), 15 (in part), 16, 19-20, 27, 30-45 (in part) |
| A | WO 2023060137 A2 (BOARD OF REGENTS, THE UNIVERSITY OF TEXAS SYSTEM et al.) 13 April 2023 (2023-04-13)<br>claims 1-308 | 1, 4-5 (in part), 12 (in part), 15 (in part), 16, 19-20, 27, 30-45 (in part) |
| A | WO 2023272924 A1 (XUZHOU MEDICAL UNIVERSITY) 05 January 2023 (2023-01-05)<br>claims 1-157 | 1, 4-5 (in part), 12 (in part), 15 (in part), 16, 19-20, 27, 30-45 (in part) |

Form PCT/ISA/210 (second sheet) (July 2022)

## INTERNATIONAL SEARCH REPORT

| International application No. |
|---|
| **PCT/CN2024/090299** |

**Box No. I          Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT

| International application No. |
|---|
| **PCT/CN2024/090299** |

**Box No. II        Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☑ Claims Nos.: **4-5 (in part), 12 (in part)**
   because they relate to parts of the international application that do not comply with the prescribed requirements to such an extent that no meaningful international search can be carried out, specifically:

   The nucleotide and/or amino acid sequence listing does not recite SEQ ID NOs: 184, 196 and 280 appearing in claims 4-5 (in part) and 12 (in part). Therefore, no search can be performed on the technical solution.

3. ☐ Claims Nos.:
   because they are dependent claims and are not drafted in accordance with the second and third sentences of Rule 6.4(a).

**Box No. III        Observations where unity of invention is lacking (Continuation of item 3 of first sheet)**

This International Searching Authority found multiple inventions in this international application, as follows:

Invention 1: claims 1 and 16, the technical solutions of claim 15 relating to SEQ ID NOs. 2 and 7, and the technical solutions of claims 30-45, which refer to the preceding claims.

Invention 2: claims 2 and 17, the technical solutions of claim 15 relating to SEQ ID NOs.12 and 17, and the technical solutions of claims 30-45, which refer to the preceding claims.

Invention 3: claims 3 and 18, the technical solutions of claim 15 relating to SEQ ID NOs. 22 and 27, and the technical solutions of claims 30-45, which refer to the preceding claims.

...

Invention 14: claims 14 and 29, the technical solutions of claim 15 relating to SEQ ID NOs. 132 and 137, and the technical solutions of claims 30-45, which refer to the preceding claims.

Inventions 1-14 relate to a plurality of antibodies or antigen-binding fragments thereof, and the same or corresponding technical feature of the inventions is an antibody or antigen-binding fragment that specifically bind to human CD276 protein. The prior art discloses a human antibody capable of specifically binding to human CD276 protein (see WO 2023060922 A1, claims 1-21, and description, embodiment 2, publication date: 20 April 2023). Therefore, the above 14 groups of inventions do not have a same or corresponding special technical feature, and thus lack unity of invention and do not comply with PCT Rule 13.1.

Form PCT/ISA/210 (continuation of first sheet) (July 2022)

## INTERNATIONAL SEARCH REPORT

| International application No. |
| --- |
| **PCT/CN2024/090299** |

**Box No. III      Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☑ As only some of the required additional search fees were timely paid by the applicant, this international search report covers only those claims for which fees were paid, specifically claims Nos.: **claims 1, 4-5, 12, 16, 19-20 and 27, the technical solutions of claim 15 relating to SEQ ID NOs: 2, 7, 32, 37, 42, 47, 112 and 117, and the technical solutions of claims 30-45, which refer to the preceding claims.**

4. ☐ No required additional search fees were timely paid by the applicant. Consequently, this international search report is restricted to the invention first mentioned in the claims; it is covered by claims Nos.:

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☑ No protest accompanied the payment of additional search fees.

Form PCT/ISA/210 (continuation of first sheet) (July 2022)

66

International application No.

**PCT/CN2024/090299**

| Patent document cited in search report | | | Publication date (day/month/year) | Patent family member(s) | | | Publication date (day/month/year) |
|---|---|---|---|---|---|---|---|
| WO | 2023060922 | A1 | 20 April 2023 | None | | | |
| WO | 2022151960 | A1 | 21 July 2022 | EP | 4279506 | A1 | 22 November 2023 |
| | | | | EP | 4279506 | A4 | 17 July 2024 |
| CN | 113621068 | A | 09 November 2021 | None | | | |
| US | 2016053017 | A1 | 25 February 2016 | US | 9790282 | B2 | 17 October 2017 |
| | | | | US | 2020255534 | A1 | 13 August 2020 |
| | | | | US | 11512138 | B2 | 29 November 2022 |
| | | | | US | 2017369585 | A1 | 28 December 2017 |
| | | | | US | 10604583 | B2 | 31 March 2020 |
| | | | | US | 2023110875 | A1 | 13 April 2023 |
| | | | | WO | 2014160627 | A1 | 02 October 2014 |
| WO | 2022237820 | A1 | 17 November 2022 | EP | 4341297 | A1 | 27 March 2024 |
| | | | | JP | 2024521045 | A | 28 May 2024 |
| | | | | IL | 308382 | A | 01 January 2024 |
| | | | | CA | 3219642 | A1 | 17 November 2022 |
| | | | | KR | 20240025513 | A | 27 February 2024 |
| | | | | AU | 2022274204 | A1 | 04 January 2024 |
| WO | 2023060137 | A2 | 13 April 2023 | WO | 2023060137 | A3 | 02 November 2023 |
| WO | 2023272924 | A1 | 05 January 2023 | None | | | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20130078234 A1

- US 20170369585 A1

**Non-patent literature cited in the description**

- **CHOTHIA** ; **LESK**. *J. Mol. Biol.*, 1987, vol. 196, 901-917
- **CHOTHIA et al.** *Nature*, 1989, vol. 342, 878-883

- **MP LEFRANC**. *Nucleic Acids Res.*, 1999, vol. 27, 209-12
- **MP LEFRANC**. *Nucleic Acids Res.*, 2015, vol. 43, D413-22